# EUROPÄISCHE PATENTSCHRIFT

(11) **EP 3 400 949 B1**
(45) Veröffentlichungstag und Bekanntmachung des Hinweises auf die Patenterteilung: **27.01.2021**
(21) Anmeldenummer: 17173824.8
(22) Anmeldetag: 31.05.2017
(51) Int. Cl.: A61K 31/728, A61K 31/737, A61P 13/10

(54) **ZUSAMMENSETZUNG FÜR DIE TOPISCHE ANWENDUNG, INSBESONDERE ZUR BLASENINSTILLATION**
COMPOSITION FOR TOPICAL USE, IN PARTICULAR FOR BLADDER INSTILLATION
COMPOSITION POUR L'APPLICATION TOPIQUE EN PARTICULIER POUR L'INSTILLATION DANS LA VESSIE

(30) Priorität: 12.05.2017 EP 17000822
(43) Veröffentlichungstag der Anmeldung: 14.11.2018
(73) Patentinhaber: Farco-Pharma GmbH, 50670 Köln (DE)
(72) Erfinder: SODHA, Frank, D - 50670 Köln (DE)
(74) Vertreter: Von Rohr Patentanwälte Partnerschaft mbB

(56) Entgegenhaltungen:
- WO-A1-2008/071245
- Li Marzi ET AL: "INTRAVESICAL ADMINISTRATION OF HYALURONIC ACID PLUS CONDROITIN SULPHATE TO REDUCE LOCAL BACILLUS CALMETTE GUERIN (BCG) TOXICITY: A RANDOMIZED PROSPECTIVE PILOT STUDY", , 2013, XP055423089, Gefunden im Internet: URL:https://www.ics.org/Abstracts/Publish/ 180/000554.pdf [gefunden am 2017-11-09]
- JUNG-SOO PYO ET AL.: "Systematic Review and Meta-Analysis of Intravesical Hyaluronic Acid and Hyaluronic Acid/Chondroitin Sulfate Instillation for Interstitial Cystitis/Painful Bladder Syndrome", CELLULAR PHYSIOLOGY AND BIOCHEMISTRY, Bd. 39, 2016, Seiten 1618-1625, XP002775407,

## Beschreibung

Die vorliegende Erfindung betrifft das medizinisch-therapeutische Gebiet von insbesondere malignen Erkrankungen des Urogenitaltraktes, wie Harnblasenkarzinomen, und einer damit in Verbindung stehenden BCG-Therapie (*Bacillus-Calmette-Guerin*-Therapie). Insbesondere betrifft die vorliegende Erfindung das Gebiet der Verhinderung bzw. Verringerung von mit einer BCG-Therapie einhergehenden bzw. hierdurch hervorgerufenen Nebenwirkungen.

Insbesondere betrifft die vorliegende Erfindung eine Zusammensetzung zur Verwendung bei der Erhöhung der Verträglichkeit einer BCG-Therapie von malignen Erkrankungen des Urogenitaltraktes, wie Harnblasenkarzinomen. In diesem Zusammenhang betrifft die vorliegende Erfindung auch eine Zusammensetzung zur Verwendung als Co-Therapeutikum bzw. zur Verwendung in der Co-Therapierung bei der in Rede stehenden BCG-Therapie. Gleichermaßen betrifft die vorliegende Erfindung auch eine Aufbewahrungs- bzw. Applikationsvorrichtung, welche eine entsprechende Zusammensetzung zur Verwendung nach der Erfindung enthält. Die vorliegende Erfindung betrifft auch eine die erfindungsgemäße Aufbewahrungs- bzw. Applikationsvorrichtung enthaltende Verpackungseinheit sowie ein Kit, welches die Aufbewahrungs- bzw. Applikationsvorrichtung nach der Erfindung, eine entsprechende Zusammensetzung zur Verwendung nach der Erfindung sowie eine an die Aufbewahrungs- bzw. Applikationsvorrichtung als solche anschließbare Instillationsvorrichtung enthält.

Unter dem Begriff "Blasenkrebs", synonym auch als Blasenkarzinom bezeichnet, werden im Allgemeinen in der Harnblase vorkommende bzw. von der Harnblase ausgehende und insbesondere in bösartiger Form vorliegende Geschwülste bzw. Tumoren bezeichnet. Mit einem Anteil von etwa 2 % sämtlicher maligner Krebsarten stellt der Blasenkrebs eine relativ häufig auftretende Erkrankung dar, welche an fünfter bzw. zehnter Stelle der häufigsten Krebsarten bei Männern bzw. Frauen steht. In diesem Zusammenhang erkranken in Deutschland jährlich etwa 15.000 Menschen Blasenkrebs, wobei Männer mit jährlich über 10.000 Neuerkrankungen etwa doppelt so häufig wie Frauen von einer Blasenkrebserkrankung betroffen sind. Dabei liegt das mittlere Erkrankungsalter bei etwa 69 Jahren für Männer und bei etwa 73 Jahren für Frauen. In Industrieländern tritt das Harnblasenkarzinom rund sechs mal häufiger auf als in Entwicklungsländern, wobei die Häufigkeit der Erkrankung während des 20. Jahrhunderts insgesamt angestiegen ist.

Was die Ursachen für die Entstehung eines Blasentumors anbelangt, so wurde für Zigarettenraucher ein diesbezüglich höheres Risiko im Vergleich zu Nichtrauchern nachgewiesen, wobei davon ausgegangen wird, dass der Anteil der Blasentumoren, die durch Zigarettenkonsum mitverursacht werden, an der Gesamtzahl diagnostizierter Blasentumore zwischen 30 % und 40 % beträgt. Einen großen Einfluss auf die Karzinomentstehung haben insbesondere aromatische Kohlenwasserstoffe. Neben dem Tabakkonsum können auch Medikamente mit der Entstehung von Blasentumoren in Verbindung gebracht werden, was auch für verschiedene Arbeitsstoffe, wie zum Beispiel aromatische Amine oder dergleichen, gilt. Zudem findet sich eine erhöhte Inzidenz des Blasenkarzinoms bei Patienten mit chronischen Harnwegsinfekten. Zudem können chronische Infektionen der Harnblase die Entstehung entsprechender Karzinome begünstigen. Auch chronische Entzündungen im Bereich der Harnblase können das Risiko für eine bösartige Tumorerkrankung erhöhen. Hierzu zählen insbesondere auch langwierige Blasensteinleiden sowie chronische Harnwegsinfekte.

Der häufigste in der Harnblase vorkommende bösartige Tumor ist das vom Urothel der Harnblase ausgehende urotheliale Karzinom. Es können auch Plattenepithelkarzinome vorkommen, welche jedoch im Allgemeinen relativ selten auftreten. Bei der ersten Diagnose wird zu rund 75 % ein oberflächliches Karzinom aufgefunden. In etwa 20 % der Fälle ist das zugrundeliegende Karzinom bereist invasiv und in 5 % der vorliegenden Fälle liegen bereits Metasthasenbildungen vor.

Die in Rede stehenden Harnblasenkarzinome fallen oftmals durch eine schmerzlose Mikrohämaturie, bei welcher Blut im Urin zunächst nicht durch optische Sichtkontrolle, sondern insbesondere mittels eines Streifentests nachgewiesen werden kann, bzw. durch eine schmerzlose Makrohämaturie, bei welcher Blut im Urin mit dem Auge sichtbar ist, auf. Zudem liegt mitunter eine Reizsymptomatik mit häufigem Wasserlassen und Dysurie, also einem erschwerten unangenehmen Wasserlassen, vor. Im fortgeschrittenen Tumorstadium können zudem Schmerzen in der Blasen- bzw. Darmregion auftreten.

Was die Therapie des Harnblasenkarzinoms anbelangt, so erfolgte diese insbesondere stadienabhängig und unter Berücksichtigung der Lebensumstände der betroffenen Patienten.

In diesem Zusammenhang kommt im Allgemeinen eine Behandlung oberflächlicher Tumoren auf Basis einer lokalen Verabreichung in Form einer intravesikalen Applikation bzw. Instillation in die Harnblase von Chemotherapeutika, wie beispielsweise Mitomycin C, welche auch unmittelbar postoperativ erfolgen kann, in Betracht.

Insbesondere für den Fall, dass das zugrundeliegende Karzinom als urotheliales Karzinom bzw. oberflächliches Karzinom (oberflächliches Harnblasenkarzinom) bzw. als *Carcinoma in situ* ("Krebs am Ursprungsort") vorliegt, kann eine Behandlung in Form einer BCG-Therapie (*Bacillus-Calmette-Guérin*-Therapie), gegebenenfalls begleitend zu einer insbesondere lokalen Chemotherapie bzw. begleitend zu anderen therapeutischen Maßnahmen, durchgeführt werden. Bei BCG *(Bacillus-Calmette-Guérin*) handelt es sich um ein abgeschwächt-virulentes bzw. attenuiertes Bakterium. Die BCG-Therapie erfolgt dabei im Allgemeinen durch intravesikale Applikation bzw. Instillation in die Harnblase einer BCG-haltigen Zusammensetzung. Die in Rede stehende BCG-Therapie wird insgesamt auf Basis eines immuntherapeutischen Ansatzes zur Behandlung des Harnblasenkarzinoms, insbesondere des urothelialen Harnblasenkarzinoms bzw. des oberflächlichen Harnblasenkarzinoms, eingesetzt, und zwar in Form einer lokalen Anwendung unter Instillation BCG-haltiger Zusammensetzungen in die Harnblase, wie zuvor angeführt.

Die lokale Applikation bzw. Instillation mit BCG führt - ohne sich auf diese Theorie festlegen oder beschränken zu wollen - zu einer insbesondere lokalen Immun- bzw. Entzündungsreaktion unter Granulombildung, bei welcher gleichermaßen auch Granulozyten und Lymphozyten aktiviert werden. Die zugrundeliegende, durch BCG induzierte lokale Entzündungsreaktion führt dabei im Idealfall auch zu einer Zerstörung von Tumorzellen des Harnblasenkarzinoms bzw. zu einer Immunisierung gegen die in Rede stehenden Tumorzellen, so dass Tumorzellen abgetötet werden bzw. deren Neuentstehung verhindert wird. Folglich kann auch einer Rezidivbildung entgegengewirkt werden.

Im Allgemeinen kann eine BCG-Therapie somit vorrangig zur Behandlung insbesondere nicht-invasiver urothelialer Harnblasenkarzinome in Betracht kommen, und zwar einerseits zur kurativen Behandlung eines *Carcinoma in situ* sowie andererseits zur prophylaktischen Behandlung zur Vermeidung eines erneuten Auftretens von urothelialen Karzinomen bzw. eines *Carcinoma in situ.*

In diesem Zusammenhang ist die BCG-Therapie insbesondere für die intravesikale Immuntherapie bei einem vorzugsweise oberflächlichen Harnblasenkarzinom mit insbesondere hohem Rezidivrisiko bzw. insbesondere hohem Progressionsrisiko indiziert. Zudem kann eine BCG-Therapie auch nach erfolgter Tumorresektion erfolgen.

Die Behandlung mit einer entsprechenden BCG-haltigen Zusammensetzung kann dabei als Kurzzeittherapie (z. B. 6 bis 8 Wochen) oder als Langzeittherapie (z. B. 12 bis 36 Monate) erfolgen, wobei auf eine Induktionstherapie grundsätzlich eine Erhaltungstherapie erfolgen sollte.

Was die Verabreichung bzw. Instillation von BCG anbelangt, so kann beispielsweise zunächst ein sechswöchiger Behandlungszyklus mit einer Verabreichung der Zusammensetzung einmal pro Woche erfolgen, wobei dem sechswöchigen Behandlungszyklus eine Behandlungspause von sechs Wochen angeschlossen werden kann, gegebenenfalls gefolgt von einer entsprechenden Nachresektion. Anschließend kann eine Erhaltungstherapie durchgeführt werden, wobei beispielsweise drei Instillationen im wöchentlichen Abstand in den Monaten 3, 6, 12, 18, 24, 30 und 36 durchgeführt werden können.

Im Allgemeinen besteht ein therapeutisches Ziel der intravesikalen Therapie mit BCG darin, entsprechende Tumorzellen abzutöten und das Rezidivintervall zu verlängern bzw. eine Tumorprogression zu verhindern. Die Behandlung weist im Allgemeinen eine hohe therapeutische Wirksamkeit auf, wobei sich bei etwa zwei Dritteln der mit einer BCG-Therapie behandelten Patienten ein langfristiger Erfolg einstellt.

Die wissenschaftliche Publikation gemäß Li Marzi et al.: "Intravesical Administration of Hyaluronic Acid plus Condroitin Sulphate to Reduce Local Bacillus Calmette Guerin (BCG) Toxicity: A Randomized Prospective Pilot Study*"* beschreibt den Einsatz von Zusammensetzungen auf Basis von Hyaluronsäure und Chondroitinsulfat zur Reduzierung der BCG-Toxizität, wobei lediglich auf eine Reduzierung von lokalen Nebenwirkungen abgestellt wird.

Die WO 2008/071245 A1 betrifft eine pharmazeutische Zusammensetzung zur Behandlung von entzündlichen Erkrankungen des Urogenitaltrakts, vorzugsweise von Cystitis. Die darin beschriebene Zusammensetzung enthält in Kombination und jeweils in pharmazeutisch wirksamen Mengen Hyaluronsäure in Kombination mit einem weiteren, hiervon verschiedenen Glykosaminoglykan, vorzugsweise Chondroitinsulfat.

Die EP 0 488 583 A2 bzw. die zu derselben Patentfamilie gehörende US 5 194 254 betrifft ein Kit zur In-vivo-Behandlung oberflächlicher Blasentumore durch Verabreichung mittels intravesikaler Instillation von *Bacillus Calmette-Guérin* in lyophilisierter Form, wobei die verabreichte Zusammensetzung zudem Natriumchlorid und einen Puffer zur Aufrechterhaltung des pH-Wertes in einem bestimmten Bereich enthalten soll.

Nachteilig bei der Behandlung bzw. Therapie des Harnblasenkarzinoms mit einer BCG-Zusammensetzung, welche im Allgemeinen intravesikal mit entsprechender Instillation der Zusammensetzung in die Harnblase verabreicht bzw. appliziert wird, ist deren mitunter schlechte Verträglichkeit bzw. das hohe Maß an damit oftmals einhergehenden und mitunter schwerwiegenden Nebenwirkungen, welche sowohl lokal als auch systemisch auftreten können und welche sogar zu Therapieabbrüchen oder unerwünschten Therapiepausen führen können, was insgesamt dem Behandlungserfolg abträglich ist.

Zu den in Rede stehenden Nebenwirkungen zählen insbesondere übermäßige entzündliche Reaktionen bzw. Veränderungen der Schleimhäute bzw. des Urothels, insbesondere des Harnblasenurothels. Zudem können Pollakisurie, Dysurie, Hämaturie, Prostatitis und eine verminderte Harnblasenkapazität sowie Schmerzen im Bereich des Urogenitaltrakts, insbesondere infolge von übermäßigen entzündlichen Reaktionen, auftreten, was den gewünschten Therapieerfolg insgesamt gefährden kann. Zudem führen die mit der BCG-Therapie einhergehenden Nebenwirkungen und Unverträglichkeiten in ihrer Gesamtheit auch zu einer verringerten Patientencompliance, was dem gewünschten Therapieerfolg gleichermaßen entgegenstehen kann.

Die mit der BCG-Therapie einhergehenden Unverträglichkeiten bzw. Nebenwirkungen gehen oftmals auch mit einer weiterführenden bzw. übermäßigen Schädigung insbesondere der Blasenschleimhaut bzw. des (Harnblasen-)Urothels einher, was insbesondere auch mit einer entsprechenden Schmerzsymptomatik verbunden ist. Insbesondere kann aufgrund übermäßiger entzündlicher Reaktionen insbesondere als Primäreffekt eine Schwächung des Urothels der Harnblase bzw. des Blasenepithels vorliegen, wobei auch eine Schädigung der Blasenschleimhaut bzw. der der Harnblase zugrundeliegenden inneren Schutzschicht vorliegen kann, welche im intakten Zustand das darunter liegende Gewebe der Harnblase gegenüber pathogenen Faktoren, wie Mikroorganismen, krebsverursachenden Substanzen und im Urin selbst vorkommenden schädlichen Substanzen abschirmt. In diesem Zusammenhang kann somit auf Basis von Sekundäreffekten eine weiterführende Schädigung der zugrundeliegenden physikalischen Barriere gegen schädliche Substanzen vorliegen, was im Ergebnis zu einer Verstärkung bzw. Verschlechterung der unerwünschten Nebenwirkungen führen kann.

Auch vor diesem Hintergrund besteht im Stand der Technik insgesamt großer Bedarf hinsichtlich einer weiterführenden Verbesserung der BCG-Therapie bei insbesondere malignen Erkrankungen des Urogenitaltraktes, bevorzugt Harnblasenkarzinomen, und zwar insbesondere was die Verringerung von Nebenwirkungen bzw. eine Verbesserung der Verträglichkeit anbelangt, zumal die zugrundeliegende BCG-Therapie insgesamt einen wirksamen und erfolgsversprechenden Ansatz zur Behandlung von Karzinomerkrankungen darstellt. Dabei ist auch beachtlich, dass eine Verringerung von Nebenwirkungen bzw. eine Verbesserung der Verträglichkeit auch mit einer erhöhten Patientencompliance einhergeht, was zu einer Verbesserung auch der Anwendungseigenschaften sowie der Wirksamkeit einer BCG-Therapie insgesamt führt.

Vor diesem Hintergrund besteht daher eine Aufgabe der vorliegenden Erfindung darin, eine entsprechende Zusammensetzung, insbesondere pharmazeutische Zusammensetzung, zur Verwendung bei der prophylaktischen bzw. therapeutischen Behandlung von Nebenwirkungen einer BCG-Therapie von insbesondere malignen Erkrankungen des Urogenitaltraktes, wie Harnblasenkarzinomen, bereitzustellen, welche die zuvor geschilderten Nachteile des Standes der Technik zumindest weitgehend vermeidet oder aber wenigstens abschwächt.

Insbesondere soll eine solche Zusammensetzung bereitgestellt werden, welche zu einer effektiven Behandlung bzw. zu einer Verringerung von mit einer BCG-Therapie einhergehenden Nebenwirkungen führt, wobei auf dieser Basis gleichermaßen die Verträglichkeit der zugrundeliegenden Zusammensetzung verbessert werden soll. Somit soll im Rahmen der vorliegenden Erfindung auch eine solche Zusammensetzung bereitgestellt werden, welche zu einer Verbesserung bzw. Erhöhung der Patientencompliance der in Rede stehenden BCG-Therapie führt.

Darüber hinaus ist eine wiederum weitere Aufgabe der vorliegenden Erfindung auch darin zu sehen, eine solche Zusammensetzung bereitzustellen, welche zu einer Verbesserung bzw. Erhöhung der Wirksamkeit bzw. zu einer Wirkverstärkung einer BCG-Therapie im Hinblick auf die Behandlung von insbesondere malignen Erkrankungen des Urogenitaltraktes, wie insbesondere Harnblasenkarzinomen, führt. In diesem Zusammenhang soll im Rahmen der vorliegenden Erfindung somit gleichermaßen eine solche Zusammensetzung bereitgestellt werden, welche sich als Co-Therapeutikum für eine BCG-Therapie als solche eignet.

Insbesondere soll die in diesem Zusammenhang im Rahmen der vorliegenden Erfindung bereitgestellte Zusammensetzung darüber hinaus eine hohe Stabilität, insbesondere Lagerungsstabilität, aufweisen und insbesondere im Hinblick auf längere Lagerungszeiträume konstante Wirkstoffmengen und gleichbleibende physikochemische Eigenschaften aufweisen.

Zur Lösung der zuvor geschilderten Aufgabe schlägt die vorliegende Erfindung - gemäß einem **ersten** Aspekt der vorliegenden Erfindung - eine Zusammensetzung, insbesondere pharmazeutische Zusammensetzung, zur Verwendung nach Anspruch 1 vor; weitere, insbesondere vorteilhafte Ausgestaltungen der erfindungsgemäßen Zusammensetzung sind Gegenstand der diesbezüglichen Unteransprüche.

Zudem betrifft die vorliegende Erfindung - gemäß einem **zweiten** Aspekt der vorliegenden Erfindung - eine die erfindungsgemäße Zusammensetzung aufweisende bzw. enthaltende Aufbewahrungs- bzw. Applikationsvorrichtung, wie sie in dem die Aufbewahrungs- bzw. Applikationsvorrichtung betreffenden unabhängigen Anspruch definiert ist; weitere, insbesondere vorteilhafte Ausgestaltungen der erfindungsgemäßen Aufbewahrungs- bzw. Applikationsvorrichtung sind Gegenstand des diesbezüglichen Unteranspruchs.

Darüber hinaus betrifft die vorliegende Erfindung - gemäß einem **dritten** Aspekt der vorliegenden Erfindung - auch eine Verpackungseinheit, welche die Aufbewahrungs- bzw. Applikationsvorrichtung nach der Erfindung enthält, gemäß dem die Verpackungseinheit betreffenden unabhängigen Anspruch.

Schließlich betrifft die vorliegende Erfindung - gemäß einem **vierten** Aspekt der vorliegenden Erfindung - auch ein Kit, insbesondere Installationssystem, wie es in dem das Kit betreffenden unabhängigen Anspruch definiert ist.

Es versteht sich im Zusammenhang mit den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt aufgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbezug sämtlicher Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Weiterhin gilt, dass der Fachmann - anwendungsbezogen oder einzelfallbedingt - von den nachfolgend angeführten Konzentrations-, Gewicht-, Mengen- und Bereichsangaben abweichen kann, ohne dass er den Rahmen der vorliegenden Erfindung verlässt.

Zudem gilt, dass sämtliche im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Der Begriff des Arzneimittels bzw. Medikaments (synonym auch "Pharmazeutikum"), wie er im Rahmen der vorliegenden Erfindung verwendet wird, ist sehr umfänglich zu verstehen und umfasst nicht nur Arzneimittel bzw. Pharmazeutika als solche (d. h. in arzneimittelrechtlicher Hinsicht), sondern vor allem auch sogenannte Medizinprodukte und darüber hinaus aber auch Homöopathika und Nahrungsergänzungsmittel sowie Kosmetika und Gebrauchsgegenstände. Mit anderen Worten kann also die erfindungsgemäße Zusammensetzung in Form eines Arzneimittels (Pharmazeutikums), Medizinprodukts, Homöopathikums, Nahrungsergänzungsmittels, Kosmetikums oder in Form eines Gebrauchsgegenstands vorliegen.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit eine Zusammensetzung, insbesondere pharmazeutische Zusammensetzung, zur Verwendung bei der Erhöhung der Verträglichkeit einer BCG-Therapie (*Bacillus-Calmette-Guérin*-Therapie) von malignen Erkrankungen des Urogenitaltraktes, insbesondere der Harnblase, vorzugsweise von malignen Karzinomen, bevorzugt von Harnblasenkarzinomen,
wobei die Zusammensetzung in Kombination und jeweils in wirksamen, insbesondere pharmazeutisch wirksamen Mengen enthält:
(a) Chondroitinsulfat und/oder ein physiologisch verträgliches Chondroitinsulfat-Salz (Komponente (a)), wobei die Zusammensetzung das Chondroitinsulfat und/oder das physiologisch verträgliche Chondrointinsulfat-Salz in einer Konzentration in einem Bereich von (2 ± 0,5) mg/ml bis (25 ± 2) mg/ml aufweist; und
(b) Hyaluronsäure und/oder ein physiologisch verträgliches Hyaluronsäure-Salz (Hyaluronat) (Komponente (b)), wobei die Zusammensetzung die Hyaluronsäure und/oder das physiologisch verträgliche Hyaluronsäure-Salz in einer Konzentration in einem Bereich von (1 ± 0,5) mg/ml bis (20 ± 2) mg/ml aufweist.

Bei der zugrundeliegenden malignen Erkrankung des Urogenitaltraktes handelt es sich insbesondere um ein Harnblasenkarzinom, welches insbesondere als urotheliales Harnblasenkarzinom bzw. als oberflächliches Harnblasenkarzinom ausgebildet ist bzw. vorliegt.

Die Anmelderin hat in völlig überraschender Weise gefunden, dass mit der erfindungsgemäßen Zusammensetzung auf Basis einer Kombination von Chondroitinsulfat bzw. eines physiologisch verträglichen Chondroitinsulfat-Salzes einerseits und Hyaluronsäure bzw. eines physiologisch verträglichen Hyaluronsäure-Salzes (Hyaluronat) andererseits die mit einer BCG-Therapie einhergehenden Nebenwirkungen signifikant verringert bzw. vermieden werden können, insbesondere im Hinblick auf unerwünschte übermäßige entzündliche Erkrankungen bzw. Veränderungen der Schleimhäute bzw. des Epithels des Urogenitaltraktes bzw. des Urothels, insbesondere des Harnblasenurothels. Auf Basis der erfindungsgemäßen Konzeption wird somit die Verträglichkeit einer BCG-Therapie mit der diesbezüglichen intravesikalen Applikation bzw. Instillation einer entsprechenden BCG-haltigen Zusammensetzung nachhaltig verbessert, einhergehend mit einer Verbesserung der Patientencompliance.

In diesem Zusammenhang ist es gleichermaßen vollkommen überraschend, dass die erfindungsgemäß bereitgestellte Verhinderung bzw. Verringerung von durch eine BSG-Therapie hervorgerufenen Nebenwirkungen zudem nicht zu einer negativen Beeinflussung bzw. Abschwächung der Wirkeffizienz der zugrundeliegenden BCG-Therapie als solcher führen. Vielmehr verhält es sich im Rahmen der vorliegenden Erfindung derart, dass unter Anwendung der erfindungsgemäßen Zusammensetzung im Sinne einer Co-Therapierung sogar eine weiterführende Wirksamkeitssteigerung der zugrundeliegenden BCG-Therapie erreicht wird, was beispielsweise auch dadurch erklärt werden kann, dass - ohne sich auf diese Theorie beschränken oder festlegen zu wollen - die zur BCG-Therapie eingesetzte BCG-haltige Zusammensetzung aufgrund der durch die erfindungsgemäße Zusammensetzung hervorgerufene Verringerung der Nebenwirkungen, wie übermäßiger Harndrang oder dergleichen, länger in der Harnblase verbleiben kann, einhergehend mit einer längeren Einwirkdauer der entsprechenden BCG-Wirckomponenten. Die erfindungsgemäße Zusammensetzung kann somit gleichermaßen zur Unterstützung und weiterführenden Verbesserung einer BCG-Therapie eingesetzt werden.

Eine weitere mögliche Erklärung für die hervorragende Wirkung der erfindungsgemäßen Zusammensetzung im Hinblick auf eine Verringerung von mit einer BCG-Therapie einhergehenden Nebenwirkungen kann - gleichermaßen ohne sich auf diese Theorie beschränken oder festlegen zu wollen - zudem darin gesehen werden, dass die Wirksubstanzen der erfindungsgemäßen Zusammensetzungen in besonders effektiver Weise insbesondere mit der Schleimhaut bzw. dem Urothel der Harnblase wechselwirken, wobei diesbezüglich eine An- bzw. Einlagerung der Wirksubstanzen an diese Schicht vorliegt, was sozusagen zu einer Reparatur der infolge der BCG-Therapie etwaig übermäßig geschädigten Glykosaminoglykanschicht bzw. zu einer diesbezüglichen Regeneration führt. Die mit dieser Wirkweise verbundene Verringerung der Permeabilität des Urothels - was gewissermaßen einem "Abdicht-"Effekt gegenüber dem in der Blase vorhandenen Harn bzw. Urin gleichkommt - führt auch zu einer deutlichen Minderung bzw. Verringerung der zugrundeliegenden Nebenwirkungen, insbesondere auch im Hinblick auf die vorliegende Schmerzsymptomatik. So führt die Verwendung der erfindungsgemäßen Zusammensetzung im Rahmen einer zugrundeliegenden BCG-Therapie bereits nach wenigen (Co-)Behandlungen bei von insbesondere malignen Karzinomen der Harnblase betroffenen Patienten zu einem signifikant verbesserten Gesundheitsstatus, einhergehend mit einer verbesserten Verträglichkeit und Haltedauer einer instillierten BCG-haltigen Zusammensetzung. Die erfindungsgemäße Zusammensetzung kann somit gewissermaßen zumindest auch zum vorübergehenden Ersatz einer defekten Glykosaminoglykanschicht des Urothels dienen bzw. eingesetzt werden, wobei die zugrundeliegenden Defekte ursächlich durch die in Rede stehende BCG-Therapie hervorgerufen sein können.

In diesem Zusammenhang kann die Wirkweise der Zusammensetzung nach der Erfindung - ohne sich hierauf festlegen zu wollen - insbesondere in einer maßgeblich physikalischen Wechselwirkung gesehen werden, bei welcher die Wirkstoffe gemäß den Komponenten (a) bzw. (b) in das übermäßig geschädigte Urothel bzw. in die Schleimhaut eingebaut bzw. eingelagert werden bzw. sich hieran anlagern, so dass ein durch insbesondere übermäßige entzündliche Reaktionen hervorgerufener Verlust von Chondroitinsulfat bzw. Hyaluronsäure in der Blasenwand bzw. im Urothel ausgeglichen bzw. kompensiert wird. Es liegt somit gewissermaßen eine Regulation der Permeabilität bzw. Durchlässigkeit der Blasenwand vor, was zu einer Eindämmung übermäßiger Entzündungsreaktionen und somit zu einer Linderung der in Rede stehenden Nebenwirkungen führt, und zwar ohne die Wirksamkeit einer BCG-haltigen Zusammensetzung als solcher im Hinblick auf die Tumorbehandlung negativ zu beeinflussen. Die erfindungsgemäße Zusammensetzung bildet dabei zudem quasi einen Schutz des Blasenepithels vor unerwünscht irritierenden Substanzen, wie Mikrokristallen, pathogenen Substanzen bzw. Erregern oder dergleichen, wobei die Zusammensetzung nach der Erfindung als Ersatz und Schutz der Glykosaminoglykanschicht in der Harnblase und den ableitenden Harnwegen fungiert.

Was die erfindungsgemäße Zusammensetzung weiterhin anbelangt, so kann diese außerdem (c) ein Puffersystem, insbesondere Dihydrogenphosphat/Monohydrogenphosphat-Puffersystem, (Komponente (c)) enthalten, insbesondere wie nachfolgend noch im Detail angeführt.

Gleichermaßen kann es erfindungsgemäß vorgesehen sein, dass die Zusammensetzung außerdem (d) mindestens einen physiologisch verträglichen Elektrolyten (Komponente (d)) enthält. Hierzu kann auch auf die nachfolgenden Ausführungen verwiesen werden.

Überraschend wurde im Rahmen der vorliegenden Erfindung nämlich auch gefunden, dass auf Basis der erfindungsgemäßen Konzeption eine spezielle Zusammensetzung - welche nämlich als Komponente (a) Chondroitinsulfat bzw. ein physiologisch verträgliches Chondroitinsulfat-Salz, als Komponente (b) Hyaluronsäure bzw. ein physiologisch verträgliches Hyaluronsäure-Salz (synonym auch als "Hyaluronat" bezeichnet), als Komponente (c) gegebenenfalls ein Puffersystem, insbesondere ein Dihydrogenphosphat/Monohydrogenphosphat-Puffersystem (synonym auch als "H₂PO₄⁻/HPO₄²⁻-Puffer(system)" bzw. "Phosphatpuffer(system)" bzw. chemisches Puffersystem bezeichnet), sowie gegebenenfalls als Komponente (d) mindestens einen physiologisch verträglichen Elektrolyten enthält, wobei die Zusammensetzung zudem einen definierten und mittels des Puffersystems eingestellten bzw. konstantgehaltenen pH-Wert aufweisen kann - zur zweckgerichteten Applikation, insbesondere Instillation, im Rahmen der Behandlung von Nebenwirkungen einer BCG-Therapie, bereitgestellt werden kann, welche gegenüber dem Stand der Technik bei gleichzeitig hoher Wirksamkeit und sehr guter Verträglichkeit darüber hinaus eine verbesserte Stabilität, insbesondere Lagerungsstabilität, aufweist.

Gemäß dem vorliegenden Aspekt betrifft die vorliegende Erfindung somit auch eine Zusammensetzung, insbesondere pharmazeutische Zusammensetzung, zur Verwendung bei der Erhöhung der Verträglichkeit einer BCG-Therapie (*Bacillus-Calmette-Guérin*-Therapie) des Urogenitaltraktes, insbesondere der Harnblase, vorzugsweise bei malignen Karzinomen, bevorzugt bei Harnblasenkarzinomen, vorzugsweise eine zuvor definierte Zusammensetzung,
wobei die Zusammensetzung in Kombination und jeweils in wirksamen, insbesondere pharmazeutisch wirksamen Mengen enthält:
(a) Chondroitinsulfat und/oder ein physiologisch verträgliches Chondroitinsulfat-Salz (Komponente (a)), wobei die Zusammensetzung das Chondroitinsulfat und/oder das physiologisch verträgliche Chondrointinsulfat-Salz in einer Konzentration in einem Bereich von (2 ± 0,5) mg/ml bis (25 ± 2) mg/ml aufweist;
(b) Hyaluronsäure und/oder ein physiologisch verträgliches Hyaluronsäure-Salz (Hyaluronat) (Komponente (b)), wobei die Zusammensetzung die Hyaluronsäure und/oder das physiologisch verträgliche Hyaluronsäure-Salz in einer Konzentration in einem Bereich von (1 ± 0,5) mg/ml bis (20 ± 2) mg/ml aufweist;
(c) ein Puffersystem, insbesondere Dihydrogenphosphat/Monohydrogenphosphat-Puffersystem, (Komponente (c));
(d) mindestens einen physiologisch verträglichen Elektrolyten (Komponente (d));
wobei die Zusammensetzung einen pH-Wert in einem Bereich von 6,1 bis 7,9 aufweist und/oder wobei die Zusammensetzung auf einen pH-Wert in einem Bereich von 6,1 bis 7,9 eingestellt ist.

Auf Basis der erfindungsgemäßen Zusammensetzung wird somit ein hochwirksames Arzneimittel bzw. Medikament bzw. (Medizin-)Produkt bereitgestellt, welches aufgrund der gezielten Kombination und Abstimmung der zugrundeliegenden Komponenten zudem eine hervorragende Stabilität, insbesondere Lagerungsstabilität, aufweist, wobei auch nach entsprechend langen Lagerungszeiträumen kein wesentlicher und sowohl die Wirksamkeit als auch die Verträglichkeit negativ beeinflussender Abbau der Wirk- bzw. Inhaltsstoffe der Zusammensetzung selbst vorliegt.

Was die im Rahmen der vorliegenden Erfindung neben der hohen Wirksamkeit im Hinblick auf die Verringerung von Nebenwirkungen bei der BCG-Therapie zudem in völlig überraschender Weise aufgefundene Verbesserung der Stabilität, insbesondere Lagerungsstabilität, bei gleichzeitig hoher Wirksamkeit der erfindungsgemäßen Zusammensetzung anbelangt, so kommt auch diesbezüglich den in der erfindungsgemäßen Zusammensetzung vorliegenden Konzentrationen der Komponenten (a) und (b) eine entsprechende Bedeutung zu. Denn die Anmelderin hat in diesem Zusammenhang gefunden, dass auf Basis der erfindungsgemäß bevorzugt vorgesehenen Konzentrationen, wie nachfolgend definiert, und den damit einhergehenden Stoffmengen insbesondere auf Basis der Komponenten (a) und (b) ein diesbezügliches Stabilitäts- und Wirkoptimum vorliegt.

Dabei greifen die erfindungsgemäßen Maßnahmen auch im Hinblick auf die Verwendung eines Puffersystems gemäß Komponente (c) und die Einstellung eines speziellen pH-Wertes sozusagen ineinander und verstärken sich gegenseitig und im Hinblick auf die Gewährleistung einer hohen Stabilität, insbesondere Lagerungsstabilität, und einer hohen Wirksamkeit über die Wirkung der Einzelmaßnahmen hinaus, so dass diesbezüglich ein synergistischer Effekt vorliegt, wie auch anhand der nachfolgend angeführten Ausführungsbeispiele aufgezeigt. Ohne sich auf die nachfolgende Theorie beschränken oder festlegen zu wollen, liegt im Hinblick auf die erfindungsgemäße Zusammensetzung infolge der diesbezüglichen Maßnahmen sozusagen eine optimale Ausbildung einer Matrix bzw. eines Hydrogels auf Basis der zugrundeliegenden Komponenten, insbesondere im Hinblick auf die Komponenten (a) und (b), vor, was den Abbau der zugrundeliegenden Wirk- bzw. Inhaltsstoffe bzw. eine lagerungs- bzw. zeitbedingte Veränderung der physikochemischen Eigenschaften, wie Viskosität oder dergleichen, entgegenwirkt und zudem der Wirksamkeit zuträglich ist. Folglich liegt eine entsprechende Vergrößerung der Stabilität, einhergehend mit größeren Lagerungszeiträumen, vor.

Das gegebenenfalls vorhandene Puffersystem gemäß Komponente (c) dient im Rahmen der vorliegenden Erfindung dabei insbesondere zur Stabilisierung der erfindungsgemäßen Zusammensetzung. Insbesondere führt die Einstellung eines konstanten pH-Wertes mittels des Puffersystems überraschenderweise dazu, dass einem unerwünschten Abbau der Wirkstoffe, insbesondere der Komponenten (a) und (b), bei Lagerung auch über einen längeren Zeitraum effizient entgegengewirkt wird. Auf diese Weise wird die Stabilität bzw. Lagerfähigkeit der erfindungsgemäßen Zusammensetzung verbessert.

Hinsichtlich des Stabilitätsverhaltens hat es sich im Rahmen der vorliegenden Erfindung zudem in völlig überraschender Weise gezeigt, dass die bevorzugte Verwendung eines speziellen Phosphatpuffer(systems) gemäß Komponente (c), nämlich in Form eines Dihydrogenphosphat/Monohydrogenphosphat-Puffersystems, zu einer nochmaligen Verbesserung der Produkteigeschaften führt, wobei infolge der Verwendung des speziellen Phosphatpuffer(systems) auch eine weiterführende Verbesserung der zugrundeliegenden Stabilitätseigenschaften vorliegt. Gleichermaßen ohne sich auf diese Theorie beschränken bzw. festlegen zu wollen, führt der Einsatz eines speziellen Puffers auch zu einer weiterführenden Stabilisierung der aus den Komponenten (a) und (b) resultierenden Matrix bzw. des diesbezüglichen Hydrogels, und zwar auch dadurch bedingt, dass auf Basis des speziellen Puffersystems eine optimale Stabilisierung des pH-Wertes vorliegt, was der Stabilität der Zusammensetzung insgesamt zugute kommt.

Darüber hinaus zeichnet sich die erfindungsgemäße Zusammensetzung auch durch eine hervorragende Wirksamkeit bzw. Wirkeffizienz bei gleichzeitig guter Verträglichkeit im Hinblick auf die Behandlung von Nebenwirkungen einer BCG-Therapie bei bzw. von Karzinomen des Urogenitaltraktes bzw. Blasenkarzinomen, aus. Aufgrund der definierten und auch nach großen Lagerungszeiträumen vorliegenden konstanten physikochemischen Eigenschaften der erfindungsgemäßen Zusammensetzung ist zudem der Handhabung bzw. Applikation insbesondere im Hinblick auf die Instillation in die Harnblase, verbessert, was auch zu einer erhöhten Akzeptanz seitens des Patienten und zu einer Verringerung von Fehlanwendungen führt.

Auf Basis der erfindungsgemäßen Konzeption wird dabei insgesamt einer zeitlichen Degradation bzw. einem zeitlich bedingten Abbau der entsprechenden Wirk- bzw. Inhaltsstoffe, insbesondere auch im Hinblick auf die Komponenten (a) und (b), entgegengewirkt, so dass auch nach entsprechend langen Zeiträumen hohe bzw. konstante Wirkstoffkonzentrationen bei zeitlich konstantem pH-Wert vorliegen. Hierdurch ist zudem gewährleistet, dass mitunter toxische bzw. schädliche Abbauprodukte nicht bzw. nur in geringen Mengen vorliegen, was gleichermaßen positiv für die Wirkeffizienz und Verträglichkeit der erfindungsgemäßen Zusammensetzung zu werten ist.

Insgesamt wird somit auf Basis der erfindungsgemäßen Konzeption eine Zusammensetzung bereitgestellt, welche sich in hervorragender Weise zur Behandlung von Nebenwirkungen einer BCG-Therapie bzw. als diesbezügliches Co-Therapeutikum eignet und gegenüber dem Stand der Technik über signifikant verbesserte Eigenschaften verfügt.

Was die erfindungsgemäß eingesetzte Komponente (a) in Form von Chondroitinsulfat bzw. des physiologisch verträglichen Chondroitinsulfat-Salzes anbelangt (synonym auch als Chondroitinpolysulfat bzw. Chondroitinpolysulfat-Salz bezeichnet), so hat es sich erfindungsgemäß als vorteilhaft erwiesen, wenn die Zusammensetzung das Chondroitinsulfat bzw. das physiologisch verträgliche Chondroitinsulfat-Salz (Komponente (a)) in einer Konzentration in einem Bereich von (3 ± 0,5) mg/ml bis (22 ± 2) mg/ml, vorzugsweise in einem Bereich von (4 ± 0,5) mg/ml bis (20 ± 2) mg/ml, aufweist.

Insbesondere kann es erfindungsgemäß dabei vorgesehen sein, dass die Zusammensetzung das Chondroitinsulfat bzw. das physiologisch verträgliche Chondroitinsulfat-Salz (Komponente (a)) in einer Konzentration von (20 ± 1,5) mg/ml, insbesondere in einer Konzentration von (20 ± 1) mg/ml, vorzugsweise in einer Konzentration von (20 ± 0,5) mg/ml, besonders bevorzugt in einer Konzentration von etwa 20 mg/ml, aufweist bzw. enthält.

Gleichermaßen kann die Zusammensetzung gemäß einer alternativen Ausführungsform das Chondroitinsulfat und/oder das physiologisch verträgliche Chondroitinsulfat-Salz (Komponente (a)) in einer Konzentration von (4,5 ± 0,4) mg/ml, insbesondere in einer Konzentration von (4,5 ± 0,3) mg/ml, vorzugsweise in einer Konzentration von (4,5 ± 0,15) mg/ml, besonders bevorzugt in einer Konzentration von etwa 4,5 mg/ml, aufweisen.

Insbesondere hat die Anmelderin gefunden, dass, wie zuvor angeführt, in Bezug auf die in Rede stehenden Konzentrationsbereiche - insbesondere auch im Zusammenwirken mit der Komponente (b) - ein entsprechendes Stabilitäts- und Wirksamkeitsmaximum im Hinblick auf die erfindungsgemäße Zusammensetzung vorliegt.

Darüber hinaus kommt auch dem Molekulargewicht (synonym auch als "Molmasse" bezeichnet) der Komponente (a) eine hohe Bedeutung zu:
So kann es erfindungsgemäß vorgesehen sein, dass das Chondroitinsulfat bzw. das physiologisch verträgliche Chondroitinsulfat-Salz (Komponente (a)) ein zahlenmittleres Molekulargewicht (Molmasse) Mₙ im Bereich von 2 kDa bis 200 kDa, insbesondere im Bereich von 5 kDa bis 150 kDa, vorzugsweise im Bereich von 10 kDa bis 100 kDa, bevorzugt im Bereich von 12 kDa bis 75 kDa, aufweist.

Insbesondere kann das Chondroitinsulfat bzw. das physiologisch verträgliche Chondroitinsulfat-Salz (Komponente (a)) ein gewichtsmittleres Molekulargewicht (Molmasse) M_{w} im Bereich von 10 kDa bis 200 kDa, insbesondere im Bereich von 15 kDa bis 175 kDa, vorzugsweise im Bereich von 20 kDa bis 150 kDa, bevorzugt im Bereich von 30 kDa bis 120 kDa, aufweisen.

Zudem kann das Chondroitinsulfat bzw. das physiologisch verträgliche Chondroitinsulfat-Salz (Komponente (a)) ein zentrifugenmittleres Molekulargewicht (Molmasse) M_{z} im Bereich von 30 kDa bis 1.000 kDa, insbesondere im Bereich von 40 kDa bis 800 kDa, vorzugsweise im Bereich von 50 kDa bis 600 kDa, bevorzugt im Bereich von 100 kDa bis 450 kDa, aufweisen.

In diesem Zusammenhang kommt auch dem Polydispersitätsindex (PDI) eine entsprechende Bedeutung zu: Diesbezüglich kann es erfindungsgemäß vorgesehen sein, dass das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ (M_{w}: Mₙ), von mindestens 1, insbesondere mindestens 1,2, vorzugsweise mindestens 1,5, bevorzugt mindestens 2, aufweist.

Zudem kann das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, von höchstens 30, insbesondere höchstens 20, vorzugsweise höchstens 10, bevorzugt höchstens 8, aufweisen.

Weiterhin kann das Chondroitinsulfat bzw. das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, im Bereich von 1 bis 30, insbesondere im Bereich von 1,2 bis 20, vorzugsweise im Bereich von 1,5 bis 10, bevorzugt im Bereich von 2 bis 8, aufweisen.

Die vorgenannten Eigenschaften der Komponente (a) hinsichtlich des Molekulargewichts bzw. der Molmasse bzw. des diesbezüglichen Polydispersitätsindexes führen - ohne sich auf diese Theorie beschränken oder festlegen zu wollen - zu einer weiterführend verbesserten Ausbildung der der erfindungsgemäßen Zusammensetzung zugrundeliegenden und insbesondere auf Basis der Komponenten (a) und (b) resultierende Matrix, insbesondere auch im Hinblick auf die Ausbildung eines Hydrogels, mit entsprechender Stabilisierung der jeweiligen Wirk- bzw. Inhaltsstoffe. Gleichermaßen führen die in Rede stehenden Molekulargewichte bzw. Molmassen in Bezug auf Komponente (a) auch zu einer guten Wirkeffizienz der erfindungsgemäßen Zusammensetzung im Hinblick auf die zugrundeliegende Behandlung der Nebenwirkungen einer BCG-Therapie bzw. den Einsatz als entsprechendes Co-Therapeutikum, insbesondere - gleichermaßen ohne sich auf diese Theorie beschränken oder festlegen zu wollen - infolge einer guten bzw. optimalen Wechselwirkung mit bzw. Anhaftung an dem Urothel der Harnblase.

Ohne sich diesbezüglich auf eine Theorie beschränken bzw. festlegen zu wollen, eignet sich dieses spezielle Chondroitinsulfat aufgrund seiner molekularen Struktur in besonderer Weise, um - insbesondere in Verbindung mit der Komponente (b) - die Glykosaminoglykanschicht des Urothels zu regenerieren bzw. gewissermaßen aufzufüllen, wodurch die Permeabilität dieser Schicht erniedrigt wird. Hierdurch wird die Schutzfunktion der Glykosaminoklykanschicht bzw. der Schleimschicht des Urothels (Mucin-Layer) erhöht, so dass es zu einer deutlichen Linderung der Nebenwirkungen kommen kann, und zwar ohne den therapeutischen Effekt einer BCG-Zusammensetzung als solcher nachhaltig negativ zu beeinflussen, wie zuvor angeführt.

Was die Bestimmung des Molekulargewichts der erfindungsgemäß eingesetzten Komponente (a) anbelangt, so wird das zahlenmittlere Molekulargewicht Mₙ bzw. das gewichtsmittlere Molekulargewicht M_{w} bzw. das zentrifugenmittlere Molekulargewicht M_{z} und/oder der Polydispersitätsindex (PDI) des Chondroitinsulfats bzw. des physiologisch verträglichen Chondroitinsulfat-Salzes (Komponente (a)) insbesondere mittels Gelpermeationschromatographie (GPC) bzw. gemäß der DIN 55672-3:2016-03 bestimmt.

Insbesondere können das zahlenmittlere Molekulargewicht Mₙ bzw. das gewichtsmittlere Molekulargewicht M_{w} bzw. das zentrifugenmittlere Molekulargewicht M_{z} bzw. der Polydispersitätsindex (PDI) des Chondroitinsulfats bzw. des physiologisch verträglichen Chondroitinsulfat-Salzes (Komponente (a)) mittels Gelpermeationschromatographie (GPC), insbesondere bei einer Temperatur im Bereich von 20 °C bis 40 °C bzw. mit 0,1 mol/l NaCl-Lösung in deionisiertem Wasser als Elutionsmittel und/oder an einer Lösung von Chondroitinsulfat und/oder des physiologisch verträglichen Chondroitinsulfat-Salzes mit einer Konzentration von 3 g/l und/oder unter Verwendung eines Dextran/Pullulan-Standards als Kalibrierreagenz und/oder gemäß DIN 55672-3:2016-03, bestimmt sein.

In erfindungsgemäß bevorzugter Weise kann das Chondroitinsulfat bzw. das physiologisch verträgliche Chondroitinsulfat-Salz (Komponente (a)) marinen Ursprungs sein. Diesbezüglich kann das Chondroitinsulfat bzw. das physiologisch verträgliche Chondroitinsulfat-Salz (Komponente (a)) aus Knorpelfischen, insbesondere Haien, vorzugsweise Haiknorpel, gewonnen sein. Die Anmelderin hat diesbezüglich völlig überraschend gefunden, dass ein derartiges Chondroitinsulfat marinen Ursprungs insbesondere bei der Behandlung von Nebenwirkungen, wie zuvor definiert, zu besonders guten Ergebnissen führt, insbesondere wenn dies in zweckgerichteter Kombination mit der Hyaluronsäure gemäß Komponente (b) eingesetzt wird. Die diesbezügliche Isolierung der Wirksubstanzen ist dem Fachmann als solche geläufig, so dass es diesbezüglich keiner weiterführender Ausführungen bedarf.

Erfindungsgemäß ist es insbesondere vorgesehen, dass das Chondroitinsulfat bzw. das physiologisch verträgliche Chondroitinsulfat-Salz (Komponente (a)) in Form eines Alkalisalzes, vorzugsweise in Form eines Natrium-Salzes, vorliegt, was im Rahmen der vorliegenden Erfindung zu besonders guten Ergebnissen führt.

Erfindungsgemäß kann das Chondroitinsulfat bzw. das physiologisch verträgliche Chondroitinsulfat-Salz (Komponente (a)) vorzugsweise in Form von Chondroitinsulfat-Natrium vorliegen.

Erfindungsgemäß kann das Chondroitinsulfat bzw. das physiologisch verträgliche Chondroitinsulfat-Salz (Komponente (a)) zudem ausgewählt sein aus der Gruppe von Chondroitin-4-sulfat, Chondroitin-6-sulfat, Chondroitin-2,6-sulfat, Chondroitin-4,6-sulfat und deren Kombinationen oder Mischungen, vorzugsweise Chondroitin-2,6-sulfat (Chondroitinsulfat D). Auch die vorgenannten speziellen Chondroitinsulfate werden erfindungsgemäß in bevorzugter Art und Weise in Form ihrer Alkalisalze, vorzugsweise Natriumsalze, eingesetzt, was mit besonders guten Ergebnissen hinsichtlich der erfindungsgemäßen Verwendung einhergeht.

Im Rahmen der vorliegenden Erfindung kann die Komponente (a) der erfindungsgemäßen Zusammensetzung vorzugsweise auf einer sterilen und/oder hochgereinigten Lösung oder Suspension insbesondere des Natriumsalzes von Chondroitinsulfat basieren.

Im Rahmen der vorliegenden Erfindung einsetzbares Chondroitinsulfat bzw. Chondroitinsulfat-Salz ist im Allgemeinen kommerziell erhältlich, beispielsweise von Nexira, Rouen (FR), Artesan Pharma GmbH & Co. KG oder von Pharma Greven GmbH, Greven (DE).

Für weitergehende Einzelheiten zum Begriff der Chondroitinsulfate kann auf RÖMPP Chemielexikon, 10. Auflage, Band 1, 1996, Georg Thieme Verlag Stuttgart/ New York, Seite 736, Stichwort: "Chondroitinsulfate", sowie auf die dort referierte Literatur verwiesen werden, wobei der gesamte Offenbarungsgehalt der vorgenannten Literatur hiermit durch Bezugnahme vollumfänglich eingeschlossen ist.

Was die weitere Komponente in Form der Hyaluronsäure bzw. des physiologisch verträglichen Hyaluronsäure-Salzes gemäß Komponente (b) anbelangt, welche für die erfindungsgemäße Zusammensetzung eingesetzt wird, so ist diesbezüglich insbesondere Folgendes anzuführen:
In diesem Zusammenhang kann es erfindungsgemäß im Allgemeinen vorgesehen sein, dass die Zusammensetzung die Hyaluronsäure bzw. das physiologisch verträgliche Hyaluronsäure-Salz (Komponente (b)) in einer Konzentration in einem Bereich von (2 ± 0,5) mg/ml bis (18 ± 2) mg/ml, vorzugsweise in einem Bereich von (3 ± 0,5) mg/ml bis (16 ± 2) mg/ml, aufweist.

Erfindungsgemäß kann es im Speziellen zudem vorgesehen sein, dass die Zusammensetzung die Hyaluronsäure bzw. das physiologisch verträgliche Hyaluronsäure-Salz (Komponente (b)) in einer Konzentration von (16 ± 1,2) mg/ml, insbesondere in einer Konzentration von (16 ± 0,8) mg/ml, vorzugsweise in einer Konzentration von (16 ± 0,4) mg/ml, besonders bevorzugt in einer Konzentration von etwa 16 mg/ml, enthält. In den vorgenannten Konzentrationsbereichen werden besonders gute Eigenschaften im Hinblick auf Stabilität und Wirksamkeit erhalten, insbesondere auf Basis eines Zusammenwirkens insbesondere mit den Komponenten (a) und/oder gegebenenfalls (c) sowie gegebenenfalls vorliegenden speziellen pH-Wert der Zusammensetzung nach der Erfindung.

Insbesondere im Hinblick auf die Stabilität, vorzugsweise Lagerungsstabilität, der erfindungsgemäßen Zusammensetzung sowie deren Wirksamkeit bei der Behandlung der Nebenwirkungen einer BCG-Therapie werden zudem besonders gute Ergebnisse erreicht, wenn die Hyaluronsäure ein spezielles Molekulargewicht bzw. eine spezielle Molmasse aufweist.

In diesem Zusammenhang ist es erfindungsgemäß bevorzugt, wenn die Hyaluronsäure bzw. das physiologisch verträgliche Hyaluronsäure-Salz (Komponente (b)) ein zahlenmittleres Molekulargewicht (Molmasse) Mₙ im Bereich von 10 kDa bis 300 kDa, insbesondere im Bereich von 20 kDa bis 275 kDa, vorzugsweise im Bereich von 30 kDa bis 260 kDa, bevorzugt im Bereich von 50 kDa bis 250 kDa, besonders bevorzugt im Bereich von 75 kDa bis 200 kDa, aufweist.

Insbesondere kann die Hyaluronsäure bzw. das physiologisch verträgliche Hyaluronsäure-Salz (Komponente (b)) ein gewichtsmittleres Molekulargewicht (Molmasse) M_{w} im Bereich von 10 kDa bis 500 kDa, insbesondere im Bereich von 20 kDa bis 450 kDa, vorzugsweise im Bereich von 50 kDa bis 425 kDa, bevorzugt im Bereich von 100 kDa bis 400 kDa, besonders bevorzugt im Bereich von 150 kDa bis 395 kDa, aufweisen.

Darüber hinaus kann die Hyaluronsäure bzw. das physiologisch verträgliche Hyaluronsäure-Salz (Komponente (b)) ein zentrifugenmittleres Molekulargewicht (Molmasse) M_{z} im Bereich von 80 kDa bis 1.500 kDa, insbesondere im Bereich von 100 kDa bis 1.250 kDa, vorzugsweise im Bereich von 200 kDa bis 1.000 kDa, bevorzugt im Bereich von 300 kDa bis 750 kDa, aufweisen.

Auf Basis der vorgenannten Molekulargewichte resultiert insbesondere in zweckgerichteter Abstimmung und Kombination mit den entsprechenden Molekulargewichten der Komponente (a) eine besonders gute Stabilisierung und Wirkeffizienz der erfindungsgemäßen Zusammensetzung, insbesondere da - ohne sich auf diese Theorie beschränken bzw. festlegen zu wollen - auf Basis der vorgenannten Molekulargewichte eine optimale Matrix- bzw. Hydrogelausbildung mit entsprechender Stabilisierung und weiterführend optimierter Wechselwirkung insbesondere mit dem Urothel der Harnblase gewährleistet ist. Dies ist gleichermaßen auch der Wirksamkeit der Zusammensetzung nach der Erfindung zuträglich.

Diesbezüglich kommt auch dem Polydispersitätsindex der erfindungsgemäß eingesetzten Komponente (b) eine Bedeutung zu:
So kann es erfindungsgemäß vorgesehen sein, dass die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von mindestens 1, insbesondere mindestens 1,1, vorzugsweise mindestens 1,2, bevorzugt mindestens 1,3, besonders bevorzugt mindestens 1,4, aufweist.

Insbesondere kann die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von höchstens 50, insbesondere höchstens 25, vorzugsweise höchstens 10, bevorzugt höchstens 5, besonders bevorzugt höchstens 3, aufweisen.

Weiterhin kann es erfindungsgemäß vorgesehen sein, dass die Hyaluronsäure bzw. das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, im Bereich von 1 bis 50, insbesondere im Bereich von 1,1 bis 25, vorzugsweise im Bereich von 1,2 bis 10, bevorzugt im Bereich von 1,3 bis 5, besonders bevorzugt im Bereich von 1,4 bis 3, aufweist.

Die vorgenannten Molekulargewichte können mit dem Fachmann an sich bekannten Methoden bestimmt werden. Erfindungsgemäß kann das zahlenmittlere Molekulargewicht Mₙ bzw. das gewichtsmittlere Molekulargewicht M_{w} und/oder das zentrifugenmittlere Molekulargewicht M_{z} und/oder der Polydispersitätsindex (PDI) der Hyaluronsäure und/oder des physiologisch verträglichen Hyaluronsäure-Salzes (Komponente (b)) insbesondere mittels Gelpermeationschromatographie (GPC) und/oder gemäß DIN 55672-3:2016-03 bestimmt sein.

Zudem kann das zahlenmittlere Molekulargewicht Mₙ bzw. das gewichtsmittlere Molekulargewicht M_{w} bzw. das zentrifugenmittlere Molekulargewicht M_{z} bzw. der Polydispersitätsindex (PDI) der Hyaluronsäure und/oder des physiologisch verträglichen Hyaluronsäure-Salzes (Komponente (b)) jeweils mittels Gelpermeationschromatographie (GPC), insbesondere bei einer Temperatur im Bereich von 20 °C bis 40 °C und/oder mit 0,1 mol/l NaCl-Lösung in deionisiertem Wasser als Elutionsmittel und/oder an einer Lösung von Hyaluronsäure und/oder des physiologisch verträglichen Hyaluronsäure-Salzes mit einer Konzentration von 3 g/l und/oder unter Verwendung eines Dextran/Pullulan-Standards als Kalibrierreagenz und/oder gemäß DIN 55672-3:2016-03, bestimmt sein.

Erfindungsgemäß kann es insbesondere vorgesehen sein, dass das Verhältnis des jeweiligen Molekulargewichts Mₙ, M_{w} oder M_{z} von Chondroitinsulfat bzw. des physiologisch verträglichen Chondroitinsulfat-Salzes (Komponente (a)) zu dem korrespondierenden Molekulargewicht Mₙ, M_{w} oder M_{z} der Hyaluronsäure und/oder des physiologisch verträglichen Hyaluronsäure-Salzes (Komponente (b)) in einem Bereich von 1 : 3 bis 1 : 4, insbesondere 1 : 1 bis 1 : 100, vorzugsweise 1 : 1,5 bis 1 : 50, bevorzugt 1 : 2 bis 1 : 25, besonders bevorzugt 1 : 3 bis 1 : 10, liegt. Infolge der gezielten Abstimmung der jeweiligen Molekulargewichte bzw. Molmassen der Komponente (a) und der Komponente (b) ergeben sich besonders gute Ergebnisse hinsichtlich der Stabilisierung sowie Wirkeffizienz der erfindungsgemäßen Zusammensetzung, insbesondere da - ohne sich auf diese Theorie beschränken bzw. festlegen zu wollen - diesbezüglich eine Wirkergänzung bzw. hohe Kompatibilität der in Rede stehenden Komponenten (a) und (b) untereinander vorliegt.

Erfindungsgemäß kann es zudem insbesondere vorgesehen sein, dass die Hyaluronsäure bzw. das physiologisch verträgliche Hyaluronsäure-Salz (Komponente (b)) nichttierischen Ursprungs ist.

In diesem Zusammenhang kann die Hyaluronsäure bzw. das physiologisch verträgliche Hyaluronsäure-Salz (Komponente (b)) bakteriellen bzw. fermentativen Ursprungs sein. Diesbezüglich kann die Hyaluronsäure bzw. das physiologisch verträgliche Hyaluronsäure-Salz (Komponente (b)) vorzugsweise fermentativ von Bakterien der Gattung *Streptococcus,* insbesondere *Streptococcus lancefields,* bevorzugt *Streptococcus lancefields* Stamm A, gewonnen sein. Die diesbezügliche Gewinnung bzw. Isolierung der Wirksubstanzen ist dem Fachmann als solche geläufig, so dass es diesbezüglich keiner weiterführender Ausführungen bedarf.

In diesem Zusammenhang hat die Anmelderin gleichermaßen in überraschender Weise herausgefunden, dass durch die Verwendung einer nichttierischen Hyaluronsäure der vorgenannten Art auch die pharmazeutische Wirksamkeit der erfindungsgemäßen Zusammensetzung in Bezug auf die Behandlung von mit einer BCT-Therapie einhergehenden Nebenwirkungen bzw. in Bezug auf die Verwendung als Co-Therapeutikum signifikant verbessert ist. Ohne sich auf eine spezielle Theorie beschränken bzw. festlegen zu wollen, kann dies damit begründet werden, dass es sich bei der nichttierischen, insbesondere bakteriell gewonnenen Hyaluronsäure um ein besonders reines Produkt mit definierten chemischen bzw. physikalischen Eigenschaften handelt, welches hochwirksam ist. Die definierte Ausbildung der Hyaluronsäure mit der hohen Reinheit ist gleichermaßen auch der Stabilität der Zusammensetzung zuträglich, da keine der Stabilität abträglichen Verunreinigungen zugegen sind. Zudem ist die nichttierische Hyaluronsäure gut verträglich, da eine Kontamination bzw. Verunreinigung mit anderen Stoffen, wie es oftmals bei tierisch gewonnenen Produkten der Fall ist, nicht vorliegt. Somit weist Hyaluronsäure nichttierischen Ursprungs eine besonders hohe Reinheit und Homogenität auf, was auch der Stabilität, insbesondere Lagerungsstabilität, der erfindungsgemäßen Zusammensetzung zuträglich ist.

Gemäß einer erfindungsgemäß bevorzugten Ausführungsform kann die Hyaluronsäure bzw. das physiologisch verträgliche Hyaluronsäure-Salz (Komponente (b)) in Form eines Alkalisalzes, vorzugsweise in Form eines Natrium-Salzes, und/oder in Form eines Alkalihyaluronates vorliegen. Dabei werden gleichermaßen besonders gute Ergebnisse erhalten, wenn die Hyaluronsäure bzw. das physiologisch verträgliche Hyaluronsäure-Salz (Komponente (b)) in Form von Natriumhyaluronat vorliegt.

Die Komponente (b) der erfindungsgemäßen Zusammensetzung kann insbesondere auf Basis einer sterilen bzw. hochgereinigten Lösung oder Suspension insbesondere des Natriumsalzes der Hyaluronsäure eingesetzt werden.

Im Rahmen der vorliegenden Erfindung einsetzbare Hyaluronsäure bzw. einsetzbares Hyaluronsäure-Salz ist im Allgemeinen kommerziell erhältlich, beispielsweise von Vivatis Pharma Gmbh, Hamburg (DE), Contipro S.A., Dolni Dobrouc (CZ) oder von GFN Herstellung von Naturextrakten GmbH, Wald-Michelbach (DE).

Für weitergehende Einzelheiten zum Begriff der Hyaluronsäure bzw. deren physiologisch verträglichen Salze kann auf RÖMPP Chemielexikon, 10. Auflage, Band 3, 1997, Georg Thieme Verlag Stuttgart/New York, Seite 1820, Stichwort: "Hyaluronsäure", sowie auf die dort referierte Literatur verwiesen werden, wobei der Gesamtoffenbarungsgehalt der vorgenannten Literatur hiermit durch Bezugnahme vollumfänglich eingeschlossen ist.

Im Allgemeinen kann es erfindungsgemäß vorgesehen sein, dass das Chondroitinsulfat bzw. das physiologisch verträgliche Chondroitinsulfat-Salz (Komponente (a)) einerseits und die Hyaluronsäure und/oder das physiologisch verträgliche Hyaluronsäure-Salz (Komponente (b)) andererseits in der Zusammensetzung in einem Gewichtsverhältnis (Konzentrationsverhältnis) von Komponente (a) einerseits zu Komponente (b) andererseits [(a) : (b)] in einem Bereich von 2 : 1 bis 1 : 5, insbesondere 1,6 : 1 bis 1 : 4, vorzugsweise 1,2 : 1 bis 1 : 3,8, vorliegen.

Erfindungsgemäß kann es zudem vorgesehen sein, dass das Chondroitinsulfat bzw. das physiologisch verträgliche Chondroitinsulfat-Salz (Komponente (a)) einerseits und die Hyaluronsäure und/oder das physiologisch verträgliche Hyaluronsäure-Salz (Komponente (b)) andererseits in der Zusammensetzung in einem Gewichtsverhältnis (Konzentrationsverhältnis) von Komponente (a) einerseits zu Komponente (b) andererseits [(a) : (b)] in einem Bereich von 1 : 1 bis 1,6 : 1, insbesondere 1,1 : 1 bis 1,5 : 1, vorzugsweise 1,2 : 1 bis 1,4 : 1, besonders bevorzugt etwa 1,25 : 1, vorliegen.

Gemäß einer alternativen Ausführungsform kann es zudem jedoch auch vorgesehen sein, dass das Chondroitinsulfat bzw. das physiologisch verträgliche Chondroitinsulfat-Salz (Komponente (a)) einerseits und die Hyaluronsäure bzw. das physiologisch verträgliche Hyaluronsäure-Salz (Komponente (b)) andererseits in der Zusammensetzung in einem Gewichtsverhältnis (Konzentrationsverhältnis) von Komponente (a) einerseits zu Komponente (b) andererseits [(a) : (b)] in einem Bereich von 1 : 3 bis 1 : 4, insbesondere 1 : 3,2 bis 1 : 3,8, vorzugsweise 1 : 3,4 bis 1 : 3,6, besonders bevorzugt etwa 1 : 3,55, vorliegen.

Denn der Anmelderin ist es insgesamt gelungen, durch die gezielte Anpassung und Abstimmung der jeweiligen Wirksubstanzen auf Basis der Komponenten (a) und (b) in Bezug auf die Glykosaminoglykanschicht des Blasenurothels zu einer besonders guten Wirksamkeit hinsichtlich der Behandlung bzw. Verringerung der zugrundeliegenden Nebenwirkungen zu gelangen, da - ohne sich auf eine spezielle Theorie beschränken bzw. festlegen zu wollen - insbesondere bei den vorgenannten Mengenverhältnissen eine besonders gute Wechselwirkung bzw. Integration der Wirksubstanzen in die Glykosaminoglykanschicht des Blasenurothels bzw. eine gute Regeneration der Glykosaminoglykanschicht des Blasenurothels vorliegt, insbesondere in Verbindung mit den vorgenannten jeweils speziellen Molekulargewichten bzw. Molmassen der Komponenten (a) bzw. (b). Folglich wird, wie zuvor angeführt, die Permeabilität des Urothels in signifikanter Weise erniedrigt, was zu einer deutlichen Minderung der mit den Nebenwirkungen einhergehenden Symptome verbunden ist, insbesondere da andersartige Reizstoffe nicht mehr so tief in das Urothel bzw. in darunterliegende Schichten eindringen können. Die vorgenannten Gewichtsverhältnisse haben auch einen positiven Effekt in Bezug auf die Stabilität der Zusammensetzung gemäß dem chemischen Puffersystem.

Was darüber hinaus die erfindungsgemäß gegebenenfalls eingesetzte Komponente (c) anbelangt, so kann die Zusammensetzung das Puffersystem, insbesondere das Dihydrogenphosphat/Monohydrogenphosphat-Puffersystem, (Komponente (c)) in einer Gesamtkonzentration an Puffersystem, insbesondere an Dihydrogenphosphat/Monohydrogenphosphat-Puffersystem, (Komponente (c)) von (1,75 ± 1,65) mg/ml, insbesondere in einer Gesamtkonzentration von (1,75 ± 1,5) mg/ml, vorzugsweise in einer Gesamtkonzentration von (1,75 ± 1,25) mg/ml, bevorzugt in einer Gesamtkonzentration von (1,75 ± 1) mg/ml, besonders bevorzugt in einer Gesamtkonzentration von (1,75 ± 0,9) mg/ml, ganz besonders bevorzugt in einer Gesamtkonzentration von (1,75 ± 0,8) mg/ml, noch weiter bevorzugt in einer Gesamtkonzentration von etwa 1,75 mg/ml, enthalten.

Im Rahmen der vorliegenden Erfindung hat sich der gezielte Einsatz eines speziellen Puffersystems, insbesondere chemischen Puffersystems, gemäß Komponente (c) insbesondere auf Basis des Dihydrogenphosphat/Monohydrogenphosphat-Puffersystems als besonders vorteilhaft erwiesen. Bei Verwendung dieses speziellen Puffersystems, insbesondere bei gleichzeitiger Einhaltung der entsprechenden Konzentrationen des Puffers bzw. der Pufferkomponenten, wird einer unerwünschten Veränderung des pH-Wertes bzw. einem unerwünschten Abbau der Wirkstoffe bei Lagerung auch über einen längeren Zeitraum in besonderem Maße entgegengewirkt und wird die Lagerfähigkeit der erfindungsgemäßen Zusammensetzung somit in besonderem Maße verbessert, wie zuvor angeführt.

Insbesondere kann die Zusammensetzung das Puffersystem, insbesondere das Dihydrogenphosphat/Monohydrogenphosphat-Puffersystem, (Komponente (c)) in einer Gesamtmenge an Puffersystem, insbesondere an Dihydrogenphosphat/Monohydrogenphosphat-Puffersystem, (Komponente (c)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, enthalten.

Zudem kann die Zusammensetzung das Puffersystem, insbesondere das Dihydrogenphosphat des Dihydrogenphosphat/Monohydrogenphosphat-Puffersystems, (Komponente (c)), insbesondere bezogen auf die Einwaage bei Herstellung und/oder Bereitstellung der Zusammensetzung, in einer Konzentration von (0,2 ± 0,19) mg/ml, insbesondere in einer Konzentration von (0,2 ± 0,15) mg/ml, vorzugsweise in einer Konzentration von (0,2 ± 0,125) mg/ml, bevorzugt in einer Konzentration von (0,2 ± 0,1) mg/ml, besonders bevorzugt in einer Konzentration von etwa 0,2 mg/ml, enthalten.

Gleichermaßen hat es sich als vorteilhaft erwiesen, wenn die Zusammensetzung das Monohydrogenphosphat des Dihydrogenphosphat/Monohydrogenphosphat-Puffersystems (Komponente (c)), insbesondere bezogen auf die Einwaage bei Herstellung und/oder Bereitstellung der Zusammensetzung, in einer Konzentration von (1,5 ± 1,4) mg/ml, insbesondere in einer Konzentration von (1,5 ± 1,25) mg/ml, vorzugsweise in einer Konzentration von (1,5 ± 1,1) mg/ml, bevorzugt in einer Konzentration von (1,5 ± 1) mg/ml, besonders bevorzugt in einer Konzentration von (1,5 ± 0,75) mg/ml, ganz besonders bevorzugt in einer Konzentration von etwa 1,5 mg/ml, enthält.

Hinsichtlich der Stabilisierung der erfindungsgemäßen Zusammensetzung hat es sich zudem als besonders vorteilhaft erwiesen, wenn die Zusammensetzung das Dihydrogenphosphat/Monohydrogenphosphat-Puffersystem (Komponente (c)) in einem Gewichtsverhältnis (Konzentrationsverhältnis) von Dihydrogenphosphat zu Monohydrogenposphat [Dihydrogenphosphat: Monohydrogenposphat], insbesondere bezogen auf die Einwaage bei Herstellung und/oder Bereitstellung der Zusammensetzung, im Bereich von 2 : 1 bis 1 : 100, vorzugsweise im Bereich von 1 : 1 bis 1 : 75, bevorzugt im Bereich von 1 : 2 bis 1 : 50, besonders bevorzugt im Bereich von 1 : 5 bis 1 : 25, enthält.

Gemäß einer erfindungsgemäßen Ausführungsform kann das Dihydrogenphosphat/Monohydrogenphosphat-Puffersystem (Komponente (c)) als Alkalidihydrogenphosphat/Alkalimonohydrogenposphat-Puffersystem vorliegen bzw. ausgebildet sein. In bevorzugter Weise kann das Alkalimetall ausgewählt sein aus Natrium und/oder Kalium, insbesondere Natrium.

Zudem kann das Dihydrogenphosphat/Monohydrogenphosphat-Puffersystem (Komponente (c)) als Natriumdihydrogenphosphat/Natriummonohydrogenposphat-Puffersystem, vorliegen bzw. ausgebildet sein.

Darüber hinaus kann das Dihydrogenphosphat/Monohydrogenphosphat-Puffersystem (Komponente (c)) als NaH₂(PO₄) / Na₂H(PO₄)-Puffersystem, insbesondere als NaH₂(PO₄) · 2 H₂O / Na₂H(PO₄) · 2 H₂O-Puffersystem, vorliegen bzw. ausgebildet sein.

Im Allgemeinen kann das Puffersystem, insbesondere das Dihydrogenphosphat/Monohydrogenphosphat-Puffersystem, (Komponente (c)) zur Einstellung bzw. Konstanthaltung des pH-Wertes der Zusammensetzung dienen bzw. eingesetzt sein. Hierdurch erfolgt eine weiterführende Stabilisierung der erfindungsgemäßen Zusammensetzung, einhergehend mit einer besonders guten Verträglichkeit der Zusammensetzung nach der Erfindung bei deren Anwendung bzw. Applikation.

Im Rahmen der vorliegenden Erfindung einsetzbare Phosphatpuffer sind im Allgemeinen kommerziell erhältlich, beispielsweise von Merck KGaA, Darmstadt (DE).

Zum Begriff des Puffers bzw. des chemischen Puffersystems kann insbesondere verwiesen werden auf RÖMPP Lexikon Chemie, 10. Auflage, Georg-Thieme-Verlag, Stuttgart/New York, Band 5, 1998, Seiten 3618/3619, Stichwort: "Puffer", sowie auf die dort referierte Literatur, wobei der gesamte Offenbarungsgehalt der vorgenannten Literatur hiermit durch Bezugnahme vollumfänglich eingeschlossen ist.

In Ergänzung zu den vorgenannten Wirksubstanzen bzw. Inhaltsstoffen kann die erfindungsgemäße Zusammensetzung gemäß Komponente (d) mindestens einen physiologisch verträglichen Elektrolyten aufweisen:
In diesem Zusammenhang kann die Zusammensetzung den Elektrolyten (Komponente (d)) in einer Konzentration von (8 ± 6) mg/ml, insbesondere in einer Konzentration von (8 ± 4) mg/ml, vorzugsweise in einer Konzentration von (8 ± 2) mg/ml, bevorzugt in einer Konzentration von (8 ± 1) mg/ml, besonders bevorzugt in einer Konzentration von etwa 8 mg/ml, enthalten.

Insbesondere kann der Elektrolyt (Komponente (d)) in Form eines Alkalisalzes, insbesondere in Form eines Alkalichlorids, vorzugsweise in Form von Natriumchlorid, vorliegen. Erfindungsgemäß ist es dabei bevorzugt, wenn der Elektrolyt (Komponente (d)) Natriumchlorid ist.

Im Rahmen der vorliegenden Erfindung einsetzbare Elektrolyte sind im Allgemeinen kommerziell erhältlich, beispielsweise von Merck KGaA, Darmstadt (DE).

Infolge der Verwendung eines Elektrolyten können mögliche osmotische Effekte im Rahmen der Applikation bzw. Behandlung mit der erfindungsgemäßen Zusammensetzung so gering wie möglich gehalten werden, was die Verträglichkeit der erfindungsgemäßen Zusammensetzung weiter erhöht.

Gemäß einer erfindungsgemäßen Ausführungsform kann die Zusammensetzung außerdem (e) BCG (*Bacillus-Calmette-Guérin*), insbesondere in Form von lebensfähigen Einheiten, (Komponente (e)) enthalten, insbesondere in Mengen von 10⁵ bis 10¹⁰ lebensfähigen Einheiten, insbesondere 10⁶ bis 10⁹ lebensfähigen Einheiten, vorzugsweise 10⁷ bis 10⁹ lebensfähigen Einheiten, insbesondere bezogen auf das Gesamtvolumen der zu verabreichenden Zusammensetzung bzw. pro Dosiseinheit der zu verabreichenden Zusammensetzung (z. B. in 50 ml der Zusammensetzung nach der Erfindung).

Was den pH-Wert der erfindungsgemäßen Zusammensetzung im Allgemeinen anbelangt, so kann die Zusammensetzung zudem einen pH-Wert in einem Bereich von 6,1 bis 7,9, insbesondere in einem Bereich von 6,6 bis 7,7, vorzugsweise in einem Bereich von 6,9 bis 7,6, bevorzugt in einem Bereich von 7,1 bis 7,4, aufweisen. Insbesondere kann der pH-Wert der Zusammensetzung in einem Bereich von 6,1 bis 7,9, insbesondere in einem Bereich von 6,6 bis 7,7, insbesondere im Bereich von 6,9 bis 7,6, vorzugsweise im Bereich von 7,1 bis 7,4, konstant gehalten bzw. eingestellt sein. Der pH-Wert kann vorzugsweise mittels des Puffersystems, insbesondere des Alkalidihydrogenphosphat/Alkalimonohydrogenposphat-Puffersystems, (Komponente (c)) eingestellt bzw. vorgegeben sein.

Bei Einhaltung der vorgenannten pH-Wertebereiche wird einem unerwünschten Abbau der Wirkstoffe bei Lagerung auch über einen längeren Zeitraum in besonderem Maße entgegengewirkt und wird auch die Lagerfähigkeit der erfindungsgemäßen Zusammensetzung in besonderem Maße verbessert.

Aufgrund der vorgenannten Eigenschaften im Hinblick auf den pH-Wert ist die erfindungsgemäße Zusammensetzung zudem besonders gut verträglich.

Im Rahmen der vorliegenden Erfindung kann die Bestimmung des pH-Wertes mit dem Fachmann an sich bekannten Methoden erfolgen. Insbesondere kann die Bestimmung des pH-Wertes auf Basis einer potentiometrischen Analyse erfolgen. Vorzugsweise kann die Bestimmung des pH-Wertes gemäß der Methode nach Ph. Eur. [Pharmacopoea Europaea], 9th Edition (9.0), 2017, 9. Auflage Englisch, Abschnitt 2.2.3. "Potentiometric determination of pH" erfolgen.

Für weitergehende Einzelheiten zum Begriff des pH-Wertes kann insbesondere verwiesen werden auf RÖMPP Lexikon Chemie, 10. Auflage, Georg-Thieme-Verlag, Stuttgart/New York, Band 4, 1998, Seiten 3230 bis 3232, Stichwort: "pH", sowie auf die dort referierte Literatur, wobei der gesamte Offenbarungsgehalt der vorgenannten Literatur hiermit durch Bezugnahme vollumfänglich eingeschlossen ist.

Was die erfindungsgemäße Zusammensetzung weiterhin anbelangt, so kann diese bei einer Temperatur von 20 °C eine dynamische Viskosität von mindestens 2.000 mPas, insbesondere mindestens 4.000 mPas, vorzugsweise mindestens 5.000 mPas, bevorzugt mindestens 5.250 mPas, aufweisen. Zudem kann die Zusammensetzung bei einer Temperatur von 20 °C eine dynamische Viskosität von höchstens 7.900 mPas, insbesondere höchstens 6.900 mPas, vorzugsweise höchstens 6.000 mPas, bevorzugt höchstens 5.750 mPas, aufweisen.

Erfindungsgemäß kann es insbesondere vorgesehen sein, dass die Zusammensetzung bei einer Temperatur von 20 °C eine dynamische Viskosität im Bereich von 2.000 mPas bis 7.900 mPas, insbesondere im Bereich von 4.000 mPas bis 6.900 mPas, vorzugsweise im Bereich von 5.000 mPas bis 6.000 mPas, bevorzugt im Bereich von 5.250 mPas bis 5.750 mPas, aufweist.

Aufgrund der erfindungsgemäß vorgesehenen bzw. eingestellten Viskosität ist eine besonders einfache und weniger schmerzhafte Instillation der Zusammensetzung in die Harnblase gewährleistet. Weiterhin liegt aufgrund der erfindungsgemäß vorgesehenen Viskosität eine besonders gute Interaktion der der Zusammensetzung nach der Erfindung zugrundeliegenden Wirkkomponente mit dem Urothel vor, was die Wirksamkeit positiv beeinflusst. Zudem führt die gezielte Einstellung der Viskosität auch zu einer weiterführenden Stabilisierung der Zusammensetzung, insbesondere im Hinblick auf - ohne sich auf diese Theorie beschränken bzw. festlegen zu wollen - die Ausbildung einer definierten Matrix bzw. eines definierten Hydrogels, was mit einer Verbesserung der Lagerungsstabilität der erfindungsgemäßen Zusammensetzung einhergeht.

Die Bestimmung der dynamischen Viskosität kann mit dem Fachmann an sich bekannten Methoden erfolgen. Insbesondere kann die dynamische Viskosität gemäß der Methode nach Ph. Eur. [Pharmacopoea Europaea], 9th Edition (9.0), 2017, 9. Auflage Englisch, Abschnitt 2.2.8. "Viscosity" und Abschnitt 2.2.9. "Capillary viscometer method" bestimmt sein.

Weiterhin kann die erfindungsgemäße Zusammensetzung eine Osmolalität im Bereich von 150 mosmol/kg bis 600 mosmol/kg, insbesondere im Bereich von 200 mosmol/kg bis 550 mosmol/kg, vorzugsweise im Bereich von 250 mosmol/kg bis 500 mosmol/kg, bevorzugt im Bereich von 275 mosmol/kg bis 450 mosmol/kg, besonders bevorzugt im Bereich von 300 mosmol/kg bis 400 mosmol/kg, aufweisen. Die gezielte Einstellung der Osmolalität dient gleichermaßen der weiterführenden Stabilisierung und insbesondere Verbesserung der Verträglichkeit der erfindungsgemäßen Zusammensetzung.

Die Osmolalität kann dabei gemäß dem Fachmann an sich bekannten Methoden bestimmt werden. Insbesondere kann die Osmolalität gemäß der Methode nach Ph. Eur. [Pharmacopoea Europaea], 9th Edition (9.0), 2017, 9. Auflage Englisch, Abschnitt 2.2.35. "Osmolality", bestimmt sein. Die Einstellung der Osmolalität kann beispielsweise auch auf Basis des zuvor genannten Elektrolyten (Komponente (d)), beispielsweise auf Basis von Alkali-Ionen, wie Natrium-Ionen, und Chlorid-Ionen, insbesondere mittels Natriumchlorid, erfolgen.

Darüber hinaus kann die Zusammensetzung bei einer Temperatur von 20 °C und bei einem Druck von 1.013,25 mbar (Atmosphärendruck) eine Dichte im Bereich von 1,001 g/cm³ bis 1,5 g/cm³, insbesondere im Bereich von 1,005 g/cm³ bis 1,25 g/cm³, vorzugsweise im Bereich von 1,0075 g/cm³ bis 1,1 g/cm³, bevorzugt im Bereich von 1,0075 g/cm³ bis 1,075 g/cm³, besonders bevorzugt im Bereich von 1,0075 g/cm³ bis 1,05 g/cm³, aufweisen.

Insbesondere kann die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,005 bis 1,25, vorzugsweise im Bereich von 1,0075 bis 1,1, bevorzugt im Bereich von 1,0075 bis 1,075, besonders bevorzugt im Bereich von 1,0075 bis 1,05, aufweisen.

Im Rahmen der vorliegenden Erfindung kann die Bestimmung der Dichte mit dem Fachmann an sich bekannten Methoden erfolgen. Insbesondere kann die relative Dichte gemäß der Methode nach Ph. Eur. [Pharmacopoea Europaea], 9th Edition (9.0), 2017, 9. Auflage Englisch, Abschnitt 2.2.5. "Relative density" bestimmt sein. Durch die zweckgerichtete Einstellung der Dichte kann die Handhabung und Verträglichkeit der erfindungsgemäßen Zusammensetzung weiterführend verbessert werden.

Darüber hinaus ist es im Rahmen der vorliegenden Erfindung bevorzugt, wenn die Zusammensetzung als wässrige Zusammensetzung vorliegt. In diesem Zusammenhang kann es insbesondere vorgesehen sein, dass die Zusammensetzung wässrig basiert ist bzw. wässrig formuliert vorliegt.

Insbesondere kann die Zusammensetzung in Form einer wässrigen Lösung bzw. wässrigen Suspension, vorzugsweise in Form einer wässrigen Lösung, vorliegen.

In diesem Zusammenhang kann die Zusammensetzung Wasser, insbesondere gereinigtes Wasser, enthalten. Insbesondere kann die Zusammensetzung Wasser als pharmazeutisch verträglichen Träger (Exzipienten) enthalten. Im Allgemeinen kann die Zusammensetzung folglich wässrig basiert ausgebildet sein, insbesondere unter Verwendung von Wasser für Injektionszwecke.

Was die eingesetzte Menge an Wasser anbelangt, so kann diese in weiten Bereichen variieren. Erfindungsgemäß ist es bevorzugt, wenn die Zusammensetzung nach der Erfindung einen Gehalt an Wasser, insbesondere an gereinigtem Wasser, von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweist.

Im Rahmen der vorliegenden Erfindung einsetzbares Wasser, insbesondere Wasser für Injektionszwecke, ist im Allgemeinen kommerziell erhältlich, beispielsweise von Fresenius Kabi Deutschland GmbH, Bad Homburg (DE).

Gemäß einer erfindungsgemäßen Ausführungsform kann es vorgesehen sein, dass die Zusammensetzung nach der Erfindung aus den vorgenannten Komponenten (a), (b), gegebenenfalls (c), gegebenenfalls (d) und gegebenenfalls (e) sowie gegebenenfalls Wasser, insbesondere gereinigtem Wasser, insbesondere aus den vorgenannten Komponenten (a), (b), gegebenenfalls (c) und gegebenenfalls (d) sowie gegebenenfalls Wasser, besteht.

Darüber hinaus kann es erfindungsgemäß vorgesehen sein, dass die Zusammensetzung nach der Erfindung zumindest im Wesentlichen frei von organischen Lösemitteln und/oder organischen Dispersionsmitteln, insbesondere alkoholisch basierten Lösemitteln und/oder alkoholisch basierten Dispersionsmitteln, ist. Insbesondere kann es erfindungsgemäß vorgesehen sein, dass die Zusammensetzung zumindest im Wesentlichen frei von Alkoholen ist. Hierdurch wird die Verträglichkeit der erfindungsgemäßen Zusammensetzung weiterführend erhöht.

Nicht zuletzt aufgrund der hohen Stabilität, insbesondere Lagerungsstabilität, der erfindungsgemäßen Zusammensetzung kann es im Rahmen der vorliegenden Erfindung auch vorgesehen sein, dass die Zusammensetzung zumindest im Wesentlichen frei von Konservierungsmitteln bzw. zumindest im Wesentlichen frei von Desinfektionsmitteln ist. Auch hierdurch wird die Verträglichkeit der erfindungsgemäßen Zusammensetzung verbessert, da erfindungsgemäß auf den Einsatz von auf das Urothel bzw. die Schleimhaut der Harnblase gegebenenfalls reizend wirkenden Zusatzstoffen verzichtet werden kann.

Wie zuvor angeführt, ist es im Rahmen der vorliegenden Erfindung gelungen, eine Zusammensetzung bereitzustellen, welche sich auch durch eine hervorragende Stabilität auszeichnet, so dass die Zusammensetzung über große Lagerungszeiträume aufbewahrt werden kann. Auf dieser Basis wird auch die Anwendungssicherheit weiterführend erhöht.

Insbesondere kann die Zusammensetzung bei Temperaturen im Bereich von 20 °C bis 45 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerungsstabil, sein. In diesem Zusammenhang kann die Zusammensetzung zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b) bzw. der Edukte, aufweisen.

Insbesondere kann die Zusammensetzung eine Stabilität, insbesondere Lagerungsstabilität, unter beschleunigten Alterungsbedingungen gemäß ASTM F 1980 [ASTM F 1980: Standard Guide for Accelerated Aging of Sterile Barrier Systems for Medical Devices; 2007-04] bei einer Alterungstemperatur von 55 °C von mindestens 6 Monate, insbesondere mindestens 12 Monate, vorzugsweise mindestens 24 Monate, bevorzugt mindestens 36 Monate, aufweisen. Insbesondere kann die Zusammensetzung in diesem Zusammenhang zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b) bzw. der Edukte, aufweisen.

Darüber hinaus kann sich die erfindungsgemäße Zusammensetzung - sofern überhaupt vorhanden - durch einen geringen Gehalt an Erdalkali-Bestandteilen auszeichnen:
Gemäß einer erfindungsgemäßen Ausführungsform kann die Zusammensetzung eine Erdalkalisalz-Konzentration, insbesondere Calciumsalz-Konzentration bzw. Magnesiumsalz-Konzentration, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Im Rahmen der vorliegenden Erfindung kann die Zusammensetzung darüber hinaus zumindest im Wesentlichen frei von Erdalkalisalzen, insbesondere zumindest im Wesentlichen frei von Calciumsalzen und/oder Magnesiumsalzen, sein.

Im Allgemeinen kann die Zusammensetzung nach der Erfindung eine Erdalkalimetall-Konzentration, insbesondere Calcium-Konzentration bzw. Magnesium-Konzentration, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Insbesondere kann die Zusammensetzung nach der Erfindung zumindest im Wesentlichen frei von Erdalkalimetallen, insbesondere zumindest im Wesentlichen frei von Calcium bzw. Magnesiumsalzen, sein.

Zudem kann die Zusammensetzung eine Konzentration an bivalenten Ionen, insbesondere bivalenten Kationen, vorzugsweise Erdalkali-Ionen, bevorzugt Calcium-Ionen bzw. Magnesium-Ionen, von höchstens 1 mg/ml, insbesondere höchstens 0,5 mg/ml, vorzugsweise höchstens 0,1 mg/ml, bevorzugt höchstens 0,05 mg/ml, besonders bevorzugt höchstens 0,01 mg/ml, aufweisen.

Insbesondere kann die Zusammensetzung zumindest im Wesentlichen frei von bivalenten Ionen, insbesondere bivalenten Kationen, vorzugsweise Erdalkali-Ionen, bevorzugt Calcium-Ionen und/oder Magnesium-Ionen, sein.

Die erfindungsgemäße Konzeption, wonach die Zusammensetzung nach der Erfindung zumindest im Wesentlichen frei von Erdalkalisalzen bzw. Erdalkalimetallen, insbesondere in ionischer Form, gemäß den obigen Ausführungen sein kann, führt insbesondere dazu, dass eine diesbezügliche Wechselwirkung mit den Komponenten (a) bzw. (b) bzw. (c), insbesondere im Hinblick auf eine Vermeidung unerwünschter Komplexbildungen oder dergleichen, verringert bzw. verhindert wird. Folglich kann auf dieser Basis einer Ausfällung von Wirkkomponenten bzw. Pufferkomponenten entgegengewirkt werden. Insbesondere kann auf dieser Basis auch eine unerwünschte Veränderung der Viskosität insbesondere infolge einer unkontrollierten Komplexbildung oder dergleichen vermieden werden.

Gemäß einer erfindungsgemäßen Ausführungsform kann die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen.

In diesem Zusammenhang kann die Zusammensetzung, insbesondere anwendungs-, dosier- und/oder applikationsfertig, zur Verabreichung, insbesondere Instillation in die Harnblase, eines Volumens der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen bzw. hergerichtet sein. Insbesondere kann die Zusammensetzung, insbesondere anwendungs-, dosier- und/oder applikationsfertig, insbesondere mittels Instillation in die Harnblase, mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, verabreicht werden.

Im Allgemeinen kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, vorliegen.

In diesem Zusammenhang kann die erfindungsgemäße Zusammensetzung im Allgemeinen, insbesondere anwendungs-, dosier- und/oder applikationsfertig, zur Verabreichung einer Wirkstoffmenge an Chondroitinsulfat und/oder physiologisch verträglichem Chondroitinsulfat-Salz (Komponente (a)) in einem Bereich von (100 ± 25) mg bis (1.250 ±100) mg, insbesondere in einem Bereich von (150 ± 10) mg bis (1.100 ± 100) mg, vorzugsweise in einem Bereich von (200 ± 10) mg bis (1.000 ± 100) mg, vorliegen bzw. hergerichtet sein. Insbesondere kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, verabreicht werden.

Gleichermaßen kann die Zusammensetzung, insbesondere anwendungs-, dosier- bzw. applikationsfertig, mit einer Wirkstoffmenge an Chondroitinsulfat und/oder physiologisch verträglichem Chondroitinsulfat-Salz (Komponente (a)) von (1.000 ± 100) mg, insbesondere (1.000 ± 75) mg, vorzugsweise (1.000 ± 50) mg, bevorzugt etwa 1.000 mg, vorliegen. Die Zusammensetzung nach der Erfindung kann zudem, insbesondere anwendungs-, dosier- und/oder applikationsfertig, zur Verabreichung einer Wirkstoffmenge an Chondroitinsulfat bzw. physiologisch verträglichem Chondroitinsulfat-Salz (Komponente (a)) von (1.000 ± 100) mg, insbesondere (1.000 ± 75) mg, vorzugsweise (1.000 ± 50) mg, bevorzugt etwa 1.000 mg, vorliegen bzw. hergerichtet sein. In diesem Zusammenhang kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) von (1.000 ± 100) mg, insbesondere (1.000 ± 75) mg, vorzugsweise (1.000 ± 50) mg, bevorzugt etwa 1.000 mg, verabreicht werden.

Gemäß einer alternativen Ausführungsform kann die Zusammensetzung, insbesondere anwendungs-, dosier- und/oder applikationsfertig, mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, vorliegen. In diesem Zusammenhang kann die Zusammensetzung, insbesondere anwendungs-, dosier- und/oder applikationsfertig, zur Verabreichung einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, vorliegen bzw. hergerichtet sein. Insbesondere kann die Zusammensetzung, insbesondere anwendungs-, dosier- und/oder applikationsfertig, demnach mit einer Wirkstoffmenge an Chondroitinsulfat und/oder physiologisch verträglichem Chondroitinsulfat-Salz (Komponente (a)) von (225 ± 25) mg, insbesondere (225 ± 15) mg, vorzugsweise (225 ± 10) mg, verabreicht werden.

Was Komponente (b) anbelangt, so kann die Zusammensetzung nach der Erfindung, insbesondere anwendungs-, dosier- und/oder applikationsfertig, mit einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ±100) mg, insbesondere in einem Bereich von (100 ± 25) mg bis (900 ± 100) mg, vorzugsweise in einem Bereich von (150 ± 25) mg bis (800 ± 100) mg, vorliegen.

In diesem Zusammenhang kann es erfindungsgemäß insbesondere vorgesehen sein, dass die Zusammensetzung, insbesondere anwendungs-, dosier- und/oder applikationsfertig, zur Verabreichung einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, vorliegt bzw. hergerichtet ist.

Insbesondere kann die Zusammensetzung in diesem Zusammenhang, insbesondere anwendungs-, dosier- und/oder applikationsfertig, mit einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, verabreicht werden.

Die erfindungsgemäße Zusammensetzung kann in diesem Zusammenhang zudem insbesondere anwendungs-, dosier- bzw. applikationsfertig mit einer Wirkstoffmenge an Hyaluronsäure bzw. physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, vorliegen.

Erfindungsgemäß kann die Zusammensetzung insbesondere anwendungs-, dosier- bzw. applikationsfertig zur Verabreichung einer Wirkstoffmenge an Hyaluronsäure und/oder physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, vorliegen bzw. hergerichtet sein. Insbesondere kann die Zusammensetzung, insbesondere anwendungs-, dosier- und/oder applikationsfertig, mit einer Wirkstoffmenge an Hyaluronsäure bzw. physiologisch verträglichem Hyaluronsäure-Salz (Hyaluronat) (Komponente (b)) von (800 ± 80) mg, insbesondere (800 ± 60) mg, vorzugsweise (800 ± 40) mg, bevorzugt etwa 800 mg, verabreicht werden.

Erfindungsgemäß wird somit in zweckgerichteter Weise insbesondere auf eine speziell dosierte Zusammensetzung im Hinblick auf die Wirkkomponenten (a) bzw. (b) abgestellt, welche zudem vorzugsweise anwendungsfertig mit einem definierten Volumen, wie zuvor definiert, vorliegt. Denn die Anmelderin hat in diesem Zusammenhang in völlig überraschender Weise gefunden, dass die entsprechend hohen und aufeinander abgestimmten Wirkstoffmengen bei gleichzeitig hoher Stabilität der erfindungsgemäßen Zusammensetzung zu einer besonders guten Wirksamkeit hinsichtlich der Behandlung von BCG-assoziierten bzw. von mit einer BCG-Therapie in Verbindung stehenden Nebenwirkungen führen, so dass hierbei ein Stabilitäts- bzw. Wirkoptimum vorliegt. Diesbezüglich kommt auch dem speziellen Volumen, wie zuvor definiert, insofern eine hohe Bedeutung zu, als auf dieser Basis eine optimale Anpassung der Verabreichungsgrößen der zu applizierenden bzw. zu instillierenden Zusammensetzung an die pathologische Situation vorliegt, wonach nämlich als Folge der BCG-Therapie als Nebenwirkung bzw. als unerwünschter Effekt auch eine verringerte Blasenkapazität bzw. eine sogenannte Schrumpfblase vorliegen kann. Durch Verabreichung bzw. Instillation des zugrundeliegenden speziellen Volumens kann die Haltezeit bzw. -dauer und somit das Verbleiben der Zusammensetzung in der Harnblase erhöht werden, was die Wirksamkeit der erfindungsgemäßen Zusammensetzung sowie der zugrundeliegenden BCG-Therapie weiterführend verbessert.

Im Rahmen der vorliegenden Erfindung kann es dabei vorgesehen sein, dass die Zusammensetzung in einer Aufbewahrungs- bzw. Applikationsvorrichtung, insbesondere in ein Aufbewahrungs- bzw. Applikationsbehältnis, eingebracht ist bzw. vorliegt, und zwar insbesondere anwendungs-, dosier- bzw. applikationsfertig.

In diesem Zusammenhang kann die Zusammensetzung somit insbesondere anwendungs-, dosier- bzw. applikationsfertig in einer Aufbewahrungs- bzw. Applikationsvorrichtung, insbesondere in einem Aufbewahrungs- und/oder Applikationsbehältnis, eingebracht vorliegen, bevorzugt in Volumengrößen und/oder mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, je Behältnis bzw. Applikationseinheit (Dosiereinheit).

Insbesondere kann in diesem Zusammenhang die erfindungsgemäße Aufbewahrungs- bzw. Applikationsvorrichtung eine vorzugsweise sterile Spritze, insbesondere Applikationsspritze, vorzugsweise Einweg-Applikationsspritze, sein, insbesondere mit einem Aufnahme- bzw. Füllvolumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml. Demgegenüber kann die Aufbewahrungs- bzw. Applikationsvorrichtung auch in Form einer Durchstechflasche, vorzugsweise mit sterilem Verschluss, oder dergleichen vorliegen bzw. ausgebildet sein.

Gemäß dem vorliegenden Aspekt betrifft die vorliegende Erfindung auch die erfindungsgemäße Zusammensetzung zur Instillation bzw. zur vorzugsweise topischen Applikation, bevorzugt alternierend bzw. abwechselnd bzw. nachfolgend zu oder gemeinsam mit einer Instillation bzw. Applikation einer BCG-Zusammensetzung, insbesondere BCG-Instillationszusammensetzung, in den Urogenitalbereich, insbesondere in die Harnblase.

Insbesondere kann die Zusammensetzung nach der Erfindung zur Verabreichung bzw. zur Instillation bzw. zur vorzugsweise topischen Applikation, bevorzugt alternierend bzw. abwechselnd bzw. nachfolgend zu oder gemeinsam mit einer Instillation bzw. Applikation einer BCG-Zusammensetzung, in den Urogenitaltrakt, insbesondere in die Harnblase, hergerichtet sein. Gleichermaßen kann die Zusammensetzung nach der Erfindung in diesem Zusammenhang mittels Instillation bzw. zur vorzugsweise topischen Applikation in den Urogenitaltrakt, insbesondere in die Harnblase, bevorzugt alternierend bzw. abwechselnd bzw. nachfolgend zu oder gemeinsam mit einer Instillation bzw. Applikation einer BCG-Zusammensetzung, verabreicht werden.

Was die mit der BCG-Therapie auftretenden und im Rahmen der vorliegenden Erfindung auf Basis der erfindungsgemäßen Zusammensetzung behandelten Nebenwirkungen als solche anbelangt, so können die zu behandelnden Nebenwirkungen der BCG-Therapie insbesondere entzündliche Erkrankungen bzw. Veränderungen der Schleimhäute des Urogenitaltraktes, insbesondere der Harnblasenschleimhaut bzw. des Harnblasenepithels bzw. des Harnblasenurothels, sein. Erfindungsgemäß können die Nebenwirkungen ausgewählt sein aus der Gruppe von Pollakisurie, Dysurie, Hämaturie, Prostatitis, insbesondere asymptomatischer granulomatöser Prostatitis, insbesondere BCG-induzierter Sepsis, Urethrastriktur, Schrumpfblase, verminderter Harnblasenkapazität und Schmerzen insbesondere im Bereich des Urogenitaltraktes, insbesondere im Bereich der Blase. Die in Rede stehenden Nebenwirkungen können auf Basis der mit der erfindungsgemäßen Konzeption bereitgestellten Zusammensetzung wirksam behandelt bzw. verringert bzw. verhindert werden.

Vorliegend beschrieben ist auch die Verwendung der erfindungsgemäßen Zusammensetzung, insbesondere wie zuvor definiert,
zur Erhöhung der Verträglichkeit einer BCG-Therapie (*Bacillus-Calmette-Guérin-*Therapie) von malignen Erkrankungen des Urogenitaltraktes, insbesondere der Harnblase, vorzugsweise von malignen Karzinomen, bevorzugt von Harnblasenkarzinomen,
wobei die Zusammensetzung in Kombination und jeweils in wirksamen, insbesondere pharmazeutisch wirksamen Mengen enthält:
(a) Chondroitinsulfat und/oder ein physiologisch verträgliches Chondroitinsulfat-Salz (Komponente (a)), wobei die Zusammensetzung das Chondroitinsulfat und/oder das physiologisch verträgliche Chondrointinsulfat-Salz in einer Konzentration in einem Bereich von (2 ± 0,5) mg/ml bis (25 ± 2) mg/ml aufweist; und
(b) Hyaluronsäure und/oder ein physiologisch verträgliches Hyaluronsäure-Salz (Hyaluronat) (Komponente (b)), wobei die Zusammensetzung die Hyaluronsäure und/oder das physiologisch verträgliche Hyaluronsäure-Salz in einer Konzentration in einem Bereich von (1 ± 0,5) mg/ml bis (20 ± 2) mg/ml aufweist.

In diesem Zusammenhang ist vorliegend beschrieben gleichermaßen auch die Verwendung der erfindungsgemäßen Zusammensetzung, insbesondere wie zuvor definiert,
zur Herstellung eines Arzneimittels bzw. Medikaments zur Erhöhung der Verträglichkeit einer BCG-Therapie (*Bacillus-Calmette-Guérin*-Therapie) von malignen Erkrankungen des Urogenitaltraktes, insbesondere der Harnblase, vorzugsweise von malignen Karzinomen, bevorzugt von Harnblasenkarzinomen,
wobei die Zusammensetzung in Kombination und jeweils in wirksamen, insbesondere pharmazeutisch wirksamen Mengen enthält:
(a) Chondroitinsulfat und/oder ein physiologisch verträgliches Chondroitinsulfat-Salz (Komponente (a)), wobei die Zusammensetzung das Chondroitinsulfat und/oder das physiologisch verträgliche Chondrointinsulfat-Salz in einer Konzentration in einem Bereich von (2 ± 0,5) mg/ml bis (25 ± 2) mg/ml aufweist; und
(b) Hyaluronsäure und/oder ein physiologisch verträgliches Hyaluronsäure-Salz (Hyaluronat) (Komponente (b)), wobei die Zusammensetzung die Hyaluronsäure und/oder das physiologisch verträgliche Hyaluronsäure-Salz in einer Konzentration in einem Bereich von (1 ± 0,5) mg/ml bis (20 ± 2) mg/ml aufweist.

Die erfindungsgemäße Zusammensetzung bzw. Kombination, welche Chondroitinsulfat bzw. ein physiologisch verträgliches Chondroitinsulfat-Salz (Komponente (a)) einerseits und Hyaluronsäure bzw. ein physiologisch verträgliches Hyaluronsäure-Salz (Komponente (b)) andererseits in jeweils spezieller Konzentration und darüber hinaus gegebenenfalls ein spezielles (Phosphat-)Puffersystem (Komponente (c)) enthält, führt über die überraschend gefundene hohe Stabilität, insbesondere Lagerstabilität, hinausgehend auch zu einer besonders guten Wirksamkeit hinsichtlich der vorgenannten Nebenwirkungen, wie zuvor angeführt.

Zu Zwecken der Verwendungen kann die erfindungsgemäße Zusammensetzung in den Urogenitalbereich, insbesondere in die Harnblase, instilliert bzw. topisch appliziert werden. Dabei sollte die Instillation bzw. die Applikation in die vorzugsweise zuvor entleerte Harnblase erfolgen. Die Instillation kann beispielsweise alternierend bzw. abwechselnd bzw. nachfolgend zu oder gemeinsam mit einer Instillation einer BCG-Zusammensetzung erfolgen. Diesbezüglich kann die Zusammensetzung nach der Erfindung in der Harnblase beispielsweise für eine Zeitdauer von mehreren Minuten bis zu einigen Stunden verbleiben, um eine optimale Einwirkung der Wirkstoffe auf das Urothel zu ermöglichen. Durch die erfindungsgemäße Zusammensetzung mit der speziellen Auswahl und Abstimmung der Komponenten wird dabei eine besonders effiziente Wechselwirkung der Wirkstoffe in Form der Komponenten (a) bzw. (b) mit dem Urothel ermöglicht, einhergehend mit einer entsprechend hohen An- bzw. Einlagerung der in Rede stehenden Wirkstoffe an bzw. in die zugrundeliegende Schicht der Harnblase.

In diesem Zusammenhang kann insbesondere derart vorgegangen werden, dass das zugrundeliegende Volumen der Zusammensetzung, welche sich insbesondere in der nachfolgend noch beschriebenen Aufbewahrungs- bzw. Applikationsvorrichtung befindet, nach vollständiger Entleerung der Harnblase in die Harnblase instilliert wird, wobei das zugrundeliegende Volumen insbesondere etwa 50 ml betragen kann. Um optimale Ergebnisse zu erreichen, sollte die Zusammensetzung nach der Erfindung möglichst lange in der Harnblase verbleiben, und zwar - wie zuvor beschrieben - für einen Zeitraum von mehreren Minuten bis mehreren Stunden.

Beispielsweise kann im Rahmen der Behandlung von mit einer BCG-Therapie einhergehenden Nebenwirkungen derart vorgegangen werden, dass die erfindungsgemäße Zusammensetzung alternierend bzw. abwechselnd bzw. nachfolgend zu oder gemeinsam mit einer insbesondere vorangehenden Instillation einer BCG-haltigen Zusammensetzung intravesikal verabreicht bzw. instilliert wird, vorzugsweise in die zuvor entleerte Blase. In diesem Zusammenhang kann die erfindungsgemäße Zusammensetzung nach der Erfindung auch zur Behandlung von Nebenwirkungen im Rahmen bzw. begleitend zu einer Initialtherapie mit einer BCG-haltigen Zusammensetzung sowie im Rahmen bzw. begleitend zu einer diesbezüglichen Erhaltungstherapie eingesetzt werden. Was die zugrundeliegende BCG-Therapie anbelangt, so kann eine Initialtherapie mit einer BCG-haltigen Zusammensetzung beispielsweise etwa zwei bis drei Wochen nach einer Blasenbiopsie bzw. einer Tumorresektion durchgeführt und im wöchentlichen Abstand sechs Wochen lang wiederholt werden. Das Therapieschema einer sich gegebenenfalls anschließenden Erhaltungstherapie mit einer BCG-haltigen Zusammensetzung kann beispielsweise in einer zwölfmonatigen Erhaltungstherapie mit einer monatlichen Beabstandung der jeweiligen Instillationen der BCG-haltigen Zusammensetzung erfolgen. Ein weiteres Schema einer Erhaltungstherapie kann beispielsweise aus drei Instillationen einer BCG-haltigen Zusammensetzung im wöchentlichen Abstand in den Monaten 3, 6, 12, 18, 24, 30 und 36 nach Tumorresektion bzw. nach Therapiebeginn bestehen. In entsprechender Weise kann insbesondere alternierend bzw. abwechselnd bzw. nachfolgend zu oder aber gemeinsam mit einer Verabreichung der BCG-haltigen Zusammensetzung eine intravesikale Applikation bzw. Instillation der erfindungsgemäßen Zusammensetzung in Anlehnung an das obige Schema erfolgen.

Ein weiterer Vorteil der vorliegend beschriebenen Verwendungen der Zusammensetzung nach der Erfindung ist zudem darin zu sehen, dass diese hinsichtlich ihrer Anwendung sicher ist und während der Behandlung keine auf die erfindungsgemäße Zusammensetzung selbst zurückgehenden nennenswerten Nebenwirkungen auftreten, insbesondere da es sich bei den eingesetzten Substanzen um biokompatible Stoffe handelt. Die besonders gute Verträglichkeit steht insbesondere auch mit der Verwendung von Chondroitinsulfat marinen Ursprungs bzw. Hyaluronsäure nichttierischen Ursprungs in Zusammenhang.

Durch die zweckgerichtete Verwendung der vorgenannten Dosen bzw. Mengen an Wirksubstanzen auf Basis der Wirkkomponenten (a) und (b) wird zudem eine hohe Wirksamkeit der erfindungsgemäßen Zusammensetzung erreicht, welche zudem durch das spezielle Puffersystem nicht nachteilig beeinträchtigt wird. Vielmehr führt der gezielte Einsatz des in Rede stehenden Puffersystems gemäß Komponente (c) in Kombination mit den weiteren erfindungsgemäßen Maßnahmen zu einer effektiven Stabilisierung der Zusammensetzung, was zu einer hohen Anwendungssicherheit und Aufrechterhaltung der Wirksamkeit bzw. Wirkeffizienz führt.

Zusammenfassend handelt es sich bei der erfindungsgemäßen Zusammensetzung vorzugsweise um ein Medizinprodukt, das gemäß einer erfindungsgemäß bevorzugten Ausführungsform eine hochdosierte Lösung bzw. Dispersion der Wirkstoffe auf Basis der Komponenten (a) und (b) enthält.

Was die erfindungsgemäße Zusammensetzung als solche bzw. deren Verwendungen weiterhin anbelangt, so kann die Zusammensetzung insbesondere mit einem Volumen der Zusammensetzung von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, und/oder einer Wirkstoffmenge an Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, und/oder einer Wirkstoffmenge an Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, insbesondere mindestens einmal pro Monat, vorzugsweise mindestens einmal pro Woche, vorzugsweise über einen Zeitraum von vorzugsweise mindestens einem Monat, bevorzugt alternierend bzw. abwechselnd bzw. nachfolgend zu oder gemeinsam mit einer Instillation und/oder Applikation einer BCG-Zusammensetzung, in die Harnblase verabreicht bzw. instilliert bzw. vorzugsweise topisch appliziert werden bzw. zur mindestens monatlichen Verabreichung (mindestens einmal pro Monat), vorzugsweise zur mindestens wöchentlichen Verabreichung (mindestens einmal pro Woche), vorzugsweise über einen Zeitraum von vorzugsweise mindestens einem Monat, mittels Instillation bzw. topischer Applikation in die Harnblase, bevorzugt alternierend bzw. abwechselnd bzw. nachfolgend zu oder gemeinsam mit einer Instillation und/oder Applikation einer BCG-Zusammensetzung, hergerichtet sein. Insbesondere kann die Zusammensetzung mindestens einmal pro Monat, vorzugsweise mindestens einmal pro Woche, vorzugsweise über einen Zeitraum von vorzugsweise mindestens einem Monat, mittels Instillation bzw. zur topischer Applikation in die Harnblase verabreicht werden, bevorzugt alternierend bzw. abwechselnd bzw. nachfolgend zu oder gemeinsam mit einer Instillation bzw. Applikation einer BCG-Zusammensetzung.

Für weitergehende Einzelheiten zu den vorliegend beschriebenen Verwendungen der Zusammensetzung nach der Erfindung kann auf die Ausführungen zu den übrigen Erfindungsaspekten verwiesen werden, welche in Bezug auf die vorliegend beschriebenen Verwendungen entsprechend gelten.

Die erfindungsgemäße Zusammensetzung zeichnet sich auch in diesem Zusammenhang über eine hervorragende Lagerstabilität und über eine hohe Wirkeffizienz aus.

Gemäß einem wiederum **weiteren** Aspekt der vorliegenden Erfindung betrifft die vorliegende Erfindung gleichermaßen eine Aufbewahrungs- bzw. Applikationsvorrichtung zur Verwendung bei der Erhöhung der Verträglichkeit einer BCG-Therapie (*Bacillus-Calmette-Guérin*-Therapie) von malignen Erkrankungen des Urogenitaltraktes, insbesondere ein Aufbewahrungs- bzw. Applikationsbehältnis, insbesondere in Form einer vorzugsweise sterilen Spritze, insbesondere Applikationsspritze, vorzugsweise Einweg-Applikationsspritze, vorzugsweise zur Instillation bzw. zur vorzugsweise topischen Applikation in den Urogenitalbereich, insbesondere in die Harnblase, enthaltend eine Zusammensetzung zur Verwendung nach der Erfindung, wie zuvor definiert.

In diesem Zusammenhang betrifft die vorliegende Erfindung gemäß diesem Aspekt auch eine Aufbewahrungs- bzw. Applikationsvorrichtung zur Verwendung bei der Erhöhung der Verträglichkeit einer BCG-Therapie (*Bacillus-Calmette-Guérin-*Therapie) von malignen Erkrankungen des Urogenitaltraktes, insbesondere ein Aufbewahrungs- bzw. Applikationsbehältnis, insbesondere in Form einer vorzugsweise sterilen Spritze, insbesondere Applikationsspritze, vorzugsweise Einweg-Applikationsspritze, vorzugsweise zur Instillation bzw. zur vorzugsweise topischen Applikation in den Urogenitalbereich, insbesondere in die Harnblase, enthaltend eine Zusammensetzung zur Verwendung, wie zuvor definiert,
wobei die Zusammensetzung in Kombination und jeweils in wirksamen, insbesondere pharmazeutisch wirksamen Mengen enthält:
(a) Chondroitinsulfat und/oder ein physiologisch verträgliches Chondroitinsulfat-Salz (Komponente (a)), wobei die Zusammensetzung das Chondroitinsulfat und/oder das physiologisch verträgliche Chondrointinsulfat-Salz in einer Konzentration in einem Bereich von (2 ± 0,5) mg/ml bis (25 ± 2) mg/ml aufweist; und
(b) Hyaluronsäure und/oder ein physiologisch verträgliches Hyaluronsäure-Salz (Hyaluronat) (Komponente (b)), wobei die Zusammensetzung die Hyaluronsäure und/oder das physiologisch verträgliche Hyaluronsäure-Salz in einer Konzentration in einem Bereich von (1 ± 0,5) mg/ml bis (20 ± 2) mg/ml aufweist.

Insbesondere kann die Zusammensetzung außerdem (c) ein Puffersystem, insbesondere Dihydrogenphosphat/Monohydrogenphosphat-Puffersystem, (Komponente (c)) enthalten.

Zudem kann die Zusammensetzung außerdem (d) mindestens einen physiologisch verträglichen Elektrolyten (Komponente (d)) enthalten.

Erfindungsgemäß ist es dabei bevorzugt, wenn die Aufbewahrungs- bzw. Applikationsvorrichtung ein Aufnahme- bzw. Füllvolumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, aufweist.

Insbesondere kann die Zusammensetzung eine Wirkstoffmenge an (a) Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, aufweisen.

Zudem kann die Zusammensetzung eine Wirkstoffmenge an (b) Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, aufweisen.

Erfindungsgemäß kann es zudem vorgesehen sein, dass die Zusammensetzung das Puffersystem, insbesondere das Dihydrogenphosphat/Monohydrogenphosphat-Puffersystem, (Komponente (c)) in einer Gesamtmenge an Puffersystem, insbesondere an Dihydrogenphosphat/Monohydrogenphosphat-Puffersystem, (Komponente (c)) von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, aufweist.

Insbesondere kann die Zusammensetzung den Elektrolyten (Komponente (d)) in einer Menge von (400 ± 300) mg, insbesondere in einer Menge von (400 ± 200) mg, vorzugsweise in einer Menge von (400 ± 100) mg, bevorzugt in einer Menge von (400 ± 50) mg, besonders bevorzugt in einer Menge von etwa 400 mg, enthalten.

Erfindungsgemäß kann es zudem vorgesehen sein, dass das Chondroitinsulfat bzw. das physiologisch verträgliche Chondroitinsulfat-Salz (Komponente (a)) in Form eines Alkalisalzes, vorzugsweise in Form eines Natrium-Salzes, vorliegt.

Gleichermaßen kann es erfindungsgemäß vorgesehen sein, dass das Chondroitinsulfat bzw. das physiologisch verträgliche Chondroitinsulfat-Salz (Komponente (a)) in Form von Chondroitinsulfat-Natrium (Natrium-Chondroitinsulfat) vorliegt.

Zudem kann die Hyaluronsäure bzw. das physiologisch verträgliche Hyaluronsäure-Salz (Komponente (b)) in Form eines Alkalisalzes, vorzugsweise in Form eines Natrium-Salzes, und/oder in Form eines Alkalihyaluronates vorliegen.

Insbesondere kann die Hyaluronsäure bzw. das physiologisch verträgliche Hyaluronsäure-Salz (Komponente (b)) in Form von Natriumhyaluronat vorliegen.

Darüber hinaus kann das Dihydrogenphosphat/Monohydrogenphosphat-Puffersystem (Komponente (c)) als Alkalidihydrogenphosphat/Alkalimonohydrogenposphat-Puffersystem vorliegen bzw. ausgebildet sein. In diesem Zusammenhang kann das Alkalimetall ausgewählt sein aus Natrium und/oder Kalium, insbesondere Natrium.

Insbesondere kann das Dihydrogenphosphat/Monohydrogenphosphat-Puffersystem (Komponente (c)) als Natriumdihydrogenphosphat/Natriummonohydrogenposphat-Puffersystem, vorliegen bzw. ausgebildet sein.

Darüber hinaus kann der Elektrolyt (Komponente (d)) in Form eines Alkalisalzes, insbesondere in Form eines Alkalichlorids, vorzugsweise in Form von Natriumchlorid, vorliegen bzw. ausgebildet sein. Insbesondere kann der Elektrolyt (Komponente (d)) Natriumchlorid sein.

Insbesondere kann die Zusammensetzung außerdem (e) BCG (*Bacillus-Calmette-Guérin*), insbesondere in Form von lebensfähigen Einheiten, (Komponente (e)) enthalten, insbesondere in Mengen von 10⁵ bis 10¹⁰ lebensfähigen Einheiten, insbesondere 10⁶ bis 10⁹ lebensfähigen Einheiten, vorzugsweise 10⁷ bis 10⁹ lebensfähigen Einheiten, insbesondere bezogen auf das Gesamtvolumen der zu verabreichenden Zusammensetzung.

Was die Zusammensetzung weiterhin anbelangt, so kann diese einen pH-Wert in einem Bereich von 6,1 bis 7,9, insbesondere in einem Bereich von 6,6 bis 7,7, vorzugsweise in einem Bereich von 6,9 bis 7,6, bevorzugt in einem Bereich von 7,1 bis 7,4, aufweisen.

Insbesondere kann der pH-Wert der Zusammensetzung in einem Bereich von 6,1 bis 7,9, insbesondere in einem Bereich von 6,6 bis 7,7, vorzugsweise in einem Bereich von 6,9 bis 7,6, bevorzugt in einem Bereich von 7,1 bis 7,4, konstant gehalten bzw. eingestellt sein. Der pH-Wert kann vorzugsweise mittels des Puffersystems, insbesondere des Alkalidihydrogenphosphat/Alkalimonohydrogenposphat-Puffersystems, (Komponente (c)) eingestellt bzw. vorgegeben sein.

Insbesondere kann die Zusammensetzung bei einer Temperatur von 20 °C eine dynamische Viskosität von mindestens 2.000 mPas, insbesondere mindestens 4.000 mPas, vorzugsweise mindestens 5.000 mPas, bevorzugt mindestens 5.250 mPas, aufweisen.

Gleichermaßen kann die Zusammensetzung bei einer Temperatur von 20 °C eine dynamische Viskosität von höchstens 7.900 mPas, insbesondere höchstens 6.900 mPas, vorzugsweise höchstens 6.000 mPas, bevorzugt höchstens 5.750 mPas, aufweisen.

Zudem kann die Zusammensetzung bei einer Temperatur von 20 °C eine dynamische Viskosität im Bereich von 2.000 mPas bis 7.900 mPas, insbesondere im Bereich von 4.000 mPas bis 6.900 mPas, vorzugsweise im Bereich von 5.000 mPas bis 6.000 mPas, bevorzugt im Bereich von 5.250 mPas bis 5.750 mPas, aufweisen.

Weiterhin kann die Zusammensetzung eine Osmolalität im Bereich von 150 mosmol/kg bis 600 mosmol/kg, insbesondere im Bereich von 200 mosmol/kg bis 550 mosmol/kg, vorzugsweise im Bereich von 250 mosmol/kg bis 500 mosmol/kg, bevorzugt im Bereich von 275 mosmol/kg bis 450 mosmol/kg, besonders bevorzugt im Bereich von 300 mosmol/kg bis 400 mosmol/kg, aufweisen.

Weiterhin kann die Zusammensetzung bei einer Temperatur von 20 °C und bei einem Druck von 1.013,25 mbar (Atmosphärendruck) eine Dichte im Bereich von 1,001 g/cm³ bis 1,5 g/cm³, insbesondere im Bereich von 1,005 g/cm³ bis 1,25 g/cm³, vorzugsweise im Bereich von 1,0075 g/cm³ bis 1,1 g/cm³, bevorzugt im Bereich von 1,0075 g/cm³ bis 1,075 g/cm³, besonders bevorzugt im Bereich von 1,0075 g/cm³ bis 1,05 g/cm³, aufweisen.

Insbesondere kann die Zusammensetzung bei einer Temperatur von 20 °C und bei einem Druck von 1.013,25 mbar (Atmosphärendruck) eine relative Dichte, bezogen auf reines Wasser, im Bereich von 1,001 bis 1,5, insbesondere im Bereich von 1,005 bis 1,25, vorzugsweise im Bereich von 1,0075 bis 1,1, bevorzugt im Bereich von 1,0075 bis 1,075, besonders bevorzugt im Bereich von 1,0075 bis 1,05, aufweisen.

Erfindungsgemäß ist es bevorzugt, wenn die Aufbewahrungs- bzw. Applikationsvorrichtung die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, enthält.

Insbesondere kann die Zusammensetzung mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegen.

Erfindungsgemäß ist es zudem bevorzugt, wenn die Zusammensetzung als wässrige Zusammensetzung vorliegt. Insbesondere kann die Zusammensetzung insbesondere wässrig basiert sein bzw. wässrig formuliert vorliegen, insbesondere in Form einer wässrigen Lösung bzw. wässrigen Suspension, vorzugsweise in Form einer wässrigen Lösung.

Insbesondere kann die Zusammensetzung Wasser, insbesondere gereinigtes Wasser, enthalten. Insbesondere kann die Zusammensetzung Wasser als pharmazeutisch verträglichen Träger (Exzipienten) enthalten. Erfindungsgemäß kann die Zusammensetzung dabei wässrig basiert ausgebildet sein. In diesem Zusammenhang kann die Zusammensetzung einen Gehalt an Wasser von mindestens 50 Gew.-%, insbesondere mindestens 75 Gew.-%, vorzugsweise mindestens 80 Gew.-%, bevorzugt mindestens 90 Gew.-%, besonders bevorzugt mindestens 95 Gew.-%, bezogen auf die Zusammensetzung, aufweisen.

Erfindungsgemäß kann die für die Aufbewahrungs- bzw. Applikationsvorrichtung vorgesehene Zusammensetzung nach der Erfindung aus den vorgenannten Komponenten (a), (b), gegebenenfalls (c), gegebenenfalls (e) sowie gegebenenfalls Wasser, insbesondere gereinigtem Wasser, insbesondere aus den vorgenannten Komponenten (a), (b), gegebenenfalls (c) und gegebenenfalls (d) sowie gegebenenfalls Wasser, bestehen.

Für weitergehende Einzelheiten zu der erfindungsgemäßen Aufbewahrungs- bzw. Applikationsvorrichtung, welche die Zusammensetzung nach der Erfindung enthält, kann zudem auf die Ausführungen zu den übrigen Erfindungsaspekten verwiesen werden, welche in Bezug auf die erfindungsgemäße Aufbewahrungs- bzw. Applikationsvorrichtung entsprechend gelten.

Darüber hinaus betrifft die vorliegende Erfindung - gemäß einem wiederum **weiteren** Aspekt der vorliegenden Erfindung - auch die Verpackungseinheit zur Verwendung bei der Erhöhung der Verträglichkeit einer BCG-Therapie (*Bacillus-Calmette-Guérin*-Therapie) von malignen Erkrankungen des Urogenitaltraktes nach der Erfindung, welche mindestens eine Aufbewahrungs- bzw. Applikationsvorrichtung, wie zuvor definiert, enthält. In diesem Zusammenhang kann die Aufbewahrungs- bzw. Applikationsvorrichtung in einer vor Kontaminationen schützenden Umverpackung eingebracht vorliegen. Für weitergehende Einzelheiten zu der erfindungsgemäßen Verpackungseinheit kann auf die Ausführungen zu den übrigen Erfindungsaspekten verwiesen werden, welche in Bezug auf die erfindungsgemäße Verpackungseinheit entsprechend gelten.

Schließlich betrifft die vorliegende Erfindung - gemäß einem **weiteren** Aspekt der vorliegenden Erfindung - auch das erfindungsgemäße Kit zur Verwendung bei der Erhöhung der Verträglichkeit einer BCG-Therapie (*Bacillus-Calmette-Guérin-*Therapie) von malignen Erkrankungen des Urogenitaltraktes, insbesondere Instillationssystem, umfassend (i) mindestens eine Aufbewahrungs- bzw. Applikationsvorrichtung, wie zuvor definiert, und (ii) mindestens eine Zusammensetzung, wie zuvor definiert, insbesondere wobei die Zusammensetzung vorzugsweise anwendungs-, dosier- bzw. applikationsfertig in der Aufbewahrungs- und/oder Applikationsvorrichtung vorliegt, und (iii) mindestens eine an die Aufbewahrungs- bzw.

Applikationsvorrichtung anschließbare Instillationsvorrichtung, insbesondere in Form eines Instillationsschlauches oder dergleichen.

Auf Basis des erfindungsgemäßen Kits wird insbesondere ein entsprechendes Installationssystem mit einfacher Handhabbarkeit, schneller Einsatzbereitschaft und hoher Anwendungssicherheit bereitgestellt.

Für weitergehende Einzelheiten zu dem erfindungsgemäßen Kit kann auf die vorangegangenen Ausführungen zu den übrigen Erfindungsaspekten verwiesen werden, welche in Bezug auf das erfindungsgemäße Kit entsprechend gelten.

Im Rahmen der vorliegenden Erfindung wird somit insgesamt unter Zugrundelegung der erfindungsgemäßen Zusammensetzung mit den hochdosierten Wirkstoffkomponenten ein leistungsfähiges Gesamtkonzept zur Behandlung von mit einer BCG-Therapie einhergehenden Nebenwirkungen bzw. zur Bereitstellung eines diesbezüglichen Co-Therapeutikums bereitgestellt, wobei die erfindungsgemäße Zusammensetzung neben einer hohen Wirksamkeit und hervorragender Handhabbarkeit über eine signifikant erhöhte Stabilität, insbesondere Lagerungsstabilität, verfügt.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, ohne die vorliegende Erfindung jedoch hierauf zu beschränken.

### AUSFÜHRUNGSBEISPIELE

### 1. Herstellungsbeispiele

Zur Herstellung von jeweils 50 ml einer klaren, gegebenenfalls leicht gelblich gefärbten wässrigen Lösung erfindungsgemäßer Zusammensetzungen wird in dem Fachmann an sich bekannter Weise vorgegangen.

Zunächst wird bei Raumtemperatur und Umgebungsdruck in einem Glasgefäß mit Rühreinrichtung eine definierte Teilmenge (insbesondere etwa 75 % des gewünschten Endvolumens) an gereinigtem Wasser vorgelegt. Unter Rühren werden zunächst ein Elektrolyt in Form von Natriumchlorid und nachfolgend die Komponenten des Puffersystems in Form von Natriumdihydrogenphosphat und Dinatriumhydrogenphosphat hinzugegeben. Nach vollständiger Auflösung der Komponenten wird der pH-Wert bestimmt und auf den gewünschten Wert (z. B. pH-Wert von ca. 7), gegebenenfalls unter Verwendung von Phosphorsäure oder Natronlauge, eingestellt.

Anschließend werden die nachfolgenden in den Rezepturbeispielen spezifizierten Mengen und Typen der weiteren Wirk- und Inhaltsstoffe unter Rühren hinzugegeben, und zwar Komponente (a) in Form von Chondroitinsulfat-Salz (Chondroitinsulfat-Natrium) und Komponente (b) in Form von Natriumhyaluronat. Die Lösung wird mit weiterem Wasser auf das gewünschte Endvolumen von 50 ml aufgefüllt.

Anschließend wird der pH-Wert der erhaltenen Zusammensetzung nochmals bestimmt und gegebenenfalls nachreguliert. Auch die übrigen entsprechenden Spezifikationen der Lösungen werden auf die eingestellten bzw. vorgewählten Wertebereiche hin überprüft (z. B. Osmolalität; relative Dichte; mikrobiologische Reinheit und Sterilität; Ausschluss von Verunreinigungen, insbesondere Abbauprodukten der Inhalts- und Wirkstoffe; Viskosität; Aussehen).

Die so erhaltene Lösung kann anschließend einer Sterilfiltration unterzogen werden, beispielsweise mit Hilfe von Stickstoff über eine Filterkerze mit speziellem Filtersystem, und nachfolgend in ein insbesondere steriles Aufnahme- bzw. Applikationsbehältnis, wie einer Kunststoffspritze oder dergleichen, abgefüllt werden. Die erhaltene Zusammensetzung kann für weiterführende Stabilitätsuntersuchungen oder für entsprechende Anwendungs- bzw. Wirksamkeitsuntersuchungen verwendet werden.

Nach dieser allgemeinen Herstellvorschrift werden die nachfolgend spezifizierten Rezepturen bzw. Zusammensetzungen durch Auflösen in Wasser der nachfolgenden Komponenten mit den diesbezüglichen Mengenangaben (Einwaage bei Herstellung der Zusammensetzung) hergestellt.

Dabei wird eine erste Serie (Serie I) von Zusammensetzungen auf Basis höherer Mengen an Chondroitinsulfat und eine zweite Serie (Serie II) von Zusammensetzungen auf Basis niedrigerer Mengen an Chondroitinsulfat hergestellt:

### Serie I:

### Zusammensetzungen A1, B1 bis B8, C1 und C2, D1 und D2, E1 bis E4 sowie F1 bis F4

### Zusammensetzung A1 (Angabe pro 50 ml Zusammensetzung)

| **Inhaltsstoff** | **Menge / mg** | **Qualität** |
|---|---|---|
| Chondroitinsulfat-Natrium | 1.000 | Ph. Eur. |
| Mₙ = 15 kDa; M_{W} = 100 kDa; M_{z} = 430 kDa | | |
| Natriumhyaluronat | 800 | Ph. Eur. |
| Mₙ = 55 kDa; M_{W} = 200 kDa; M_{z} = 700 kDa | | |
| Puffersystem: | | Ph. Eur. |
| Natriumdihydrogenphosphat (NaH₂(PO₄) · 2 H₂O) / | 11 | |
| Dinatriumhydrogenphosphat (Na₂H(PO₄) · 2 H₂O) | 76 | |
| Elektrolyt | 395 | Ph. Eur. |
| (Natriumchlorid) | | |
| Gereinigtes Wasser | ad 50 ml | Ph. Eur. |

Der pH-Wert der Zusammensetzung A1 beträgt zudem etwa 7,2. Weiterhin weist die vorliegende Zusammensetzung eine dynamische Viskosität von etwa 5.600 mPas auf, und die Dichte beträgt etwa 1,022 g/cm³ (20 °C und Atmosphärendruck). Die Osmolalität der vorliegenden Zusammensetzung beträgt zudem etwa 370 mosmol/kg.

### Zusammensetzungen B1 bis B4

Es werden weitere Zusammensetzungen entsprechend der Zusammensetzung A1 hergestellt, jedoch mit der Maßgabe, dass unterschiedliche Mengen an Chondroitinsulfat-Natrium eingesetzt werden:

| **Zusammensetzung** | **Menge Chondroitinsulfat-Natrium / mg** |
|---|---|
| Zusammensetzung B1 | 850 |
| Zusammensetzung B2 | 900 |
| Zusammensetzung B3 | 1.100 |
| Zusammensetzung B4 | 1.150 |

### Zusammensetzungen B5 bis B8

Es werden nochmals weitere Zusammensetzungen entsprechend der Zusammensetzung A1 hergestellt, jedoch mit der Maßgabe, dass unterschiedliche Mengen an Natriumhyaluronat eingesetzt werden:

| **Zusammensetzung** | **Menge Natriumhyaluronat / mg** |
|---|---|
| Zusammensetzung B5 | 720 |
| Zusammensetzung B6 | 680 |
| Zusammensetzung B7 | 880 |
| Zusammensetzung B8 | 940 |

### Zusammensetzungen C1 und C2

Es werden nochmals weitere Zusammensetzungen entsprechend der Zusammensetzung A1 hergestellt, jedoch mit der Maßgabe, dass das Chondroitinsulfat-Natrium (CS-Na) bei einer diesbezüglich eingesetzten Menge von jeweils 1.000 mg mit unterschiedlichen Molmassen eingesetzt wird:

| **Zusammensetzung** | **CS-Na** | **CS-Na** | **CS-Na** |
|---|---|---|---|
| | **Mₙ** | **Mw** | **M_{z}** |
| Zusammensetzung C1 | 1 kDa | 5 kDa | 50 kDa |
| Zusammensetzung C2 | 35 kDa | 250 kDa | 1.100 kDa |

### Zusammensetzungen D1 und D2

Wiederum weitere Zusammensetzungen entsprechen der Zusammensetzung A1, jedoch mit der Maßgabe, dass das von Natriumhyluronat (HA-Na) bei einer diesbezüglich eingesetzten Menge von jeweils 800 mg mit unterschiedlichen Molmassen eingesetzt wird:

| **Zusammensetzung** | **HA-Na** | **HA-Na** | **HA-Na** |
|---|---|---|---|
| | **Mₙ** | **Mw** | **M_{z}** |
| Zusammensetzung D1 | 2 kDa | 4 kDa | 275 kDa |
| Zusammensetzung D2 | 275 kDa | 325 kDa | 1.750 kDa |

### Zusammensetzungen E1 bis E4

Bei wiederum weiteren Zusammensetzungen auf Basis der Zusammensetzung A1 wird der pH-Wert wie folgt dargestellt variiert:

| **Zusammensetzung** | **pH-Wert** |
|---|---|
| Zusammensetzung E1 | 5,5 |
| Zusammensetzung E2 | 6,1 |
| Zusammensetzung E3 | 7,9 |
| Zusammensetzung E4 | 8,5 |

### Zusammensetzungen F1 bis F4

Bei wiederum weiteren Zusammensetzungen wird das Puffersystem variiert; hierzu werden zu dem in Zusammensetzung A1 eingesetztem Phosphatpuffersystem korrelierende Mengen von Puffersystemen auf Basis von Kohlensäure/Bicarbonat, Essigsäure/Acetat, Kohlensäure/Silikat und Citronensäure/Citrat eingesetzt; die Zusammensetzungen F1 bis F4 entsprechen somit Zusammensetzung A1 mit der Maßgabe, dass diesbezüglich ein andersartiges Puffersystem eingesetzt wird:

| **Zusammensetzung** | **Puffersystem** |
|---|---|
| Zusammensetzung F1 | Kohlensäure/Bicarbonat |
| Zusammensetzung F2 | Essigsäure/Acetat |
| Zusammensetzung F3 | Kohlensäure/Silikat |
| Zusammensetzung F4 | Citronensäure/Citrat |

### Serie II:

Zusammensetzungen A1', B1' bis B8', C1' und C2', D1' und D2', E1' bis E4' sowie F1' bis F4'

### Zusammensetzung A1' (Angabe pro 50 ml Zusammensetzung)

| **Inhaltsstoff** | **Menge / mg** | **Qualität** |
|---|---|---|
| Chondroitinsulfat-Natrium | 225 | Ph. Eur. |
| Mₙ = 15 kDa; M_{W} = 100 kDa; M_{z} = 430 kDa | | |
| Natriumhyaluronat | 800 | Ph. Eur. |
| Mₙ = 55 kDa; M_{W} = 200 kDa; M_{z} = 700 kDa | | |
| Puffersystem: | | Ph. Eur. |
| Natriumdihydrogenphosphat (NaH₂(PO₄) · 2 H₂O) / | 11 | |
| Dinatriumhydrogenphosphat (Na₂H(PO₄) · 2 H₂O) | 76 | |
| Elektrolyt | 395 | Ph. Eur. |
| (Natriumchlorid) | | |
| Gereinigtes Wasser | ad 50 ml | Ph. Eur. |

Der pH-Wert der Zusammensetzung A1' beträgt zudem etwa 7,2. Weiterhin weist die vorliegende Zusammensetzung eine dynamische Viskosität von etwa 5.600 mPas auf, und die Dichte beträgt etwa 1,022 g/cm³ (20 °C und Atmosphärendruck). Die Osmolalität der vorliegenden Zusammensetzung beträgt zudem etwa 370 mosmol/kg.

### Zusammensetzungen B1' bis B4'

Es werden weitere Zusammensetzungen entsprechend der Zusammensetzung A1' hergestellt, jedoch mit der Maßgabe, dass unterschiedliche Mengen an Chondroitinsulfat-Natrium eingesetzt werden:

| **Zusammensetzung** | **Menge Chondroitinsulfat-Natrium / mg** |
|---|---|
| Zusammensetzung B1' | 150 |
| Zusammensetzung B2' | 200 |
| Zusammensetzung B3' | 250 |
| Zusammensetzung B4' | 300 |

### Zusammensetzungen B5' bis B8'

Es werden nochmals weitere Zusammensetzungen entsprechend der Zusammensetzung A1' hergestellt, jedoch mit der Maßgabe, dass unterschiedliche Mengen an Natriumhyaluronat eingesetzt werden:

| **Zusammensetzung** | **Menge Natriumhyaluronat / mg** |
|---|---|
| Zusammensetzung B5' | 720 |
| Zusammensetzung B6' | 680 |
| Zusammensetzung B7' | 880 |
| Zusammensetzung B8' | 940 |

### Zusammensetzungen C1' und C2'

Es werden nochmals weitere Zusammensetzungen entsprechend der Zusammensetzung A1' hergestellt, jedoch mit der Maßgabe, dass das Chondroitinsulfat-Natrium (CS-Na) bei einer diesbezüglich eingesetzten Menge von jeweils 225 mg mit unterschiedlichen Molmassen eingesetzt wird:

| **Zusammensetzung** | **CS-Na** | **CS-Na** | **CS-Na** |
|---|---|---|---|
| | **Mₙ** | **M_{W}** | **M_{z}** |
| Zusammensetzung C1' | 1 kDa | 5 kDa | 50 kDa |
| Zusammensetzung C2' | 35 kDa | 250 kDa | 1.100 kDa |

### Zusammensetzungen D1' und D2'

Wiederum weitere Zusammensetzungen entsprechen der Zusammensetzung A1', jedoch mit der Maßgabe, dass das von Natriumhyluronat (HA-Na) bei einer diesbezüglich eingesetzten Menge von jeweils 800 mg mit unterschiedlichen Molmassen eingesetzt wird:

| **Zusammensetzung** | **HA-Na** | **HA-Na** | **HA-Na** |
|---|---|---|---|
| | **Mₙ** | **M_{W}** | **M_{z}** |
| Zusammensetzung D1' | 2 kDa | 4 kDa | 275 kDa |
| Zusammensetzung D2' | 275 kDa | 325 kDa | 1.750 kDa |

### Zusammensetzungen E1' bis E4'

Bei wiederum weiteren Zusammensetzungen auf Basis der Zusammensetzung A1' wird der pH-Wert wie folgt dargestellt variiert:

| **Zusammensetzung** | **pH-Wert** |
|---|---|
| Zusammensetzung E1' | 5,5 |
| Zusammensetzung E2' | 6,1 |
| Zusammensetzung E3' | 7,9 |
| Zusammensetzung E4' | 8,5 |

### Zusammensetzungen F1' bis F4'

Bei wiederum weiteren Zusammensetzungen wird das Puffersystem variiert; hierzu werden zu dem in Zusammensetzung A1' eingesetztem Phosphatpuffersystem korrelierende Mengen von Puffersystemen auf Basis von Kohlensäure/Bicarbonat, Essigsäure/Acetat, Kohlensäure/Silikat und Citronensäure/Citrat eingesetzt; die Zusammensetzungen F1' bis F4' entsprechen somit Zusammensetzung A1' mit der Maßgabe, dass diesbezüglich ein andersartiges Puffersystem eingesetzt wird:

| **Zusammensetzung** | **Puffersystem** |
|---|---|
| Zusammensetzung F1' | Kohlensäure/Bicarbonat |
| Zusammensetzung F2' | Essigsäure/Acetat |
| Zusammensetzung F3' | Kohlensäure/Silikat |
| Zusammensetzung F4' | Citronensäure/Citrat |

### 2. Stabilitätsuntersuchungen

An jeweiligen Chargen der Zusammensetzungen gemäß Serie I) und Serie II) gemäß den zuvor beschriebenen Rezeptururen werden Stabilitätsuntersuchungen durchgeführt.

Zu diesem Zweck wird zu entsprechenden Lagerungszeiten bzw. -zeitpunkten zum einen der Gesamtgehalt an Abbauprodukten der in den angeführten Zusammensetzungen eingesetzten Komponenten, bestimmt, und zwar jeweils zu Beginn der Untersuchungen und bei entsprechenden Lagerzeiten von 3 Monaten, 6 Monaten, 12 Monaten, 18 Monaten, 24 Monaten, 36 Monaten und 39 Monaten. Zum anderen erfolgt über denselben Lagerzeitraum zu den vorgenannten Lagerzeiten auch eine Bestimmung des pH-Wertes sowie der Viskosität.

Die Lagerung erfolgt dabei für eine erste Charge der Zusammensetzungen gemäß Serie I) bzw. Serie II) bei einer Temperatur von (25 ± 2) °C und bei einer relativen Luftfeuchte der Umgebung von (60 ± 5) % r.H. (nachfolgende Tabellen 1A bis 1F (Serie I) bzw. 1A' bis 1F' (Serie II)).

Zudem wird für eine weitere Charge der Zusammensetzungen eine Lagerung bei einer Temperatur von (40 ± 2) °C und bei einer relativen Luftfeuchte der Umgebung von (75 ± 5) % r.H. durchgeführt (nachfolgende Tabellen 2A bis 2F (Serie I) bzw. 2A' bis 2F' (Serie II)).

Die nachfolgenden Tabellen 1A bis 1F und 2A bis 2F (Serie I) bzw. 1A' bis 1F' und 2A' bis 2F' (Serie II) zeigen die diesbezüglich ermittelten Ergebnisse.

In den nachfolgenden Tabellen bedeutet "++" einen Gesamtgehalt an Abbauprodukten ("Abbau") zum jeweiligen Lagerungszeitpunkt von höchstens 3 %, bezogen auf die Gesamtkonzentration der Komponenten, bzw. eine Veränderung des pH-Wertes ("pH") bzw. der Viskosität ("Visko") zum jeweiligen Lagerungszeitpunkt von höchstens 3 %, bezogen auf den jeweiligen Ausgangswert. Weiterhin bedeutet "+" einen Gesamtgehalt an Abbauprodukten zum jeweiligen Lagerungszeitpunkt von höchstens 5 %, bezogen auf die Gesamtkonzentration der Komponenten, bzw. eine Veränderung des pH-Wertes bzw. der Viskosität zum jeweiligen Lagerungszeitpunkt von höchstens 5 %, bezogen auf den jeweiligen Ausgangswert. Schließlich bedeutet "-" einen Gesamtgehalt an Abbauprodukten zum jeweiligen Lagerungszeitpunkt von mehr als 5 %, bezogen auf die Gesamtkonzentration der Komponenten, bzw. eine Veränderung des pH-Wertes bzw. der Viskosität zum jeweiligen Lagerungszeitpunkt von mehr als 5 %, bezogen auf den jeweiligen Ausgangswert.

### Serie I:

**Tabelle 1A [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| A1 | Abbau | ++ | ++ | ++ | ++ | ++ | ++ | ++ |
| | pH | ++ | ++ | ++ | ++ | ++ | ++ | ++ |
| | Visko | ++ | ++ | ++ | ++ | ++ | ++ | ++ |

**Tabelle 1B [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| B1 | Abbau | ++ | ++ | ++ | + | + | + | + |
| | pH | ++ | ++ | ++ | ++ | + | + | + |
| | Visko | ++ | ++ | ++ | ++ | + | + | + |
| B2 | Abbau | ++ | ++ | ++ | ++ | ++ | ++ | + |
| | pH | ++ | ++ | ++ | ++ | ++ | ++ | + |
| | Visko | ++ | ++ | ++ | ++ | ++ | + | + |
| B3 | Abbau | ++ | ++ | ++ | ++ | ++ | ++ | + |
| | pH | ++ | ++ | ++ | ++ | ++ | ++ | + |
| | Visko | ++ | ++ | ++ | ++ | + | + | + |
| B4 | Abbau | ++ | ++ | ++ | + | + | + | + |
| | pH | ++ | ++ | ++ | + | + | + | + |
| | Visko | ++ | ++ | ++ | + | + | + | + |
| | | | | | | | | |

| | | **3M** | **6M** | **12 M** | **18 M** | **24 M** | **36 M** | **39 M** |
|---|---|---|---|---|---|---|---|---|
| B5 | Abbau | ++ | ++ | ++ | + | + | + | + |
| | pH | ++ | ++ | ++ | + | + | + | + |
| | Visko | ++ | ++ | ++ | + | + | + | + |
| B6 | Abbau | ++ | ++ | ++ | ++ | + | + | + |
| | pH | ++ | ++ | ++ | ++ | ++ | + | + |
| | Visko | ++ | ++ | ++ | ++ | ++ | + | + |
| B7 | Abbau | ++ | ++ | ++ | ++ | + | + | + |
| | pH | ++ | ++ | ++ | ++ | ++ | ++ | + |
| | Visko | ++ | ++ | ++ | + | + | + | + |
| B8 | Abbau | ++ | ++ | ++ | + | + | + | + |
| | pH | ++ | ++ | ++ | + | + | + | + |
| | Visko | ++ | ++ | + | + | + | + | + |

**Tabelle 1C [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| C1 | Abbau | ++ | ++ | ++ | + | + | + | + |
| | pH | ++ | ++ | ++ | + | + | + | + |
| | Visko | ++ | ++ | + | + | + | + | + |
| C2 | Abbau | ++ | ++ | ++ | + | + | + | + |
| | pH | ++ | ++ | ++ | ++ | + | + | + |
| | Visko | ++ | ++ | ++ | ++ | + | + | + |

**Tabelle 1D [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| D1 | Abbau | ++ | ++ | ++ | + | + | + | + |
| | pH | ++ | ++ | ++ | + | + | + | + |
| | Visko | ++ | ++ | + | + | + | + | + |
| D2 | Abbau | ++ | ++ | + | + | + | + | + |
| | pH | ++ | ++ | ++ | + | + | + | + |
| | Visko | ++ | ++ | ++ | + | + | + | + |

**Tabelle 1E [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| E1 | Abbau | ++ | ++ | + | + | + | - | - |
| | pH | ++ | ++ | + | + | + | + | - |
| | Visko | ++ | ++ | + | + | + | - | - |
| E2 | Abbau | ++ | ++ | ++ | ++ | ++ | + | + |
| | pH | ++ | ++ | ++ | ++ | ++ | + | + |
| | Visko | ++ | ++ | ++ | ++ | ++ | + | + |
| E3 | Abbau | ++ | ++ | ++ | ++ | + | + | + |
| | pH | ++ | ++ | ++ | ++ | ++ | + | + |
| | Visko | ++ | ++ | ++ | ++ | + | + | + |
| E4 | Abbau | ++ | ++ | + | + | + | - | - |
| | pH | ++ | ++ | ++ | + | + | + | - |
| | Visko | ++ | ++ | + | + | + | + | - |

**Tabelle 1F [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| F1 | Abbau | ++ | ++ | + | - | - | - | - |
| | pH | ++ | ++ | + | + | + | - | - |
| | Visko | ++ | ++ | + | + | - | - | - |
| F2 | Abbau | ++ | ++ | + | + | - | - | - |
| | pH | ++ | ++ | + | + | + | - | - |
| | Visko | ++ | ++ | + | + | + | - | - |
| F3 | Abbau | ++ | ++ | + | + | - | - | - |
| | pH | ++ | ++ | + | + | - | - | - |
| | Visko | ++ | ++ | + | + | + | - | - |
| F4 | Abbau | ++ | ++ | + | + | + | - | - |
| | pH | ++ | ++ | + | + | + | - | - |
| | Visko | ++ | ++ | + | + | + | - | - |

**Tabelle 2A [(40 ± 2) °C und (75 ± 5) % r.H.]:**

| | | **3M** | **6M** | **12 M** | **18 M** | **24 M** | **36 M** | **39 µM** |
|---|---|---|---|---|---|---|---|---|
| A1 | Abbau | ++ | ++ | ++ | ++ | ++ | + | + |
| | pH | ++ | ++ | ++ | ++ | + | + | + |
| | Visko | ++ | ++ | ++ | ++ | ++ | + | + |

**Tabelle 2B [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| B1 | Abbau | ++ | ++ | ++ | + | + | + | - |
| | pH | ++ | ++ | + | + | + | + | - |
| | Visko | ++ | ++ | + | + | + | + | - |
| B2 | Abbau | ++ | ++ | ++ | ++ | + | + | - |
| | pH | ++ | ++ | ++ | ++ | + | + | + |
| | Visko | ++ | ++ | ++ | + | + | + | + |
| B3 | Abbau | ++ | ++ | ++ | + | + | + | + |
| | pH | ++ | ++ | ++ | + | + | + | + |
| | Visko | ++ | ++ | + | + | + | + | + |
| B4 | Abbau | ++ | ++ | + | + | + | - | - |
| | pH | ++ | ++ | + | ++ | + | + | - |
| | Visko | ++ | + | + | + | + | - | - |
| B5 | Abbau | ++ | ++ | + | + | + | - | - |
| | pH | ++ | ++ | ++ | + | + | - | - |
| | Visko | ++ | ++ | ++ | + | + | - | - |
| B6 | Abbau | ++ | ++ | ++ | + | + | + | - |
| | pH | ++ | ++ | ++ | ++ | ++ | + | - |
| | Visko | ++ | ++ | ++ | ++ | ++ | + | - |
| B7 | Abbau | ++ | ++ | ++ | ++ | + | + | - |
| | pH | ++ | ++ | ++ | ++ | + | + | - |
| | Visko | ++ | ++ | ++ | + | + | - | - |
| B8 | Abbau | ++ | + | + | + | - | - | - |
| | pH | ++ | ++ | + | + | + | - | - |
| | Visko | ++ | ++ | ++ | + | + | - | - |

**Tabelle 2C [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| C1 | Abbau | ++ | ++ | ++ | + | + | + | + |
| | pH | ++ | ++ | + | + | + | + | - |
| | Visko | ++ | + | + | + | + | + | - |
| C2 | Abbau | ++ | ++ | + | + | + | - | - |
| | pH | ++ | ++ | ++ | + | + | + | - |
| | Visko | ++ | ++ | + | + | + | - | - |

**Tabelle 2D [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| D1 | Abbau | ++ | ++ | + | + | + | + | - |
| | pH | ++ | ++ | + | + | + | + | + |
| | Visko | ++ | + | + | + | + | + | - |
| D2 | Abbau | ++ | ++ | + | + | + | + | - |
| | pH | ++ | ++ | + | + | + | + | - |
| | Visko | ++ | + | + | + | - | - | - |

**Tabelle 2E [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| E1 | Abbau | + | + | + | + | - | - | - |
| | pH | ++ | + | + | + | - | - | - |
| | Visko | ++ | + | + | + | - | - | - |
| E2 | Abbau | ++ | ++ | ++ | + | + | + | - |
| | pH | ++ | ++ | ++ | + | + | + | - |
| | Visko | ++ | ++ | ++ | + | + | + | - |
| E3 | Abbau | ++ | ++ | ++ | + | + | - | - |
| | pH | ++ | ++ | ++ | + | + | + | + |
| | Visko | ++ | ++ | ++ | + | + | - | - |
| E4 | Abbau | ++ | + | + | + | - | - | - |
| | pH | ++ | ++ | + | + | + | - | - |
| | Visko | ++ | + | + | + | + | - | - |

**Tabelle 2F [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| F1 | Abbau | ++ | + | - | - | - | - | - |
| | pH | ++ | + | + | - | - | - | - |
| | Visko | ++ | + | + | - | - | - | - |
| F2 | Abbau | ++ | + | + | - | - | - | - |
| | pH | ++ | + | + | + | - | - | - |
| | Visko | ++ | + | + | + | - | - | - |
| F3 | Abbau | + | - | - | - | - | - | - |
| | pH | ++ | + | - | - | - | - | - |
| | Visko | + | + | - | - | - | - | - |
| F4 | Abbau | ++ | + | + | - | - | - | - |
| | pH | ++ | ++ | + | + | - | - | - |
| | Visko | ++ | + | + | - | - | - | - |

### Serie II:

**Tabelle 1A' [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| A1' | Abbau | ++ | ++ | ++ | ++ | ++ | ++ | ++ |
| | pH | ++ | ++ | ++ | ++ | ++ | ++ | ++ |
| | Visko | ++ | ++ | ++ | ++ | ++ | ++ | ++ |

**Tabelle 1B' [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| B1' | Abbau | ++ | ++ | ++ | + | + | + | - |
| | pH | ++ | ++ | ++ | + | + | + | + |
| | Visko | ++ | ++ | ++ | ++ | + | + | + |
| B2' | Abbau | ++ | ++ | ++ | ++ | + | + | + |
| | pH | ++ | ++ | ++ | ++ | ++ | ++ | + |
| | Visko | ++ | ++ | ++ | ++ | ++ | + | + |
| B3' | Abbau | ++ | ++ | ++ | ++ | + | + | + |
| | pH | ++ | ++ | ++ | ++ | ++ | + | + |
| | Visko | ++ | ++ | ++ | + | + | + | + |
| B4' | Abbau | ++ | ++ | ++ | + | + | + | - |
| | pH | ++ | ++ | ++ | + | + | + | + |
| | Visko | ++ | ++ | ++ | + | + | + | - |
| | | | | | | | | |

| | | **3M** | **6M** | **12 M** | **18 M** | **24 M** | **36 M** | **39 M** |
|---|---|---|---|---|---|---|---|---|
| B5' | Abbau | ++ | ++ | ++ | + | + | + | - |
| | pH | ++ | ++ | ++ | + | + | + | + |
| | Visko | ++ | ++ | ++ | ++ | + | + | + |
| B6' | Abbau | ++ | ++ | ++ | ++ | + | + | + |
| | pH | ++ | ++ | ++ | ++ | + | + | + |
| | Visko | ++ | ++ | ++ | ++ | ++ | + | + |
| B7' | Abbau | ++ | ++ | ++ | ++ | + | + | + |
| | pH | ++ | ++ | ++ | ++ | ++ | ++ | + |
| | Visko | ++ | ++ | ++ | + | + | + | + |
| B8' | Abbau | ++ | ++ | ++ | + | + | + | - |
| | pH | ++ | ++ | + | + | + | + | + |
| | Visko | ++ | ++ | ++ | + | + | + | + |

**Tabelle 1C' [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| C1' | Abbau | ++ | ++ | + | + | + | + | + |
| | pH | ++ | ++ | ++ | + | + | + | + |
| | Visko | ++ | ++ | + | + | + | + | + |
| C2' | Abbau | ++ | ++ | + | + | + | + | + |
| | pH | ++ | ++ | ++ | + | + | + | + |
| | Visko | ++ | ++ | ++ | ++ | + | + | + |

**Tabelle 1D' [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| D1' | Abbau | ++ | ++ | + | + | + | + | + |
| | pH | ++ | ++ | ++ | + | + | + | + |
| | Visko | ++ | ++ | + | + | + | + | - |
| D2' | Abbau | ++ | ++ | + | + | + | + | + |
| | pH | ++ | ++ | ++ | + | + | + | + |
| | Visko | ++ | ++ | ++ | + | + | + | + |

**Tabelle 1E' [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| E1' | Abbau | + | + | + | + | - | - | - |
| | pH | ++ | + | + | + | + | + | - |
| | Visko | ++ | ++ | + | + | + | - | - |
| E2' | Abbau | ++ | ++ | ++ | ++ | ++ | + | + |
| | pH | ++ | ++ | ++ | ++ | ++ | + | + |
| | Visko | ++ | ++ | ++ | ++ | ++ | + | + |
| E3' | Abbau | ++ | ++ | ++ | ++ | + | + | + |
| | pH | ++ | ++ | ++ | ++ | ++ | + | + |
| | Visko | ++ | ++ | ++ | + | + | + | + |
| E4' | Abbau | ++ | + | + | + | + | - | - |
| | pH | ++ | + | + | + | + | + | - |
| | Visko | ++ | + | + | + | + | + | - |

**Tabelle 1F' [(25 ± 2) °C und (60 ± 5) % r.H.]:**

| | | **3M** | **6M** | **12 M** | **18 M** | **24 M** | **36 M** | **39 M** |
|---|---|---|---|---|---|---|---|---|
| F1' | Abbau | + | + | + | - | - | - | - |
| | pH | ++ | + | + | + | + | - | - |
| | Visko | ++ | + | + | - | - | - | - |
| F2' | Abbau | ++ | + | + | - | - | - | - |
| | pH | ++ | ++ | + | + | + | - | - |
| | Visko | ++ | + | + | + | + | - | - |
| F3' | Abbau | ++ | + | + | + | - | - | - |
| | pH | + | + | + | + | - | - | - |
| | Visko | ++ | ++ | + | + | + | - | - |
| F4' | Abbau | ++ | ++ | + | + | + | - | - |
| | pH | ++ | ++ | + | + | + | - | - |
| | Visko | ++ | ++ | + | + | + | - | - |

**Tabelle 2A' [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| A1' | Abbau | ++ | ++ | ++ | ++ | ++ | ++ | + |
| | pH | ++ | ++ | ++ | ++ | ++ | + | + |
| | Visko | ++ | ++ | ++ | ++ | + | + | + |

**Tabelle 2B' [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| B1' | Abbau | ++ | ++ | + | + | + | - | - |
| | pH | ++ | ++ | + | + | + | + | - |
| | Visko | ++ | ++ | + | + | + | + | - |
| B2' | Abbau | ++ | ++ | + | + | + | + | - |
| | pH | ++ | ++ | ++ | + | + | + | + |
| | Visko | ++ | ++ | ++ | + | + | + | + |
| B3' | Abbau | ++ | ++ | ++ | + | + | + | + |
| | pH | ++ | ++ | + | + | + | + | + |
| | Visko | ++ | ++ | + | + | + | + | + |
| B4' | Abbau | ++ | ++ | + | + | + | - | - |
| | pH | ++ | ++ | + | + | + | - | - |
| | Visko | ++ | + | + | + | + | - | - |
| B5' | Abbau | ++ | + | + | + | - | - | - |
| | pH | ++ | ++ | ++ | + | + | - | - |
| | Visko | ++ | ++ | ++ | + | + | - | - |
| B6' | Abbau | ++ | ++ | ++ | + | + | - | - |
| | pH | ++ | ++ | ++ | ++ | ++ | + | - |
| | Visko | ++ | ++ | ++ | ++ | ++ | + | - |
| B7' | Abbau | ++ | ++ | ++ | ++ | + | + | - |
| | pH | ++ | ++ | ++ | ++ | + | + | - |
| | Visko | ++ | ++ | ++ | + | + | - | - |
| B8' | Abbau | ++ | + | + | + | - | - | - |
| | pH | ++ | ++ | + | + | - | - | - |
| | Visko | ++ | + | + | + | + | - | - |

**Tabelle 2C' [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| C1' | Abbau | ++ | ++ | ++ | + | + | + | + |
| | pH | ++ | ++ | + | + | + | + | - |
| | Visko | ++ | + | + | + | + | + | - |
| C2' | Abbau | ++ | ++ | + | + | + | - | - |
| | pH | ++ | ++ | ++ | + | + | + | - |
| | Visko | ++ | ++ | + | + | + | - | - |

**Tabelle 2D' [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| D1' | Abbau | ++ | + | + | + | + | - | - |
| | pH | ++ | + | + | + | + | + | - |
| | Visko | ++ | + | + | + | + | + | - |
| D2' | Abbau | ++ | + | + | + | + | + | - |
| | pH | ++ | + | + | + | + | - | - |
| | Visko | ++ | + | + | + | - | - | - |

**Tabelle 2E' [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| E1' | Abbau | + | + | + | - | - | - | - |
| | pH | + | + | + | + | - | - | - |
| | Visko | + | + | + | - | - | - | - |
| E2' | Abbau | ++ | ++ | + | + | + | - | - |
| | pH | ++ | ++ | + | + | + | + | - |
| | Visko | ++ | ++ | ++ | + | + | + | - |
| E3' | Abbau | ++ | ++ | + | + | + | - | - |
| | pH | ++ | ++ | ++ | + | + | + | + |
| | Visko | ++ | ++ | + | + | + | - | - |
| E4' | Abbau | ++ | + | + | - | - | - | - |
| | pH | ++ | + | + | + | + | - | - |
| | Visko | ++ | + | + | + | - | - | - |

**Tabelle 2F' [(40 ± 2) °C und (75 ± 5) % r.H.]:**

| | | **3M** | **6M** | **12 M** | **18 M** | **24 M** | **36 M** | **39 M** |
|---|---|---|---|---|---|---|---|---|
| F1' | Abbau | + | + | **-** | **-** | **-** | **-** | **-** |
| | pH | + | + | - | - | - | - | - |
| | Visko | + | + | + | - | - | - | - |
| F2' | Abbau | + | + | + | - | - | - | - |
| | pH | ++ | + | + | + | - | - | - |
| | Visko | ++ | + | + | + | - | - | - |
| F3' | Abbau | + | - | - | - | - | - | - |
| | pH | + | + | - | - | - | - | - |
| | Visko | + | + | - | - | - | - | - |
| F4' | Abbau | ++ | + | + | - | - | - | - |
| | pH | ++ | + | + | - | - | - | - |
| | Visko | ++ | + | + | - | - | - | - |

Ein charakteristisches Maß für die Stabilität einer Zusammensetzung ist zum einen die Konstanz des pH-Wertes bzw. der Viskosität sowie die Konstanz des Gehalts an Wirkstoffen (d. h. Komponente (a) sowie Komponente (b)). Weiterhin kann der Gehalt an Abbauprodukten in der Zusammensetzung bestimmt werden, um die Stabilität der Zusammensetzung zu bewerten.

Vor diesem Hintergrund zeigen die durchgeführten Stabilitätsuntersuchungen, dass sowohl die Menge der Wirkstoffkomponenten in der Zusammensetzung als auch deren spezielles Molekulargewicht und darüber hinaus auch der pH-Wert und maßgeblich auch das eingesetzte Puffersystem einen signifikanten Einfluss auf die Stabilität, insbesondere Lagerungsstabilität, der zugrundeliegenden Zusammensetzungen ausüben, wobei die Zusammensetzung A1 bzw. A1' mit der speziellen Abstimmung der Komponenten die diesbezüglich besten Eigenschaften aufweist.

Zudem zeigen die Untersuchungen unter Verwendung unterschiedlicher Puffersysteme, dass sich insbesondere mit dem erfindungsgemäß bevorzugt verwendeten Phosphatpuffersystem eine weiterführend verbesserte Langzeitstabilität erreichen lässt. Denn, wie die Stabilitätsuntersuchungen der Anmelderin in überraschender Weise zeigen, bewirkt insbesondere ein solches Phosphatpuffersystem die angestrebte und zuverlässige Langzeitstabilisierung der erfindungsgemäßen Zusammensetzung auch über große Zeiträume. Ohne sich auf eine bestimmte Theorie beschränken oder festlegen zu wollen, lässt sich dieser Effekt des erfindungsgemäß in bevorzugter Weise eingesetzten Phosphatpuffersystems möglicherweise auch auf sekundäre Effekte beispielsweise im Hinblick auf eine weiterführende Stabilisierung der Matrix bzw. des Hydrogels auf Basis der Komponenten (a) bzw. (b) in der Zusammensetzung zurückführen.

Wie die vorstehend angeführten Ergebnisse der Stabilitätsuntersuchungen der Anmelderin belegen, bedurfte es zur Auffindung einer stabilen Zusammensetzung für von Nebenwirkungen einer BCG-Therapie von insbesondere malignen Erkrankungen des Urogenitaltraktes, insbesondere der Harnblase, vorzugsweise von insbesondere malignen Karzinomen, bevorzugt von Harnblasenkarzinomen, nach Art der vorliegenden Erfindung eines intensiven Forschungsaufwands seitens der Anmelderin.

Die Ergebnisse belegen nicht zuletzt in ihrer Gesamtheit die ausgezeichnete Langzeitstabilität der erfindungsgemäßen Zusammensetzung.

### 3. Anwendungsbeobachtungen und Wirksamkeitsstudien

Jeweilige Gruppen von Probanden mit einem diagnostizierten Harnblasenkarzinom werden begleitend zu einer BCG-Therapie die oben angeführten Zusammensetzungen A1, A1' sowie B1, B1' und B4' (d. h. unterschiedliche Mengen an Chondroitinsulfat-Natrium) verabreicht. Die Verabreichung erfolgt mittels Instillation in die Harnblase. Das Volumen der über den Behandlungszeitraum instillierten Zusammensetzungen beträgt pro Verabreichung jeweils 50 ml. Dabei werden folgende Untersuchungskomplexe durchgeführt:
Die Probanden werden insbesondere auf Basis von Befragungen, entsprechenden medizinischen bzw. klinischen Untersuchungen sowie gegebenenfalls auf Basis von Blasenspiegelungen im Hinblick auf das Vorliegen von Pollakisurie, Dysurie, Hämaturie sowie im Hinblick auf das Vorliegen einer Schmerzsymptomatik untersucht.

Die Untersuchungen und Wirksamkeitsstudien zeigen, dass für die Zusammensetzung A1, gefolgt von der Zusammensetzung A1', im Vergleich zu den weiteren Zusammensetzungen, wie zuvor angeführt, die beste Wirksamkeit in Bezug auf die Behandlung der mit der zugrundeliegenden BCG-Therapie einhergehenden bzw. hiervon hervorgerufenen Nebenwirkungen vorliegt, wobei jedoch auch die weiteren Zusammensetzungen B1, B1' sowie B4' jeweils zu insgesamt zumindest zufriedenstellenden Ergebnissen führen. Insbesondere kann im Vergleich zu einer Probandengruppe ohne entsprechende Co-Therapierung mit den erfindungsgemäßen Zusammensetzungen eine deutliche Verbesserung des gesundheitlichen Zustands im Hinblick auf Pollakisurie, Dysurie und Hämaturie festgestellt werden, wobei auch die Schmerzsymptomatik unter Verwendug der erfindungsgemäßen Zusammensetzungen deutlich verbessert ist, insbesondere im Speziellen was die Zusammensetzungen A1 und A1' anbelangt.

Die Untersuchungen zeigen somit insgesamt auch die hervorragende therapeutische Wirksamkeit der erfindungsgemäßen Konzeption.

## Patentansprüche

1. Zusammensetzung zur Verwendung bei der Erhöhung der Verträglichkeit einer BCG-Therapie (*Bacillus-Calmette-Guérin-*Therapie) von malignen Erkrankungen des Urogenitaltraktes,
wobei die Zusammensetzung in Kombination und jeweils in wirksamen Mengen enthält:
(a) Chondroitinsulfat und/oder ein physiologisch verträgliches Chondroitinsulfat-Salz (Komponente (a)), wobei die Zusammensetzung das Chondroitinsulfat und/oder das physiologisch verträgliche Chondrointinsulfat-Salz in einer Konzentration in einem Bereich von (2 ± 0,5) mg/ml bis (25 ± 2) mg/ml aufweist; und
(b) Hyaluronsäure und/oder ein physiologisch verträgliches Hyaluronsäure-Salz (Hyaluronat) (Komponente (b)), wobei die Zusammensetzung die Hyaluronsäure und/oder das physiologisch verträgliche Hyaluronsäure-Salz in einer Konzentration in einem Bereich von (1 ± 0,5) mg/ml bis (20 ± 2) mg/ml aufweist.

2. Zusammensetzung zur Verwendung nach Anspruch 1 zur Verwendung als Co-Therapeutikum bei der BCG-Therapie (*Bacillus-Calmette-Guérin-*Therapie) von malignen Erkrankungen des Urogenitaltraktes, insbesondere der Harnblase, vorzugsweise von insbesondere malignen Karzinomen, bevorzugt von Harnblasenkarzinomen.

3. Zusammensetzung zur Verwendung nach Anspruch 1 oder 2,
wobei die Zusammensetzung außerdem (c) ein Puffersystem, insbesondere Dihydrogenphosphat/Monohydrogenphosphat-Puffersystem, (Komponente (c)) enthält; und/oder
wobei die Zusammensetzung außerdem (d) mindestens einen physiologisch verträglichen Elektrolyten (Komponente (d)) enthält; und/oder
wobei die Zusammensetzung das Chondroitinsulfat und/oder das physiologisch verträgliche Chondrointisulfat-Salz (Komponente (a)) in einer Konzentration in einem Bereich von (3 ± 0,5) mg/ml bis (22 ± 2) mg/ml, vorzugsweise in einem Bereich von (4 ± 0,5) mg/ml bis (20 ± 2) mg/ml, aufweist.

4. Zusammensetzung zur Verwendung nach einem der vorangehenden Ansprüche,
wobei die Zusammensetzung das Chondroitinsulfat und/oder das physiologisch verträgliche Chondrointisulfat-Salz (Komponente (a)) in einer Konzentration von (20 ± 1,5) mg/ml aufweist oder
wobei die Zusammensetzung das Chondroitinsulfat und/oder das physiologisch verträgliche Chondrointisulfat-Salz (Komponente (a)) in einer Konzentration von (4,5 ± 0,4) mg/ml aufweist.

5. Zusammensetzung zur Verwendung nach einem der vorangehenden Ansprüche,
wobei das Chondroitinsulfat und/oder das physiologisch verträgliche Chondroitinsulfat-Salz (Komponente (a)) ein zahlenmittleres Molekulargewicht (Molmasse) Mₙ im Bereich von 2 kDa bis 200 kDa aufweist; und/oder
wobei das Chondroitinsulfat und/oder das physiologisch verträgliche Chondroitinsulfat-Salz (Komponente (a)) einen Polydispersitätsindex (PDI), berechnet als Quotient aus gewichtsmittlerem Molekulargewicht M_{w} und zahlenmittlerem Molekulargewicht Mₙ, von mindestens 1, insbesondere mindestens 1,2, vorzugsweise mindestens 1,5, bevorzugt mindestens 2, aufweist.

6. Zusammensetzung zur Verwendung nach einem der vorangehenden Ansprüche,
wobei die Zusammensetzung die Hyaluronsäure und/oder das physiologisch verträgliche Hyaluronsäure-Salz (Komponente (b)) in einer Konzentration in einem Bereich von (2 ± 0,5) mg/ml bis (18 ± 2) mg/ml, vorzugsweise in einem Bereich von (3 ± 0,5) mg/ml bis (16 ± 2) mg/ml, aufweist; und/oder
wobei die Zusammensetzung die Hyaluronsäure und/oder das physiologisch verträgliche Hyaluronsäure-Salz (Komponente (b)) in einer Konzentration von (16 ± 1,2) mg/ml, insbesondere in einer Konzentration von (16 ± 0,8) mg/ml, vorzugsweise in einer Konzentration von (16 ± 0,4) mg/ml, besonders bevorzugt in einer Konzentration von etwa 16 mg/ml, enthält.

7. Zusammensetzung zur Verwendung nach einem der vorangehenden Ansprüche,
wobei die Hyaluronsäure und/oder das physiologisch verträgliche Hyaluronsäure-Salz (Komponente (b)) ein zahlenmittleres Molekulargewicht (Molmasse) Mₙ im Bereich von 10 kDa bis 300 kDa aufweist; und/oder
wobei die Hyaluronsäure und/oder das physiologisch verträgliche Hyaluronsäure-Salz (Komponente (b)) einen Polydispersitätsindex (PDI), berechnet als Quotient aus gewichtsmittlerem Molekulargewicht M_{w} und zahlenmittlerem Molekulargewicht Mₙ, von mindestens 1, insbesondere mindestens 1,1, vorzugsweise mindestens 1,2, bevorzugt mindestens 1,3, besonders bevorzugt mindestens 1,4, aufweist.

8. Zusammensetzung zur Verwendung nach einem der vorangehenden Ansprüche,
wobei die Zusammensetzung das Puffersystem, insbesondere das Dihydrogenphosphat/Monohydrogenphosphat-Puffersystem (Komponente (c)), in einer Gesamtkonzentration an Puffersystem, insbesondere Dihydrogenphosphat/Monohydrogenphosphat-Puffersystem, von (1,75 ± 1,65) mg/ml enthält; und/oder
wobei das Dihydrogenphosphat/Monohydrogenphosphat-Puffersystem (Komponente (c)) als Alkalidihydrogenphosphat/Alkalimonohydrogenposphat-Puffersystem vorliegt; und/oder
wobei das Dihydrogenphosphat/Monohydrogenphosphat-Puffersystem (Komponente (c)) als NaH₂(PO₄) / Na₂H(PO₄)-Puffersystem vorliegt.

9. Zusammensetzung zur Verwendung nach einem der vorangehenden Ansprüche,
wobei die Zusammensetzung den Elektrolyten (Komponente (d)) in einer Konzentration von (8 ± 6) mg/ml enthält; und/oder
wobei der Elektrolyt (Komponente (d)) in Form eines Alkalisalzes, insbesondere in Form eines Alkalichlorids, vorzugsweise in Form von Natriumchlorid, vorliegt.

10. Zusammensetzung zur Verwendung nach einem der vorangehenden Ansprüche,
wobei die Zusammensetzung außerdem (e) BCG (*Bacillus-Calmette-Guérin*), insbesondere in Form von lebensfähigen Einheiten, (Komponente (e)) enthält, insbesondere in Mengen von 10⁵ bis 10¹⁰ lebensfähigen Einheiten, insbesondere 10⁶ bis 10⁹ lebensfähigen Einheiten, vorzugsweise 10⁷ bis 10⁹ lebensfähigen Einheiten, bezogen auf das Gesamtvolumen der zu verabreichenden Zusammensetzung; und/oder
wobei die Zusammensetzung einen pH-Wert in einem Bereich von 6,1 bis 7,9, insbesondere in einem Bereich von 6,6 bis 7,7, vorzugsweise in einem Bereich von 6,9 bis 7,6, bevorzugt in einem Bereich von 7,1 bis 7,4, aufweist.

11. Zusammensetzung zur Verwendung nach einem der vorangehenden Ansprüche,
wobei die Zusammensetzung bei Temperaturen im Bereich von 20 °C bis 45 °C, bei einem Druck von 1.013,25 mbar (Atmosphärendruck) und bei einer relativen Luftfeuchtigkeit im Bereich von 50 % bis 90 % mindestens 6 Monate, insbesondere mindestens 12 Monate, vorzugsweise mindestens 24 Monate, bevorzugt mindestens 36 Monate, stabil, insbesondere lagerungsstabil, ist; und/oder
wobei die Zusammensetzung eine Stabilität, insbesondere Lagerungsstabilität, unter beschleunigten Alterungsbedingungen gemäß ASTM F 1980 bei einer Alterungstemperatur von 55 °C von mindestens 6 Monate, insbesondere mindestens 12 Monate, vorzugsweise mindestens 24 Monate, bevorzugt mindestens 36 Monate, aufweist; wobei die Zusammensetzung zum jeweiligen Lagerungszeitpunkt einen Gesamtgehalt an Abbauprodukten der Komponenten (a) und (b) von höchstens 5 %, insbesondere höchstens 4 %, vorzugsweise höchstens 3 %, bevorzugt höchstens 2 %, besonders bevorzugt höchstens 1 %, bezogen auf die Gesamtkonzentration der Komponenten (a) und (b), aufweist; und/oder
wobei die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegt.

12. Aufbewahrungs- und/oder Applikationsvorrichtung zur Verwendung bei der Erhöhung der Verträglichkeit einer BCG-Therapie (*Bacillus-Calmette-Guerin-Therapie*) von malignen Erkrankungen des Urogenitaltraktes, enthaltend eine Zusammensetzung zur Verwendung nach einem der vorangehenden Ansprüche.

13. Aufbewahrungs- und/oder Applikationsvorrichtung zur Verwendung nach Anspruch 12,
wobei die Zusammensetzung eine Wirkstoffmenge an (a) Chondroitinsulfat und/oder physiologisch verträglichem Chondroitinsulfat-Salz (Komponente (a)) in einem Bereich von (100 ± 25) mg bis (1.250 ± 100) mg, insbesondere in einem Bereich von (150 ± 10) mg bis (1.100 ± 100) mg, vorzugsweise in einem Bereich von (200 ± 10) mg bis (1.000 ± 100) mg, aufweist; und/oder
wobei die Zusammensetzung eine Wirkstoffmenge an (b) Hyaluronsäure und/oder physiologisch verträglichem Hyaluronsäure-Salz (Hyaluronat) (Komponente (b)) in einem Bereich von (50 ± 25) mg bis (1.000 ± 100) mg, insbesondere in einem Bereich von (100 ± 25) mg bis (900 ± 100) mg, vorzugsweise in einem Bereich von (150 ± 25) mg bis (800 ± 100) mg, aufweist; und/oder
wobei die Zusammensetzung das Puffersystem (Komponente (c)), insbesondere das Dihydrogenphosphat/Monohydrogenphosphat-Puffersystem, in einer Gesamtmenge an Puffersystem, insbesondere an Dihydrogenphosphat/Monohydrogenphosphat-Puffersystem, von (87,5 ± 85) mg, insbesondere in einer Gesamtmenge von (87,5 ± 75) mg, vorzugsweise in einer Gesamtmenge von (87,5 ± 62,5) mg, bevorzugt in einer Gesamtmenge von (87,5 ± 50) mg, besonders bevorzugt in einer Gesamtmenge von (87,5 ± 45) mg, ganz besonders bevorzugt in einer Gesamtmenge von (87,5 ± 40) mg, noch weiter bevorzugt in einer Gesamtmenge von etwa 87,5 mg, aufweist; und/oder
wobei die Zusammensetzung den Elektrolyten (Komponente (d)) in einer Menge von (400 ± 300) mg, insbesondere in einer Menge von (400 ± 200) mg, vorzugsweise in einer Menge von (400 ± 100) mg, bevorzugt in einer Menge von (400 ± 50) mg, besonders bevorzugt in einer Menge von etwa 400 mg, enthält; und/oder
wobei die Aufbewahrungs- und/oder Applikationsvorrichtung die Zusammensetzung, insbesondere anwendungs-, dosier- und/oder applikationsfertig, mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, enthält; und/oder
wobei die Zusammensetzung mit einem Volumen von (50 ± 10) ml, insbesondere (50 ± 5) ml, vorzugsweise (50 ± 2) ml, bevorzugt (50 ± 1) ml, besonders bevorzugt (50 ± 0,5) ml, ganz besonders bevorzugt etwa 50 ml, vorliegt.

14. Verpackungseinheit zur Verwendung bei der Erhöhung der Verträglichkeit einer BCG-Therapie (*Bacillus-Calmette-Guérin-*Therapie) von malignen Erkrankungen des Urogenitaltraktes, enthaltend mindestens eine Aufbewahrungs- und/oder Applikationsvorrichtung nach Anspruch 12 oder 13, wobei die Aufbewahrungs- und/oder Applikationsvorrichtung in einer vor Kontaminationen schützenden Umverpackung eingebracht vorliegt.

15. Kit zur Verwendung bei der Erhöhung der Verträglichkeit einer BCG-Therapie (*Bacillus-Calmette-Guérin-*Therapie) von malignen Erkrankungen des Urogenitaltraktes, umfassend (i) mindestens eine Aufbewahrungs- und/oder Applikationsvorrichtung nach Anspruch 12 oder 13, (ii) mindestens eine Zusammensetzung nach einem der vorangehenden Ansprüche und (iii) mindestens eine an die Aufbewahrungs- und/oder Applikationsvorrichtung anschließbare Instillationsvorrichtung.

## Claims

1. A composition for use in increasing the tolerability of a BCG treatment (*Bacillus Calmette-Guérin* treatment) of malignant diseases of the urogenital tract,
wherein the composition contains, in combination and in effective amounts in each case:
(a) chondroitin sulphate and/or a physiologically tolerable chondroitin sulphate salt (component (a)), wherein the composition includes the chondroitin sulphate and/or the physiologically tolerable chondroitin sulphate salt in a concentration in a range from (2 ± 0.5) mg/ml to (25 ± 2) mg/ml; and
(b) hyaluronic acid and/or a physiologically tolerable hyaluronic acid salt (hyaluronate) (component (b)), wherein the composition includes the hyaluronic acid and/or the physiologically tolerable hyaluronic acid salt in a concentration in a range from (1 ± 0.5) mg/ml to (20 ± 2) mg/ml.

2. The composition for use according to claim 1 for use as a co-treatment agent in the BCG treatment (*Bacillus Calmette-Guérin* treatment) of malignant diseases of the urogenital tract, in particular the bladder, preferably of in particular malignant carcinomas, preferably of bladder carcinomas.

3. The composition for use according to claim 1 or 2,
wherein the composition also contains (c) a buffer system, in particular a dihydrogen phosphate/monohydrogen phosphate buffer system, (component (c)); and/or
wherein the composition also contains (d) at least one physiologically tolerable electrolyte (component (d)); and/or
wherein the composition includes the chondroitin sulphate and/or the physiologically tolerable chondroitin sulphate salt (component (a)) in a concentration in a range from (3 ± 0.5) mg/ml to (22 ± 2) mg/ml, preferably in a range from (4 ± 0.5) mg/ml to (20 ± 2) mg/ml.

4. The composition for use according to any one of the preceding claims,
wherein the composition includes the chondroitin sulphate and/or the physiologically tolerable chondroitin sulphate salt (component (a)) in a concentration of (20 ± 1.5) mg/ml or
wherein the composition includes the chondroitin sulphate and/or the physiologically tolerable chondroitin sulphate salt (component (a)) in a concentration of (4.5 ± 0.4) mg/ml.

5. The composition for use according to any one of the preceding claims,
wherein the chondroitin sulphate and/or the physiologically tolerable chondroitin sulphate salt (component (a)) has a number-average molecular weight (molar mass) Mₙ in the range from 2 kDa to 200 kDa; and/or
wherein the chondroitin sulphate and/or the physiologically tolerable chondroitin sulphate salt (component (a)) has a polydispersity index (PDI), calculated as the quotient of weight-average molecular weight M_{w} and number-average molecular weight Mₙ, of at least 1, in particular at least 1.2, preferably at least 1.5, more preferably at least 2.

6. The composition for use according to any one of the preceding claims,
wherein the composition contains the hyaluronic acid and/or the physiologically tolerable hyaluronic acid salt (component (b)) in a concentration in a range from (2 ± 0.5) mg/ml to (18 ± 2) mg/ml, preferably in a range from (3 ± 0.5) mg/ml to (16 ± 2) mg; and/or
wherein the composition contains the hyaluronic acid and/or the physiologically tolerable hyaluronic acid salt (component (b)) in a concentration of (16 ± 1.2) mg/ml, in particular in a concentration of (16 ± 0.8) mg/ml, preferably in a concentration of (16 ± 0.4) mg/ml, particularly preferably in a concentration of about 16 mg/ml.

7. The composition for use according to any one of the preceding claims,
wherein the hyaluronic acid and/or the physiologically tolerable hyaluronic acid salt (component (b)) has a number-average molecular weight (molar mass) Mₙ in the range from 10 kDa to 300 kDa; and/or
wherein the hyaluronic acid and/or the physiologically tolerable hyaluronic acid salt (component (b)) has a polydispersity index (PDI), calculated as the quotient of weight-average molecular weight M_{w} and number-average molecular weight Mₙ, of at least 1, in particular at least 1.1, preferably at least 1.2, more preferably at least 1.3, particularly preferably at least 1.4.

8. The composition for use according to any one of the preceding claims,
wherein the composition contains the buffer system, in particular the dihydrogen phosphate/monohydrogen phosphate buffer system (component (c)), in a total concentration of buffer system, in particular dihydrogen phosphate/monohydrogen phosphate buffer system, of (1.75 ± 1.65) mg/ml; and/or
wherein the dihydrogen phosphate/monohydrogen phosphate buffer system (component (c)) is present as an alkali dihydrogen phosphate/alkali monohydrogen phosphate buffer system; and/or
wherein the dihydrogen phosphate/monohydrogen phosphate buffer system (component (c)) is present as an NaH₂(PO₄) / Na₂H(PO₄) buffer system.

9. The composition for use according to any one of the preceding claims,
wherein the composition contains the electrolyte (component (d)) in a concentration of (8 ± 6) mg/ml; and/or
wherein the electrolyte (component (d)) is present in the form of an alkali salt, in particular in the form of an alkali chloride, preferably in the form of sodium chloride.

10. The composition for use according to any one of the preceding claims,
wherein the composition further contains (e) BCG (*Bacillus Calmette-Guérin*), in particular in the form of viable units (component (e)), in particular in amounts of 10⁵ to 10¹⁰ viable units, in particular 10⁶ to 10⁹ viable units, preferably 10⁷ to 10⁹ viable units, in relation to the total volume of the composition to be administered; and/or
wherein the composition has a pH value in a range from 6.1 to 7.9, in particular in a range from 6.6 to 7.7, preferably in a range from 6.9 to 7.6, more preferably in a range from 7.1 to 7.4.

11. The composition for use according to any one of the preceding claims,
wherein the composition is stable, in particular storage-stable, at temperatures in the range from 20°C to 45°C, at a pressure of 1013.25 mbar (atmospheric pressure) and at a relative humidity in the range from 50% to 90% for at least 6 months, in particular at least 12 months, preferably at least 24 months, more preferably at least 36 months; and/or
wherein the composition has a stability, in particular storage stability, under accelerated ageing conditions according to ASTM F 1980 at an ageing temperature of 55°C of at least 6 months, in particular at least 12 months, preferably at least 24 months, more preferably at least 36 months; wherein the composition at the point of storage has a total content of degradation products of components (a) and (b) of at most 5%, in particular at most 4%, preferably at most 3%, more preferably at most 2%, particularly preferably at most 1%, in relation to the total concentration of components (a) and (b); and/or
wherein the composition, in particular ready for use, dosing and/or application, is present with a volume of (50 ± 10) ml, in particular (50 ± 5) ml, preferably (50 ± 2) ml, more preferably (50 ± 1) ml, particularly preferably (50 ± 0.5) ml, very particularly preferably about 50 ml.

12. A storage and/or application device for use in increasing the tolerability of a BCG treatment (*Bacillus Calmette-Guérin* treatment) of malignant diseases of the urogenital tract, containing a composition for use according to any one of the preceding claims.

13. The storage and/or application device for use according to claim 12,
wherein the composition includes an active ingredient amount of (a) chondroitin sulphate and/or physiologically tolerable chondroitin sulphate salt (component (a)) in a range from (100 ± 25) mg to (1,250 ± 100) mg, in particular in a range from (150 ± 10) mg to (1,100 ± 100) mg, preferably in a range from (200 ± 10) mg to (1,000 ± 100) mg; and/or
wherein the composition includes an active ingredient amount of (b) hyaluronic acid and/or physiologically tolerable hyaluronic acid salt (hyaluronate) (component (b)) in a range from (50 ± 25) mg to (1,000 ± 100) mg, in particular in a range from (100 ± 25) mg to (900 ± 100) mg, preferably in a range from (150 ± 25) mg to (800 ± 100) mg; and/or
wherein the composition includes the buffer system (component (c)), in particular the dihydrogen phosphate/monohydrogen phosphate buffer system, in a total amount of buffer system, in particular of dihydrogen phosphate/monohydrogen phosphate buffer system, of (87.5 ± 85) mg, in particular in a total amount of (87.5 ± 75) mg, preferably in a total amount of (87.5 ± 62.5) mg, more preferably in a total amount of (87.5 ± 50) mg, particularly preferably in a total amount of (87.5 ± 45) mg, very particularly preferably in a total amount of (87.5 ± 40) mg, even more preferably in a total amount of about 87.5 mg; and/or
wherein the composition contains the electrolyte (component (d)) in an amount of (400 ± 300) mg, in particular in an amount of (400 ± 200) mg, preferably in an amount of (400 ± 100) mg, more preferably in an amount of (400 ± 50) mg, particularly preferably in an amount of about 400 mg; and/or
wherein the storage and/or application device contains the composition, in particular ready for use, dosing and/or application, with a volume of (50 ± 10) ml, in particular (50 ± 5) ml, preferably (50 ± 2) ml, more preferably (50 ± 1) ml, particularly preferably (50 ± 0.5) ml, very particularly preferably about 50 ml; and/or
wherein the composition is present with a volume of (50 ± 10) ml, in particular (50 ± 5) ml, preferably (50 ± 2) ml, more preferably (50 ± 1) ml, particularly preferably (50 ± 0.5) ml, very particularly preferably about 50 ml.

14. A packaging unit for use in increasing the tolerability of a BCG treatment (*Bacillus Calmette-Guérin* treatment) of malignant diseases of the urogenital tract, containing at least one storage and/or application device according to claim 12 or 13, wherein the storage and/or application device is present in an outer packaging that protects against contamination.

15. A kit for use in increasing the tolerability of a BCG treatment (*Bacillus Calmette-Guérin* treatment) of malignant diseases of the urogenital tract, comprising (i) at least one storage and/or application device according to claim 12 or 13, (ii) at least one composition according to any one of the preceding claims, and (iii) at least one instillation device that can be connected to the storage and/or application device.

## Revendications

1. Composition destinée à être utilisée pour augmenter la tolérance d'une BCG-thérapie (thérapie par *Bacille de Calmette-Guérin)* des maladies malignes du tractus urogénital,
dans laquelle la composition contient en combinaison et respectivement en quantités efficaces :
(a) du sulfate de chondroïtine et/ou un sel de sulfate de chondroïtine physiologiquement acceptable (composant (a)), dans laquelle la composition comporte le sulfate de chondroïtine et/ou le sel de sulfate de chondroïtine physiologiquement acceptable à une concentration située dans une plage allant de (2 ± 0,5) mg/ml à (25 ± 2) mg/ml ; et
(b) de l'acide hyaluronique et/ou un sel d'acide hyaluronique physiologiquement acceptable (hyaluronate) (composant (b)), dans laquelle la composition comporte l'acide hyaluronique et/ou le sel d'acide hyaluronique physiologiquement acceptable à une concentration située dans une plage allant de (1 ± 0,5) mg/ml à (20 ± 2) mg/ml.

2. Composition destinée à être utilisée selon la revendication 1, afin d'être utilisée en tant qu'agent co-thérapeutique dans la BCG-thérapie (thérapie par *Bacille de Calmette-Guérin*) des maladies malignes du tractus urogénital, en particulier de la vessie, de préférence, en particulier des carcinomes malins, de préférence du carcinome de la vessie.

3. Composition destinée à être utilisée selon la revendication 1 ou 2,
dans laquelle la composition contient également (c) un système tampon, en particulier un système tampon dihydrogénophosphate/monohydrogénophosphate (composant (c)) ; et/ou
dans laquelle la composition contient également (d) au moins un électrolyte physiologiquement acceptable (composant (d)) ; et/ou
dans laquelle la composition comporte le sulfate de chondroïtine et/ou le sel de sulfate de chondroïtine physiologiquement acceptable (composant (a)) à une concentration située dans une plage allant de (3 ± 0,5) mg/ml à (22 ± 2) mg/ml, de préférence dans une plage allant de (4 ± 0,5) mg/ml à (20 ± 2) mg/ml.

4. Composition destinée à être utilisée selon l'une quelconque des revendications précédentes,
dans laquelle la composition comporte le sulfate de chondroïtine et/ou le sel de sulfate de chondroïtine physiologiquement acceptable (composant (a)) à une concentration de (20 ± 1,5) mg/ml ou
dans laquelle la composition contient le sulfate de chondroïtine et/ou le sel de sulfate de chondroïtine physiologiquement acceptable (composant (a)) à une concentration de (4,5 ± 0,4) mg/ml.

5. Composition destinée à être utilisée selon l'une quelconque des revendications précédentes,
dans laquelle le sulfate de chondroïtine et/ou le sel de sulfate de chondroïtine physiologiquement acceptable (composant (a)) présentent une masse moléculaire moyenne en nombre (masse molaire) Mₙ située dans la plage allant de 2 kDa à 200 kDa ; et/ou
dans laquelle le sulfate de chondroïtine et/ou le sel de sulfate de chondroïtine physiologiquement acceptable (composant (a)) présentent un indice de polydispersité (PDI), calculé comme le quotient du poids moléculaire moyen en poids M_{w} et du poids moléculaire moyen en nombre Mₙ, d'au moins 1, en particulier d'au moins 1,2, de préférence d'au moins 1,5, préférentiellement d'au moins 2.

6. Composition destinée à être utilisée selon l'une quelconque des revendications précédentes,
dans laquelle la composition comporte l'acide hyaluronique et/ou le sel d'acide hyaluronique physiologiquement acceptable (composant (b)) à une concentration située dans une plage allant de (2 ± 0,5) mg/ml à (18 ± 2) mg/ml, de préférence dans une plage allant de (3 ± 0,5) mg/ml à (16 ± 2) mg/ml ; et/ou
dans laquelle la composition contient l'acide hyaluronique et/ou le sel d'acide hyaluronique physiologiquement acceptable (composant (b)) à une concentration de (16 ± 1,2) mg/ml, en particulier à une concentration de (16 ± 0,8) mg/ml, de préférence à une concentration de (16 ± 0,4) mg/ml, de manière particulièrement préférée à une concentration d'environ 16 mg/ml.

7. Composition destinée à être utilisée selon l'une quelconque des revendications précédentes,
dans laquelle l'acide hyaluronique et/ou le sel d'acide hyaluronique physiologiquement acceptable (composant (b)) présentent un poids moléculaire moyen en nombre (masse molaire) Mₙ situé dans la plage allant de 10 kDa à 300 kDa ; et/ou
dans laquelle l'acide hyaluronique et/ou le sel d'acide hyaluronique physiologiquement acceptable (composant (b)) présentent un indice de polydispersité (PDI), calculé comme le quotient de la masse moléculaire moyenne en poids M_{w} et de la masse moléculaire moyenne en nombre Mₙ, d'au moins 1, en particulier d'au moins 1,1, de préférence d'au moins 1,2, préférentiellement d'au moins 1,3, de manière particulièrement préférée d'au moins 1,4.

8. Composition destinée à être utilisée selon l'une quelconque des revendications précédentes,
dans laquelle la composition contient le système tampon, en particulier le système tampon dihydrogénophosphate/monohydrogénophosphate (composant (c)), à une concentration totale du système tampon, en particulier du système tampon dihydrogénophosphate/monohydrogénophosphate, de (1,75 ± 1,65) mg/ml ; et/ou
dans laquelle le système tampon dihydrogénophosphate/monohydrogénophosphate (composant (c)) est présent sous la forme d'un système tampon dihydrogénophosphate alcalin/monohydrogénophosphate alcalin ; et/ou
dans laquelle le système tampon dihydrogénophosphate/monohydrogénophosphate (composant (c)) est présent sous la forme d'un système tampon NaH₂(PO₄)/Na₂H(PO₄).

9. Composition destinée à être utilisée selon l'une quelconque des revendications précédentes,
dans laquelle la composition contient les électrolytes (composant (d)) à une concentration de (8 ± 6) mg/ml ; et/ou
dans laquelle l'électrolyte (composant (d)) est sous la forme d'un sel alcalin, en particulier sous la forme d'un chlorure alcalin, de préférence sous la forme de chlorure de sodium.

10. Composition destinée à être utilisée selon l'une quelconque des revendications précédentes,
dans laquelle la composition contient en outre (e) le BCG *(Bacille de Calmette-Guérin),* en particulier sous la forme d'unités viables (composant (e)), en particulier en quantité allant de 10⁵ à 10¹⁰ unités viables, en particulier de 10⁶ à 10⁹ unités viables, de préférence 10⁷ à 10⁹ unités viables, rapporté au volume total de ladite composition à administrer ; et/ou
dans laquelle la composition présente un pH dans une plage allant de 6,1 à 7,9, en particulier dans une plage allant de 6,6 à 7,7, de préférence dans une plage allant de 6,9 à 7,6, préférentiellement dans une plage allant de 7,1 à 7,4.

11. Composition destinée à être utilisée selon l'une quelconque des revendications précédentes,
dans laquelle la composition, à des températures dans la plage allant de 20 °C à 45 °C, à une pression de 1013,25 mbar (pression atmosphérique) et à une humidité relative dans la plage allant de 50 % à 90 %, pendant au moins 6 mois, en particulier au moins 12 mois, de préférence au moins 24 mois, préférentiellement au moins 36 mois, est stable, en particulier stable au stockage ; et/ou
dans laquelle la composition présente une stabilité, en particulier une stabilité au stockage, dans des conditions de vieillissement accéléré selon ASTM F 1980 à une température de vieillissement de 55 °C d'au moins 6 mois, en particulier d'au moins 12 mois, de préférence d'au moins 24 mois, préférentiellement d'au moins 36 mois ; ladite composition au moment respectif du stockage présentant une teneur totale en produits de dégradation des composants (a) et (b) d'au plus 5 %, en particulier d'au plus 4 %, de préférence d'au plus 3 %, préférentiellement d'au plus 2 %, de manière particulièrement préférée d'au plus 1 %, rapporté à la concentration totale des composants (a) et (b) ; et/ou
dans laquelle la composition, en particulier prête à l'emploi, au dosage et/ou à l'application, présente un volume de (50 ± 10) ml, en particulier de (50 ± 5) ml, de préférence de (50 ± 2) ml, de préférence de (50 ± 1) ml, de manière particulièrement préférée de (50 ± 0,5) ml, de manière encore plus particulièrement préférée d'environ 50 ml.

12. Dispositif de stockage et/ou d'application destiné à être utilisé lors d'une augmentation de la tolérance d'une BCG-thérapie (thérapie par *Bacille de Calmette-Guérin)* des maladies malignes du tractus urogénital, comprenant une composition destinée à être utilisée selon l'une quelconque des revendications précédentes.

13. Dispositif de stockage et/ou d'application destiné à être utilisé selon la revendication 12,
dans lequel la composition comporte une quantité de principe actif (a) de sulfate de chondroïtine et/ou de sel de sulfate de chondroïtine physiologiquement acceptable (composant (a)) dans une plage allant de (100 ± 25) mg à (1250 ± 100) mg, en particulier dans une plage allant de (150 ± 10) mg à (1100 ± 100) mg, de préférence dans une plage allant de (200 ± 10) mg à (1000 ± 100) mg ; et/ou
dans lequel la composition comporte une quantité de principe actif (b) d'acide hyaluronique et/ou de sel d'acide hyaluronique physiologiquement acceptable (hyaluronate) (composant (b)) dans une plage allant de (50 ± 25) mg à (1000 ± 100) mg, en particulier dans une plage allant de (100 ± 25) mg à (900 ± 100) mg, de préférence dans une plage allant de (150 ± 25) mg à (800 ± 100) mg ; et/ou
dans lequel la composition comporte le système tampon (composant (c)), en particulier le système tampon dihydrogénophosphate/monohydrogénophosphate, en une quantité totale de système tampon, en particulier de système tampon dihydrogénophosphate/monohydrogénophosphate, de (87,5 ± 85) mg, en particulier en une quantité totale de (87,5 ± 75) mg, de préférence en une quantité totale de (87,5 ± 62,5) mg, préférentiellement en une quantité totale de (87,5 ± 50) mg, de manière particulièrement préférée en une quantité totale de (87,5 ± 45) mg, de manière encore plus particulièrement préférée en une quantité totale de (87,5 ± 40) mg, encore plus préférentiellement en une quantité totale d'environ 87,5 mg ; et/ou
dans lequel la composition contient les électrolytes (composant (d)) en une quantité de (400 ± 300) mg, en particulier en une quantité de (400 ± 200) mg, de préférence en une quantité de (400 ± 100) mg, préférentiellement en une quantité de (400 ± 50) mg, de manière particulièrement préférée en une quantité d'environ 400 mg ; et/ou
dans lequel le dispositif de stockage et/ou d'application contient ladite composition, en particulier prête à l'emploi, au dosage et/ou à l'application, d'un volume de (50 ± 10) ml, en particulier de (50 ± 5) ml, de préférence de (50 ± 2) ml, préférentiellement de (50 ± 1) ml, de manière particulièrement préférée de (50 ± 0,5) ml, de manière encore plus particulièrement préférée de 50 ml ; et/ou
dans lequel la composition présente un volume de (50 ± 10) ml, en particulier de (50 ± 5) ml, de préférence de (50 ± 2) ml, préférentiellement de (50 ± 1) ml, de manière particulièrement préférée de (50 ± 0,5) ml, de manière encore plus particulièrement préférée d'environ 50 ml.

14. Unité de conditionnement destinée à être utilisée pour augmenter de la tolérance d'une BCG-thérapie (thérapie par *Bacille de Calmette-Guérin*) des maladies malignes du tractus urogénital, contenant au moins un dispositif de stockage et/ou d'application selon la revendication 12 ou 13, dans laquelle le dispositif de stockage et/ou d'application est présent dans un emballage extérieur protégeant contre les contaminations.

15. Kit destiné à être utilisé pour augmenter la tolérance d'une BCG-thérapie (thérapie par *Bacille de Calmette-Guérin)* des maladies malignes du tractus urogénital, comprenant (i) au moins un dispositif de stockage et/ou d'application selon la revendication 12 ou 13, (ii) au moins une composition selon l'une quelconque des revendications précédentes et (iii) au moins un dispositif d'instillation pouvant être raccordé au dispositif de stockage et/ou d'application.
